# EUROPEAN PATENT APPLICATION

(11) **EP 4 725 970 A1**
(43) Date of publication of application: **15.04.2026**
(21) Application number: 24818686.8
(22) Date of filing: 05.06.2024
(51) Int. Cl.: C07K 19/00, C07K 16/28, A61K 47/68, A61P 35/00, G01N 33/68, C12N 5/10

(54) **PREPARATION AND USE OF ANTI-GPRC5D/BCMA/CD3 TRISPECIFIC ANTIBODY**

(30) Priority: 06.06.2023 CN 202310664900; 04.06.2024 CN 202410713270
(71) Applicant: Fortvita Biologics Inc., 1205 Grand Cayman (KY)
(72) Inventor: WANG, Xuan, Suzhou, Jiangsu 215123 (CN); YANG, Xiao, Suzhou, Jiangsu 215123 (CN); JIANG, Yun, Suzhou, Jiangsu 215123 (CN); WANG, Yi, Suzhou, Jiangsu 215123 (CN); ISSAFRAS, Hassan, Suzhou, Jiangsu 215123 (CN); CHI, Liqing, Suzhou, Jiangsu 215123 (CN); WANG, Yue, Suzhou, Jiangsu 215123 (CN); PRINZ, Bianka, Suzhou, Jiangsu 215123 (CN); BOLAND, Nadthakarn, Suzhou, Jiangsu 215123 (CN); GEOGHEGAN, James, Suzhou, Jiangsu 215123 (CN)
(74) Representative: Sagittarius IP
(86) International application number: PCT/CN2024/097542
(87) International publication number: WO 2024/251154

(57) **Abstract**

The present invention relates to a trispecific antigen-binding protein, and particularly to a trispecific antibody specifically binding to two tumor antigens of GPRC5D and BCMA and a T-cell surface antigen CD3, a pharmaceutical composition comprising the same, a preparation method therefor, and use thereof.

## Description

### CROSS REFERENCE TO RELATED APPLICATIONS

This application is based on and claims priority to Chinese Patent Application No. 202310664900.5, filed on June 6, 2023, and Chinese Patent Application No. 202410713270.0, filed on June 4, 2024, the contents of which are incorporated herein by reference in their entirety.

### TECHNICAL FIELD

The present invention relates to a trispecific antigen-binding protein, and particularly to a trispecific antibody specifically binding to two tumor antigens of GPRC5D and BCMA and a T-cell surface antigen CD3, a pharmaceutical composition comprising the same, a preparation method therefor, and use thereof.

### BACKGROUND

T cell bispecific antibodies (BsAbs) have been used for tumor treatment. Such antibodies can form synapses between cytotoxic T lymphocytes and tumor cells, inducing tumor cell destruction. The BsAbs generally involve dual targeting against tumor-associated antigens (TAAs) and T-cell surface antigens (also known as T-cell engaging antigens (TEAs)). However, therapeutic strategies based on the BsAbs typically rely on the distribution of the tumor-associated antigens on the tumor cells to be treated. This leads to the selectivity of treatment for the patient population and the limitation of treatment. In addition, studies have also shown that therapeutic modalities targeting a single TAA site may cause the recurrence of the disease due to the escape mechanism of the tumor, thereby limiting the effectiveness of the treatment.

The development of trispecific and/or tetraspecific antibodies has been proposed to effectively recruit immune cells to tumor sites and improve the efficacy of the treatment. For this reason, in recent years, various multivalent and multispecific antibody formats have been described. However, the diversity of requirements for therapeutic functionality and therapeutic behavior of different therapeutic products has determined that there is no "optimal form" that can be applied to most different desired molecular combinations. Accordingly, in achieving multispecific properties, a variety of factors need to be considered, including, for example, the spatial distribution or size of the different target antigens, as well as the tumor-associated antigen expression density at the surface of the tumor cells. In many cases, the antibody format that is effective for a particular combination of target antigens must be identified by generating and comparing the functionality of the different antibody formats.

B-cell maturation antigen (BCMA, i.e., CD269 or TNFRSF17) is a member of the tumor necrosis factor receptor superfamily (TNFRSF). BCMA is a type III transmembrane protein with a cysteine-rich domain (CRD) forming a ligand-binding motif in the extracellular domain (ECD), which is distinctive to TNFR superfamily. Ligands of BCMA include B-cell activating factor (BAFF) and a proliferation-inducing ligand (APRIL), wherein a proliferation-inducing ligand (APRIL) of B cell binds to BCMA with higher affinity, promoting tumor cell proliferation.

BCMA has been found to be overexpressed in multiple myeloma (MM) cells in preclinical models and human tumors, which up-regulates canonical and non-canonical NF-κB signals, promotes the growth, survival and adhesion of MM cells, and induces osteoclast activation, angiogenesis, metastasis, and immunosuppression. The expression of BCMA has become an important marker for diagnosing MM. In addition, the increase of sBCMA levels in the serum of patients with MM is in direct proportion to the number of MM cells in the bone marrow, and changes in their concentrations are closely correlated with MM prognosis and therapeutic response. Multiple myeloma, also known as plasmacytoma or Kahler's disease, is a malignant tumor of refractory B cell lines characterized by abnormal proliferation of plasma cells. In view of the characteristics that BCMA is only expressed in plasma cells and not expressed in natural and memory B cells, BCMA has become a hot target for the treatment of B cell malignant tumors, particularly multiple myeloma. There is a need in the art to develop antibodies, particularly multispecific antibodies, directed against BCMA.

Although a trispecific antibody specifically binding to CD3, BCMA and GPRC5D has been disclosed in the prior art, such as WO2022174813A1, in this molecular structure, the BCMA antigen-binding region is in the form of an scFv and is at the C-terminus of Fc. It faces the following problems: the purification yield of antibodies is low and the aggregation is high; the BCMA antigen-binding region scFv has a low affinity for the antigen and poor spatial position, so that the BCMA-mediated tumor cell killing is very weak; the BCMA antigen-binding region comprises a part of hotspot sites with poor druggability.

Accordingly, there is a need for a novel anti-GPRC5D/BCMA/CD3 trispecific antibody that can overcome the above disadvantages, has a better tumor killing effect, is easier to be purified, and has good druggability.

### SUMMARY

The present invention provides a trispecific antibody comprising a first antigen-binding region specifically binding to BCMA, and second and third antigen-binding regions specifically binding to other antigens.

In some embodiments, the second antigen-binding region specifically binds to CD3, and the third antigen-binding region specifically binds to GPRC5D.

In some embodiments, the trispecific antibody of the present invention prevents light chain mispairing by applying charge mutations and disulfide bond mutations. In some embodiments, in the trispecific antibody of the present invention, the first antigen-binding region specifically binding to BCMA is an Fab.

In some embodiments, the second antigen-binding region specifically binding to CD3 is an Fab.

In some embodiments, the antigen-binding region such as Fab specifically binding to BCMA is located at the N-terminus of the antigen-binding region such as Fab specifically binding to CD3. In some embodiments, when the trispecific antibody forms an immunological synapse for T cell killing, the T cell is closer to the tumor cell than the trispecific antibodies known in the prior art (such as the trispecific antibody disclosed in WO2022174813A1, particularly the scFv specifically binding to BCMA is located at the C-terminus of Fc), thus the trispecific antibody of the present invention has stronger T cell killing. In some embodiments, the trispecific antibody of the present invention has a structure in which the first antigen-binding region specifically binding to BCMA is capable of blocking the function of CD3, thereby avoiding the activation of non-specific T cells in the whole body and subsequent toxicity in the absence of tumor antigens.

In some embodiments, the trispecific antibody of the present invention has a particular antigen-binding region specifically binding to BCMA, thereby having improved druggability, and/or reducing the production of antibody immunogenicity.

Accordingly, the trispecific antibody of the present invention has one or more or all of the following advantages, particularly compared to the trispecific antibody disclosed in WO2022174813A1:
i) having improved affinity for BCMA;
ii) having improved BCMA-mediated tumor killing;
iii) having a reduced aggregation tendency of antibody molecules and/or an improved purification yield;
iv) having stronger T cell killing;
v) having reduced non-specific cytotoxicity, for example, to avoid the activation of non-specific T cells in the whole body and subsequent toxicity in the absence of tumor antigens;
vi) having reduced antibody immunogenicity; and/or
vii) having improved druggability.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to accurate arrangement and means of the embodiments shown in the drawings.
FIG. 1 shows exemplary structures of trispecific antibodies, wherein FIG. 1A corresponds to the B3 and B3b formats, FIG. 1B corresponds to the B5 structure, and FIG. 1C corresponds to the F2 format.
FIG. 2A shows the cell-based affinity-1 of exemplary antibodies for human BCMA (GS-CHO-hBCMA).
FIG. 2B shows the cell-based affinity-2 of exemplary antibodies for human BCMA (GS-CHO-hBCMA).
FIG. 2C shows the cell-based affinity-1 of exemplary antibodies for cynomolgus monkey BCMA (GS-CHO-cynoBCMA).
FIG. 2D shows the cell-based affinity-1 of exemplary antibodies for human GPRC5D (GS-CHO-hGPRC5D).
FIG. 2E shows the cell-based affinity-2 of exemplary antibodies for human GPRC5D (GS-CHO-hGPRC5D).
FIG. 2F shows the cell-based affinity-1 of exemplary antibodies for cynomolgus monkey GPRC5D (GS-CHO-cynoGPRC5D).
FIG. 2G shows the cell-based affinity-1 of exemplary antibodies for human CD3 (Jurkat).
FIG. 2H shows the cell-based affinity of exemplary antibodies for human CD3 (CD8⁺ T cells and CD4⁺ T cells).
FIG. 2I shows the cell affinity of exemplary antibodies for 293T cells expressing mutant BCMA antigens.
FIG. 2J shows the cell affinity of exemplary antibodies for GS CHO cells expressing mutant BCMA antigens.
FIG. 3A shows the killing effect-1 of PBMCs mediated by exemplary antibodies on H929 ko GPRC5D cells.
FIG. 3B shows the killing effect-2 of PBMCs mediated by exemplary antibodies on H929 ko GPRC5D cells.
FIG. 3C shows the killing effect-3 of PBMCs mediated by exemplary antibodies on H929 ko GPRC5D cells.
FIG. 3D shows the killing effect-1 of PBMCs mediated by exemplary antibodies on H929 ko BCMA cells.
FIG. 3E shows the killing effect-2 of PBMCs mediated by exemplary antibodies on H929 ko BCMA cells.
FIG. 3F shows the killing effect-3 of PBMCs mediated by exemplary antibodies on H929 ko BCMA cells.
FIG. 3G shows the killing effect-1 of PBMCs mediated by exemplary antibodies on H929 cells.
FIG. 3H shows the killing effect-2 of PBMCs mediated by exemplary antibodies on H929 cells.
FIG. 3I shows the killing effect-3 of PBMCs mediated by exemplary antibodies on H929 cells.
FIG. 3J shows the killing effect-1 of PBMCs mediated by exemplary antibodies on L363 cells.
FIG. 3K shows the killing effect-2 of PBMCs mediated by exemplary antibodies on L363 cells.
FIG. 3L shows the killing effect-3 of PBMCs mediated by exemplary antibodies on L363 cells.
FIG. 3M shows the killing effect-4 of PBMCs mediated by exemplary antibodies on L363 cells.
FIG. 3N shows the killing effect-1 of PBMCs mediated by exemplary antibodies on mixed cells.
FIG. 3O shows the killing effect-2 of PBMCs mediated by exemplary antibodies on mixed cells.
FIG. 3P shows the killing effect of PBMCs mediated by exemplary antibodies on non-target cell Calu-6.
FIG. 3Q shows the release amount-1 (3 antibody concentrations) of three cytokines accompanied by the killing process of PBMCs mediated by exemplary antibodies on H929.
FIG. 3R shows the release amount-2 (3 antibody concentrations) of three cytokines accompanied by the killing process of PBMCs mediated by exemplary antibodies on H929.
FIG. 3S shows the release amount-1 (no target cells, 3 antibody concentrations) of non-antigen specific cytokines of PBMCs mediated by exemplary antibodies.
FIG. 3T shows the release amount-2 (no target cells, 3 antibody concentrations) of non-antigen specific cytokines of PBMCs mediated by exemplary antibodies.
FIG. 3U shows the killing effect of PBMCs mediated by exemplary antibodies on GS CHO overexpressing BCMA mutant.
FIG. 4A shows the activation of CD4 T cells in PBMCs mediated by exemplary antibodies in the presence of sBCMA.
FIG. 4B shows the activation of CD8 T cells in PBMCs mediated by exemplary antibodies in the presence of sBCMA.
FIG. 4C shows the killing of PBMCs mediated by exemplary antibodies on H929 cells in the presence of sBCMA.
FIG. 5A shows the tumor inhibitory effect of exemplary antibodies in the H929 ko GPRC5D tumor-bearing humanized mouse model, wherein the administration dose and statistical method are indicated in the figure legends, and the administration is performed on day 7, day 14 and day 21 post tumor cell inoculation.
FIG. 5B shows the tumor inhibitory effect of exemplary antibodies in the H929 ko BCMA tumor-bearing humanized mouse model, wherein the administration dose and statistical method are indicated in the figure legends, and the administration is performed on day 7 and day 14 post tumor cell inoculation.
FIG. 5C shows the tumor inhibitory effect of exemplary antibodies in the MM1S tumor-bearing humanized mouse model, wherein the administration dose is indicated in the figure legends, and the administration is performed on day 7 and day 14 post tumor cell inoculation.
FIG. 5D shows the tumor inhibitory effect of exemplary antibodies in the L363 tumor-bearing humanized mouse model, wherein the administration is performed on day 7 and day 18 post tumor cell inoculation.
FIG. 6 shows the expression results of BCMA and GPRC5D in H929, H929 ko GPRC5D and H929 ko BCMA cell lines by flow cytometry.
FIG. 7 shows the aggregate difference between the B3(24) molecule and the control molecule (F2 (24)).

### DETAILED DESCRIPTION

Unless otherwise defined, all technical and scientific terms used herein have the same meaning as commonly understood by those of ordinary skill in the art to which the present invention belongs. All publications, patent applications, patents, and other references mentioned herein are incorporated by reference in their entireties. In addition, the materials, methods, and examples described herein are illustrative only and are not intended to be limiting. Other features, objectives, and advantages of the present invention will be apparent from the specification and drawings, and from the appended claims.

### Definitions

It should be understood that the present invention is not limited to the particular methodology, protocols, and reagents described herein, as these may vary. It should also be understood that the terminology used herein is only intended to describe specific embodiments rather than limit the scope of the present invention, which will be limited only by the appended claims. Unless otherwise defined, all technical and scientific terms used herein have the same meaning as commonly understood by those of ordinary skill in the art to which the present invention belongs.

For the purpose of explaining this specification, the following definitions will be used, and wherever appropriate, terms used in the singular form may also include the plural form, and vice versa.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more or all of the options.

As used herein, the term "comprise" or "include" is intended to mean that the described elements, integers or steps are included, but not to the exclusion of any other elements, integers or steps. The term "comprise" or "include" used herein, unless otherwise indicated, also encompasses the situation where the entirety consists of the described elements, integers or steps. For example, when referring to an antibody variable region "comprising" a particular sequence, it is also intended to encompass an antibody variable region consisting of the particular sequence.

The term "GPRC5D" refers to a tumor-associated antigen, G protein-coupled receptor family C group 5 member D (e.g., human GPRC5D protein under UniProt accession No. Q9NZD1). In one embodiment, the antigen-binding region of the antibody molecule of the present invention that binds to GPRC5D may have high affinity binding activity for GPRC5D-expressing cells, and have an EC₅₀ value of, for example, 1-150 nM, such as 20-135 nM, for human GPRC5D-expressing cells, as measured by flow cytometry. In one embodiment, the antigen-binding specificity is cross-reactive to human and monkey GPRC5D.

The term "BCMA" refers to a tumor-associated antigen, B-cell maturation antigen, also known as BCMA, TR17_HUMAN, or TNFRSF17 (e.g., human BCMA protein under UniProt accession No. Q02223). "BCMA" encompasses not only the wild-type protein, but also mutants, e.g., natural variants, or variants that are mutated in disease (in particular, variants with mutations that lead to drug resistance). In one embodiment, the antigen-binding region of the antibody molecule of the present invention that binds to BCMA may have high affinity binding activity for BCMA, and, for example, have a K_{D} value of 0.1-10 nM, such as 0.125 nM, for human BCMA, as measured by bio-layer interferometry. In one embodiment, the antigen-binding specificity is cross-reactive to human and monkey BCMA.

The term "CD3" refers to a T-cell engaging antigen, T-cell surface glycoprotein CD3 (e.g., human CD3 protein under UniProt accession No. P07766). In one embodiment, the antigen-binding region of the antibody molecule of the present invention that binds to CD3 may have high affinity binding activity for CD3 (such as CD3E&G), and have a KD value of, for example, 10-100 nM, such as 10-70 nM, for human CD3E&G, as measured by bio-layer interferometry. In one embodiment, the antigen-binding specificity is cross-reactive to human and monkey CD3. When referring to "first", "second", and "third" herein, it is only to distinguish three domains or three chains or three antigen-binding regions, and does not indicate the location of the three domains or three antigen-binding regions in any way.

When describing the structure of the antibody of the present invention, the term "N-terminus/N-terminal end" refers to the last amino acid at the N-terminus, and the term "C-terminus/C-terminal end" refers to the last amino acid at the C-terminus.

As used herein, the term "binding" or "specific binding" means that the binding effect is selective for antigens and can be distinguished from unwanted or non-specific interactions. The ability of an antigen-binding site to bind to a particular antigen can be determined by an enzyme-linked immunosorbent assay (ELISA) or a conventional binding assay known in the art.

"Affinity" or "binding affinity" refers to inherent binding affinity that reflects the interaction between members of a binding pair. The affinity of molecule X for its partner Y can be generally represented by the dissociation constant (KD), which is a ratio of the dissociation rate constant to the association rate constant (kdis and kon, respectively). The affinity can be measured by common methods known in the art. One particular method for measuring the affinity is the ForteBio kinetic binding assay herein.

The term "target" refers to the bound substance against which the binding molecule is directed. The target may be an antigen, or may be a ligand or a receptor. The term "antigen" refers to a molecule that induces an immune response. Such an immune response may involve antibody production or activation of specific immune cells, or both. Those skilled will appreciate that any macromolecules, including essentially all proteins or peptides, can be used as antigens. In addition, an antigen may be derived from recombinant or genomic DNA. As used herein, the term "epitope" refers to a portion, which specifically interacts with an antibody molecule, of an antigen. In some embodiments, the antigen is a tumor-associated antigen (i.e., an antigen associated with the development and progression of a tumor) or a T-cell engager.

The term "target-binding region" as used herein refers to a portion of a multispecific binding molecule, e.g., a trispecific binding molecule, that binds to a particular target or antigen. The target-binding region may be, for example, an antibody or immunoglobulin per se or an antibody fragment. Such target-binding regions may or may not have a tertiary structure independent of the remainder of the multispecific antibody molecule, and may or may not bind to their targets as separate entities. The target-binding region may also be a receptor or a ligand, or a domain of a receptor capable of binding to a ligand. In the case of multispecific antibodies, the "target-binding region" is also referred to as the "antigen-binding region". In one embodiment, the antigen-binding region used in the multispecific antibody molecule of the present invention comprises a VH/VL pair consisting of a light chain variable region (VL) and a heavy chain variable region (VH) of the antibody, and the VH/VL pair may be contained in two separate polypeptide chains (e.g., contained in an Fab heavy chain and an Fab light chain, respectively). In one embodiment, the antigen-binding region used in the trispecific antibody molecule of the present invention may be an Fab.

The term "IgG-like multispecific antibody" as described herein refers to a multispecific antibody comprising an Fc dimer.

The term "multispecific binding molecule" refers to a multispecific binding molecule that is at least bispecific, such as a trispecific binding molecule, that is, the molecule comprises at least a first target-binding region, a second target-binding region and a third target-binding region, wherein the first target-binding region binds to one target, the second target-binding region binds to another target, and the third target-binding region binds to a third target. Accordingly, the multispecific binding molecule according to the present invention comprises specificities for at least two different targets. In some embodiments, where the binding molecule is an antibody, the target is an antigen. In some embodiments, the multispecific binding molecule of the present invention is a multispecific antibody, e.g., a trispecific antibody.

As used herein, the term "multispecific" antibody refers to an antibody having at least two antigen-binding regions, each antigen-binding site of which binds to a different epitope of the same antigen or a different epitope of a different antigen. A multispecific antibody is an antibody having binding specificities for at least two different antigen epitopes. In one embodiment, provided herein is a trispecific antibody having binding specificities for a first antigen, a second antigen, and a third antigen. For example, the present invention provides a trispecific antibody directed against BCMA, GPRC5D, and CD3.

When referring to a "first antigen-binding region" in a multispecific antibody or a trispecific antibody, it refers to a binding region that binds to a first antigen and is not intended to limit the number of such antigen-binding regions contained in the antibody, for example, the multispecific antibody can comprise one or more first antigen-binding regions. For example, the trispecific antibody comprises a first antigen-binding region, a second antigen-binding region, and a third antigen-binding region, but can comprise one or more first antigen-binding regions, one or more second antigen-binding regions, or one or more third antigen-binding regions. In some embodiments, the antibody molecule of the present invention comprises at least one antigen-binding region specifically binding to GPRC5D, at least one antigen-binding region specifically binding to BCMA, and at least one antigen-binding region specifically binding to CD3. In some embodiments, the antibody molecule of the present invention comprises one antigen-binding region specifically binding to GPRC5D, one antigen-binding region specifically binding to BCMA, and one antigen-binding region specifically binding to CD3.

When referring to "an antigen-binding region derived from an antibody", it is meant that the binding domain constituting the antigen-binding region is or is derived from a binding domain of the antibody that specifically binds to the antigen, for example, the fragment, such as Fab, of the antigen-binding region that specifically binds to the antigen is or is derived from a corresponding fragment, such as Fab, of the antibody, or the heavy chain variable region and/or the light chain variable region of the antigen-binding region is or is derived from a heavy chain variable region and/or a light chain variable region of the antibody, or 1, 2, 3, 4, 5, or 6 CDRs of the antigen-binding region are CDRs of the antibody. The term "derived from" means that the fragment in the antigen-binding region is substantially identical to the fragment of the antibody from which it is derived, but has mutations, such as substitutions, deletions or additions, at one or more sites. In a specific embodiment, the mutation is not in a CDR of the antibody. In a specific embodiment, the mutation is not in a variable region of the antibody.

The terms "whole antibody" and "full-length antibody" are used interchangeably herein to refer to an antibody molecule having the structure of a native immunoglobulin molecule. In the case of a conventional four-chain IgG antibody, the full-length antibody comprises two heavy chains (H) and two light chains (L) interconnected by disulfide bonds. In the case of a heavy chain antibody having only heavy chains but lacking light chains, the full-length antibody comprises two heavy chains (H) interconnected by disulfide bonds. For the conventional four-chain IgG antibody, the full-length antibody heavy chain generally consists of a heavy chain variable region (abbreviated herein as VH) and a heavy chain constant region, wherein the heavy chain constant region comprises at least 3 domains CH1, CH2, and CH3. The full-length antibody light chain consists of a light chain variable region (abbreviated herein as VL) and a light chain constant region, wherein the light chain constant region consists of one domain CL. Each heavy chain variable region VH or each light chain variable region consists of three CDRs and 4 FRs, arranged from amino-terminus to carboxyl-terminus in the following order: FR1, CDR1, FR2, CDR2, FR3, CDR3, and FR4. The term "antibody fragment" includes a portion of an intact antibody. In a preferred embodiment, the antibody fragment is an antigen-binding fragment.

The term "antigen-binding fragment" of an antibody is a molecule different from a full-length antibody, which comprises a portion of the full-length antibody, but is capable of binding to an antigen of the full-length antibody or competes with the full-length antibody (i.e., a full-length antibody from which the antigen-binding fragment is derived) for binding to an antigen. The antigen-binding fragment may be prepared by recombinant DNA techniques, or by enzymatic or chemical cleavage of an intact antibody. The antigen-binding fragment includes, but is not limited to, an Fab, an Fab', an F(ab')2, an Fv, a single-chain Fv, a diabody, a single-domain antibody (sdAb), and a nanobody. For example, an Fab fragment can be obtained by papain digestion of a full-length antibody. In addition, an F(ab')2, a dimer of Fab', is a bivalent antibody fragment produced by pepsin digestion of a portion below disulfide bonds in a hinge region of a complete antibody. The F(ab')2 can be reduced by disrupting the disulfide bonds in the hinge region under neutral conditions and the F(ab')2 dimer is thus converted into Fab' monomers. The Fab' monomer is essentially an Fab fragment with a hinge region. The Fv fragment consists of the VL and VH domains of a single arm of an antibody. The two domains VL and VH of the Fv fragment can be encoded by separate genes, but the two domains can also be linked, using recombinant methods, by a synthetic linking peptide so that they are produced as a single protein chain in which the VL and VH regions pair to form a single-chain Fv (scFv).

The term "variable region" refers to a domain of a heavy chain or light chain of an antibody involved in the binding of the antibody to an antigen. Variable regions of heavy and light chains of native antibodies generally have similar structures, wherein each domain comprises four conserved framework regions (FRs) and three complementarity determining regions. In some cases, a single VH or VL domain can be sufficient to provide antigen-binding specificity.

The "Fab fragment" and "Fab" are used interchangeably herein to refer to an immunoglobulin fragment consisting of two polypeptide chains and comprising an immunoglobulin heavy chain variable region VH, a heavy chain constant domain CH1, a light chain variable region VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains pair to form an antigen-binding site. As used herein, an Fab polypeptide chain comprising a heavy chain constant region CH1 is also referred to as an "Fab heavy chain"; correspondingly, an Fab polypeptide chain comprising a light chain constant region CL is also referred to as an "Fab light chain".

"Complementarity determining region" or "CDR region" or "CDR" is a region in an antibody variable region that is highly variable in sequence and forms a structurally defined loop ("hypervariable loop") and/or comprises antigen-contacting residues ("antigen-contacting sites"). CDRs are primarily responsible for binding to antigen epitopes. The CDRs of the heavy and light chains are generally referred to as CDR1, CDR2, and CDR3, and are numbered sequentially from the N-terminus. The CDRs located in a heavy chain variable region of an antibody are referred to as HCDR1, HCDR2, and HCDR3, whereas the CDRs located in a light chain variable region of an antibody are referred to as LCDR1, LCDR2, and LCDR3. In a given amino acid sequence of a light chain variable region or a heavy chain variable region, the exact amino acid sequence boundary of each CDR can be determined using any one or a combination of many well-known antibody CDR assignment schemes including, e.g., Chothia based on the three-dimensional structure of antibodies and the topology of the CDR loops (Chothia et al., (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

The term "Fc domain" or "Fc region" is used herein to define a C-terminus region of an immunoglobulin heavy chain, which comprises at least a portion of a constant region. The "Fc region" includes Fc regions of native sequences and variant Fc regions. A native immunoglobulin "Fc domain" comprises two or three constant domains, i.e., a CH2 domain, a CH3 domain, and an optional CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third and the fourth constant domains (CH2 domain, CH3 domain, and CH4 domain) derived from two heavy chains of IgM and IgE antibodies. Unless otherwise stated herein, amino acid residues in Fc regions or heavy chain constant regions are numbered according to the EU numbering scheme (also known as the EU Index) described in, for example, Kabat et al., Sequences of Proteins of Immunological Interes, 5th Ed., Public Health Service, National Institutes of Health, Bethesda, MD, 1991. However, the C-terminal lysine (Lys447) of the Fc region may or may not be present. Two Fc regions can form a dimeric Fc by dimerization, and two different Fc form a heterodimeric Fc by heterodimerization. As used herein, the terms "Fc region", "Fc portion", and "dimeric Fc (e.g., heterodimeric Fc)" do not comprise a heavy chain variable region VH and a light chain variable region VL as well as a heavy chain constant region CH1 and a light chain constant region CL of an immunoglobulin, but in some cases, they can comprise a hinge region at the N-terminus of the heavy chain constant region. In one embodiment, a human IgG heavy chain Fc region extends from Asp221 or from Cys226 or from Asp231 to the carboxyl-terminus of the heavy chain.

In some embodiments, the multispecific antibody of the present invention comprises an Fc region. In one embodiment, the Fc region is a human Fc region. In one embodiment, the Fc region belongs to a human IgG4 subclass. In one embodiment, the Fc region belongs to a human IgG1 subclass.

In one embodiment, a human IgG1 Fc region polypeptide (comprising a hinge region) comprises the following amino acid sequence:

DKTHTCPPCPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGNVFSCSVMHE
ALHNHYTQKSLSLSPGK (SEQ ID NO:87).

A human IgG4 Fc region polypeptide (comprising a hinge region) comprises the following amino acid sequence:

PPCPSCPAPEFLGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPPSQEEMTKNQV
SLTCLVKGFYPSDIAVEWESNGQPENNYKTTPPVLDSDGSFFLYSRLTVDKSRWQEGNVFSCSVMHEALH
NHYTQKSLSLSLGK (SEQ ID NO:88).

As used herein, "heterodimeric Fc or Fc heterodimer" refers to a scaffold comprising two different Fc regions or formed by dimerization of two different Fc regions, which may be linked to a domain that binds to an antigen at the N-terminus or C-terminus (e.g., a heavy and/or light chain variable region of an antibody or an antigen-binding fragment of an antibody that can bind to a target molecule, or a soluble moiety of a ligand or receptor that can bind to a target molecule) to constitute a multispecific antibody, e.g., a bispecific antibody.

The term "CH1 region" refers to a portion of an antibody heavy chain polypeptide that extends from EU position 118 to EU position 220 (EU numbering scheme). In one embodiment, the CH1 domain comprises an amino acid sequence set forth in ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC (SEQ ID NO: 82). In one embodiment, the internal disulfide bonds are removed by mutating cysteines in a CH1 into non-cysteines, or new disulfide bonds are remodeled by mutating non-cysteines into cysteines.

The term "CH2 region" refers to a portion of an antibody heavy chain polypeptide that extends from EU position 231 to EU position 340 (EU numbering scheme). In one embodiment, the CH2 domain comprises an amino acid sequence set forth in APELLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAK (SEQ ID NO:83). The term "CH3 region" refers to a portion of an antibody heavy chain polypeptide that extends from EU position 341 to EU position 447. In one embodiment, the CH3 domain comprises an amino acid sequence set forth in GQPREPQVYTLPPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK (SEQ ID NO:84).

The term "hinge region" refers to a portion of an antibody heavy chain polypeptide that links to the CH1 and CH2 regions in the wild-type antibody heavy chain, e.g., an IgG1 hinge region, such as a sequence from D221 to P230 according to EU numbering. The hinge regions of other IgG subclasses can be determined by alignment with the cysteine residues in the hinge region of the IgG1 subclass sequence. The hinge region is generally a dimeric molecule consisting of two polypeptides having identical amino acid sequences. In one embodiment, the hinge region has an amino acid sequence set forth in DKTHTCPXCP (SEQ ID NO: 99), wherein X is S or P. In one embodiment, the hinge region comprises an amino acid sequence set forth in HTCPXCP (SEQ ID NO: 19), wherein X is S or P. In one embodiment, the hinge region comprises an amino acid sequence set forth in CPXCP (SEQ ID NO: 81), wherein X is S or P. In one embodiment, the internal disulfide bonds of the hinge region are removed by mutating cysteines in the hinge region into non-cysteines.
As used herein, the amino acid positions of the entire variable regions of the heavy and light chains are numbered according to the Kabat numbering scheme described in Kabat et al., Sequences of Proteins of Immunological Interest, 5th Ed., Public Health Service, National Institutes of Health, Bethesda, MD (1991) and referred to herein as "Kabat numbering".
As used herein, when used to refer to an amino acid position in a domain of an antibody other than a variable region (e.g., a constant region, such as an Fc region), it is numbered according to the EU numbering scheme described in Edelman, G.M. et al., Proc. Natl. Acad. USA, 63: 78-85 (1969) and referred to herein as "EU numbering". When a position number and/or an amino acid residue is assigned to a particular antibody isotype, it is intended to apply to the corresponding position and/or amino acid residue of any other antibody isotype, which is known to those skilled in the art.

The term "linker" as used herein refers to any molecule that enables direct connection of different portions of a multispecific antibody. Examples of linkers to establish covalent linkages between different portions of a multispecific antibody include peptide linkers and non-proteinaceous polymers including, but not limited to, polyethylene glycol (PEG), polypropylene glycol, polyalkylene oxide, or copolymers of polyethylene glycol and polypropylene glycol. In some embodiments, the term "peptide linker" according to the present invention refers to an amino acid sequence, wherein the sequence links the amino acid sequences of various portions of a multispecific antibody together. Preferably, the peptide linker has a length sufficient to link two entities in a manner that maintains their conformation relative to each other without interference with the desired activities. The peptide linker may or may not comprise predominantly the following amino acid residues: Gly, Ser, Ala or Thr. Useful linkers include glycine-serine polymers including, for example, (GS)ₙ(SEQ ID NO:112), (GSGGS)ₙ(SEQ ID NO:113), (GGGGS)ₙ(SEQ ID NO:114), (GGGS)ₙ(SEQ ID NO:115) and (GGGGS)ₙG(SEQ ID NO:116), where n is an integer of at least 1 (preferably 2, 3, 4, 5, 6, 7, 8, 9 or 10). Useful linkers also include glycine-alanine polymers, alanine-serine polymers, and other flexible linkers.

An "isolated" antibody is an antibody which has been separated from components of its natural environment. In some embodiments, the antibody is purified to a purity greater than 95% or 99% as determined by, e.g., electrophoresis (e.g., SDS-PAGE, isoelectric focusing (IEF) and capillary electrophoresis) or chromatography (e.g., ion exchange or reverse-phase HPLC).

An "isolated" nucleic acid is a nucleic acid molecule which has been separated from components of its natural environment. The isolated nucleic acid includes a nucleic acid molecule contained in a cell that generally comprises the nucleic acid molecule, but the nucleic acid molecule exists extrachromosomally or at a chromosomal location that is different from its natural chromosomal location. An "isolated nucleic acid encoding an antibody" refers to one or more nucleic acid molecules encoding chains of an antibody or a fragment thereof, including such nucleic acid molecules in a single vector or separate vectors, and such nucleic acid molecules present at one or more positions in a host cell.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

The term "amino acid substitution" or "amino acid mutation" refers to the replacement of at least one amino acid residue in a predetermined parent amino acid sequence with a different "substituted" amino acid residue. The substituted residue or residues may be "naturally occurring amino acid residues" (i.e., encoded by the genetic codes) and selected from: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). Replacements with one or more non-naturally occurring amino acid residues are also encompassed within the definition of amino acid substitutions herein. The "non-naturally occurring amino acid residue" refers to a residue that is capable of covalently binding to adjacent amino acid residues in a polypeptide chain, in addition to those naturally occurring amino acid residues listed above. Examples of the non-naturally occurring amino acid residues include norleucine, ornithine, norvaline, homoserine, Aib, and other amino acid residue analogs.

The term "conservative alteration" or "conservative modification" refers to an amino acid modification or alteration that does not significantly affect or alter the binding characteristics of an antibody or an antibody fragment comprising the amino acid sequence. Such conservative modifications include conservative amino acid substitutions, additions and deletions. Modifications can be introduced into the antibody or the antibody fragment of the present invention by standard techniques known in the art, such as site-directed mutagenesis and PCR-mediated mutagenesis. Conservative substitutions are amino acid substitutions in which an amino acid residue is replaced with an amino acid residue having a similar side chain. Families of the amino acid residues having similar side chains have been defined in the art. These families include amino acids having basic side chains (e.g., lysine, arginine, and histidine), amino acids having acidic side chains (e.g., aspartic acid and glutamic acid), amino acids having uncharged polar side chains (e.g., glycine, asparagine, glutamine, serine, threonine, tyrosine, cysteine, and tryptophan), amino acids having non-polar side chains (e.g., alanine, valine, leucine, isoleucine, proline, phenylalanine, and methionine), amino acids having β-side chains (e.g., threonine, valine, and isoleucine), and amino acids having aromatic side chains (e.g., tyrosine, phenylalanine, tryptophan, and histidine).

An amino acid mutation is indicated by "original amino acid, amino acid position, mutated amino acid". For example, when the mutation site is located in the V region, "Q39D" means that the glutamine amino acid at position 39 according to Kabat is substituted with aspartic acid (D); when the mutation site is located in the C region, "Q124C" means that the glutamine at position 124 according to EU is substituted with cysteine (C). Where an amino acid position is mentioned in the present invention, unless otherwise explicitly stated, it refers to an amino acid position according to the IgG1 heavy chain or Kappa light chain numbering, i.e., it encompasses amino acid positions based on the IgG1 heavy chain or Kappa light chain numbering, as well as amino acid positions on other heavy chains or light chains corresponding to the amino acid position. It should be noted that when describing a mutation, the original amino acid at a particular position may be the described amino acid, or may be other amino acids at the corresponding position.

A "knob-in-hole" mutation is used herein to refer to the introduction of mutations in a first Fc polypeptide and a second Fc polypeptide, respectively, using the "knob-in-hole" technique to form a protuberance ("knob") and a complementary cavity ("hole") at the interface of the first Fc polypeptide and at the interface of the second Fc polypeptide. It is known in the art that the "knob-in-hole" technique enables the engineering of the interface between different chains of an antibody molecule to promote the correct association of the chains of the antibody molecule. Generally, this technique involves introducing a "protuberance" at the interface of one chain, and introducing a corresponding "cavity" at the interface of the other chain to be paired with, such that the protuberance can be placed at the cavity. A preferred interface comprises the CH3 domain from the heavy chain constant domains of one chain and the CH3 domain from the heavy chain constant domains of the other chain to be paired with. The protuberance can be constructed by replacing small amino acid side chains at an interface of the CH3 domain from the heavy chain constant domains of one chain with large side chains, such as tyrosine or tryptophan. The compensating cavity of the same size as, or a similar size to, the protuberance is constructed at an interface of the CH3 domain from the heavy chain constant domains of the other chain to be paired with, by replacing large amino acid side chains with small side chains, such as alanine or threonine. Another optional interface comprises a light chain CL domain and a heavy chain CH1 domain of the Fab fragment described above, and the correct heterodimerization between the two chains of the Fab fragment is promoted by constructing a protuberance-cavity interaction.

The term "effector function" refers to biological activities attributed to an immunoglobulin Fc region that vary with immunoglobulin isotype. Examples of immunoglobulin effector functions include: Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, C1q binding and complement-dependent cytotoxicity (CDC), down regulation of cell surface receptors (such as B-cell receptors), and B-cell activation.

The term "antibody-dependent cell-mediated cytotoxicity (ADCC)" is one of the major mechanisms by which certain cytotoxic effector cells (e.g., natural killer (NK) cells) mediate the killing of target cells and foreign host cells. In some embodiments, the antibody of the present invention provides antibody-dependent cytotoxicity of T lymphocytes and enhances antibody-dependent cytotoxicity of NK cells.

The term "antibody-dependent cellular phagocytosis (ADCP)" refers to a cellular response in which activation of macrophages is induced by binding of a target cell-binding antibody to FcγRIIIa on the macrophage surface, thereby internalizing the target cell and acidifying and degrading the target cell by a phagosome. ADCP may also be mediated by FcγRIIa and FcγRI, but the proportion is relatively small.

The term "pharmaceutical composition" refers to such a composition that exists in a form allowing effective biological activity of the active ingredient contained therein, and does not contain additional ingredients having unacceptable toxicity to a subject to which the composition is administered.

The term "pharmaceutical supplementary material" refers to pharmaceutically acceptable carriers, diluents, adjuvants (e.g., Freund's adjuvants (complete and incomplete)), excipients, buffers, stabilizers, or the like, which are administered with the active substance.

A "conjugate" is an antibody conjugated to one or more other substances, including but not limited to therapeutic agents or labels.

The terms "individual" and "subject" are used interchangeably and include mammals. The mammals include, but are not limited to, domesticated animals (e.g., cows, sheep, cats, dogs, and horses), primates (e.g., human and non-human primates such as monkeys), rabbits and rodents (e.g., mice and rats). In particular, the individual or subject is a human.

The term "tumor" refers to all neoplastic cell growth and proliferation, whether being malignant or benign, and all pre-cancerous and cancerous cells and tissues. The term "tumor" encompasses solid tumors and liquid tumors. In some embodiments, the tumor is cancer. The terms "cancer" and "cancerous" refer to a physiological disease in mammals in which cell growth is unregulated. The terms "cancer", "cancerous" and "tumor" are not mutually exclusive when referred to herein.

As used herein, "treatment" (or "treat" or "treating") refers to slowing, interrupting, arresting, alleviating, stopping, lowering, or reversing the progression or severity of an existing symptom, disorder, condition, or disease. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis. In some embodiments, the antibody molecule of the present invention is used to delay the progression of a disease or to slow the progression of a disease.

As used herein, "prevention" (or "prevent" or "preventing") includes the inhibition of the onset or progression of symptoms of a disease or disorder, or a specific disease or disorder. In some embodiments, subjects with family history of cancer are candidates for preventive regimens. Generally, in the context of cancer, the term "prevention" refers to the administration of a drug prior to the onset of signs or symptoms of cancer, particularly in subjects at risk of cancer.

The term "effective amount" refers to an amount or dosage of the antibody or composition of the present invention which generates expected effects in a patient in need of treatment or prevention after administration to the patient in a single or multiple doses.

The "therapeutically effective amount" refers to an amount effective to achieve a desired therapeutic result at a necessary dosage for a necessary period of time. The therapeutically effective amount is also such an amount that any toxic or undesired effect of the antibody or the antibody fragment or composition thereof is inferior to the therapeutically beneficial effect.

The "prophylactically effective amount" refers to an amount effective to achieve a desired prophylactic result at a necessary dosage for a necessary period of time. Generally, since a prophylactic dose is administered in a subject before or at an earlier stage of a disease, a prophylactically effective amount will be less than a therapeutically effective amount.

The term "pharmaceutical combination" refers to a non-fixed combination product or a fixed combination product, including but not limited to a kit. The term "non-fixed combination" means that the active ingredients (e.g., (i) the antibody of the present invention, and (ii) an additional therapeutic agent) are administered, either simultaneously or sequentially (without specific time limitation or at identical or different time intervals), to a patient as separate entities, wherein such administration provides two or more prophylactically or therapeutically effective active agents in the patient. In some embodiments, the antibodies of the present invention used in the pharmaceutical combination are administered at levels that do not exceed their levels when used alone. The term "fixed combination" means that two or more active agents are administered to a patient simultaneously in the form of a single entity. The dose and/or time intervals of two or more active agents are preferably selected such that the combined use of the components can result in a therapeutic effect on the disease or disorder which is greater than that achieved by the use of either component alone. The ingredients may each take a separate formulation form and such separate formulation forms may be the same or different.

The term "combination therapy" refers to the administration of two or more therapeutic agents or modalities (e.g., radiotherapy or surgery) to treat the diseases as described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (such as tablets, capsules, powder and liquid). The powder and/or liquid can be reconstituted or diluted to a desired dosage before administration. In addition, such administration also includes using each type of the therapeutic agents at approximately the same time or in a sequential manner at different times. In any case, the therapeutic regimen will provide the beneficial effect of the pharmaceutical combination in the treatment of disorders or symptoms described herein.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are called "expression vectors" herein.

The term "host cell" refers to a cell into which an exogenous polynucleotide has been introduced, including progeny of such cells. Host cells include "transformants" and "transformed cells", which include primary transformed cells and progeny derived therefrom, regardless of the number of passages. Host cells are any type of cell systems that can be used to produce the antibody molecule of the present invention, including eukaryotic cells, e.g., mammalian cells, insect cells, and yeast cells; and prokaryotic cells, e.g., *E. coli* cells. Host cells include cultured cells, as well as cells within a transgenic animal, a transgenic plant, or a cultured plant tissue or animal tissue.

The term "label" used herein refers to a compound or composition which is directly or indirectly conjugated or fused to an agent, such as an antibody, and facilitates the detection of the agent to which it is conjugated or fused. The label itself can be detectable (e.g., a radioisotope label or a fluorescent label) or can catalyze a chemical change to a detectable substrate compound or composition in the case of enzymatic labeling. The term is intended to encompass direct labeling of a probe or an antibody by coupling (i.e., physical linking) a detectable substance to the probe or the antibody and indirect labeling of a probe or an antibody by reacting with another reagent which is directly labeled. Examples of indirect labeling include detection of a first antibody using a fluorescently labeled second antibody, and end labeling of a biotinylated DNA probe, such that it can be detected with a fluorescently labeled streptavidin.

"Subject/patient sample" refers to a collection of cells, tissues, or body fluids obtained from a patient or a subject. The source of tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development. Tissue samples may comprise compounds which are naturally not mixed with tissues, such as preservatives, anticoagulants, buffers, fixatives, nutrients, antibiotics, and the like. Examples of tumor samples include but are not limited to tumor biopsies, fine needle aspirates, bronchial lavage fluids, pleural fluids, sputa, urine, surgical specimens, circulating tumor cells, serum, plasma, circulating plasma proteins, ascites, primary cell cultures or cell lines derived from tumors or exhibiting tumor-like properties, and preserved tumor samples such as formalin-fixed, paraffin-embedded tumor samples or frozen tumors samples.

The term "detection" used herein includes quantitative or qualitative detection. Exemplary detection methods include, but are not limited to, immunohistochemistry, immunocytochemistry, flow cytometry (e.g., FACS), magnetic beads complexed with antibody molecules, ELISA assays, and PCR-techniques (e.g., RT-PCR). In some embodiments, the biological sample comprises body fluid, cells or tissue. In certain embodiments, the biological sample is blood, serum, or other liquid samples of biological origin.

All publications, patent applications, patents, and other references mentioned herein are incorporated by reference in their entireties. Any or all of the features described above and throughout the present application may be combined in various embodiments of the present invention. In addition, the materials, methods, and examples described herein are illustrative only and are not intended to be limiting. Other features, objectives, and advantages of the present invention will be apparent from the specification and drawings, and from the appended claims.

### I. Multispecific antibody

One aspect of the present invention relates to a multispecific antibody, e.g., trispecific antibody, comprising a first antigen-binding region specifically binding to BCMA, and second and third antigen-binding regions specifically binding to second and third antigens.

One aspect of the present invention relates to a trispecific antibody specifically binding to GPRC5D, BCMA, and CD3, comprising a first antigen-binding region specifically binding to BCMA, a second antigen-binding region specifically binding to CD3, and a third antigen-binding region specifically binding to GPRC5D.

In some embodiments, the first antigen-binding region is from an anti-BCMA antibody or an antigen-binding fragment thereof, such as an Fab fragment of an anti-BCMA antibody. In some embodiments, the first antigen-binding region is from the anti-BCMA antibody or the antigen-binding fragment thereof described herein.

In some embodiments, the second antigen-binding region is from an anti-CD3 antibody or an antigen-binding fragment thereof, such as an Fab fragment of an anti-CD3 antibody. In some embodiments, the second antigen-binding region is from the anti-CD3 antibody or the antigen-binding fragment thereof described herein.

In some embodiments, the third antigen-binding region is from an anti-GPRC5D antibody or an antigen-binding fragment thereof, such as an Fab fragment of an anti-GPRC5D antibody. In some embodiments, the third antigen-binding region is from the anti-GPRC5D antibody or the antigen-binding fragment thereof described herein.

The first antigen-binding region suitable for use in the trispecific antibody of the present invention can comprise or consist of the anti-BCMA full-length antibody or the antigen-binding fragment thereof of the present invention as long as it is capable of specifically binding to BCMA, including, but not limited to, for example, a full-length antibody, a single-chain Fv, an Fab, an Fab', an (Fab)2, a single-domain antibody, a VHH, a heavy chain antibody, or the like specifically binding to BCMA. Preferably, the first antigen-binding region is an Fab specifically binding to BCMA.

The second antigen-binding region suitable for use in the trispecific antibody of the present invention can comprise or consist of the anti-CD3 full-length antibody or the antigen-binding fragment thereof as long as it is capable of specifically binding to CD3, including, but not limited to, for example, a full-length antibody, a single-chain Fv, an Fab, an Fab', an (Fab)2, a single-domain antibody, a VHH, a heavy chain antibody, or the like specifically binding to CD3. Preferably, the second antigen-binding region is an Fab specifically binding to CD3.

The third antigen-binding region suitable for use in the trispecific antibody of the present invention can comprise or consist of the anti-GPRC5D full-length antibody or the antigen-binding fragment thereof as long as it is capable of specifically binding to GPRC5D, including, but not limited to, for example, a full-length antibody, a single-chain Fv, an Fab, an Fab', an (Fab)2, a single-domain antibody, a VHH, a heavy chain antibody, or the like specifically binding to GPRC5D. Preferably, the third antigen-binding region is an Fab specifically binding to GPRC5D.

In some embodiments, the trispecific antibody of the present invention is an IgG-like trispecific antibody. In some embodiments, the trispecific antibody of the present invention comprises an Fc dimer, such as an Fc heterodimer. In some embodiments, the trispecific antibody of the present invention comprises an Fab specifically binding to BCMA as the first antigen-binding region.

In some embodiments, the three antigen-binding regions of the trispecific antibody of the present invention are all Fab fragments.

### ➢ Antigen-binding regions suitable for use in trispecific antibody of the present invention

In some embodiments, the antigen-binding region of the trispecific antibody of the present invention is an Fab fragment.

The Fab fragments suitable for use in the antigen-binding region of the trispecific antibody consist of two polypeptide chains comprising VH, CH1, VL, and CL domains of the antibody, wherein the VH is paired with the VL and the CH1 is paired with the CL to form the antigen-binding region. In some embodiments, in the Fab, one chain comprises, from N-terminus to C-terminus, a VH and a CH1 (i.e., VH-CH1), and the other chain comprises, from N-terminus to C-terminus, a VL and a CL (i.e., VL-CL).

In some embodiments, in the trispecific antibody, the Fab may be fused to the N-terminus of the Fc domain of the antibody via the C-terminus of the chain comprising the VH; or fused to the C-terminus of the Fc domain of the antibody via the N-terminus of the chain comprising the VH, wherein the Fc domain may or may not comprise a hinge region. In some embodiments, the Fab comprises a VH-CH1 chain and a VL-CL chain and is fused to the N-terminus of the Fc domain of the antibody via the C-terminus of the CH1 of the VH-CH1 chain, or is fused to the C-terminus of the Fc domain of the antibody via the N-terminus of the VH of the VH-CH1 chain.

In some embodiments, the fusion is direct fusion or fusion through a linker. In some embodiments, the linker is (GGGGS)n, wherein n = 1, 2, 3, or 4.

In some embodiments, the antigen-binding region, such as the Fab, in the trispecific antibody of the present invention is mutated to reduce mispairing.

In some embodiments, one antigen-binding region, such as an Fab fragment, of the trispecific antibody of the present invention comprises a disulfide bond remodeling mutation.

In some embodiments, one antigen-binding region, such as an Fab fragment, of the trispecific antibody of the present invention comprises a charge mutation by which both amino acids of the amino acid pair are mutated into amino acids with opposite charges, respectively.

In some embodiments, one antigen-binding region, such as an Fab fragment, of the trispecific antibody of the present invention comprises a charge mutation and a disulfide bond remodeling mutation.

In some embodiments, in the two antigen-binding regions, such as two Fab fragments, of the trispecific antibody of the present invention, one antigen-binding region comprises a charge mutation and the other antigen-binding region comprises a charge mutation and a disulfide bond remodeling mutation. In some embodiments, the two antigen-binding regions comprise charge mutations at the corresponding position, respectively. Preferably, the two antigen-binding regions comprise opposite charge mutations.

### Disulfide bond remodeling mutation:

In some embodiments, the disulfide bond remodeling mutation is that in CH1-CL of an antigen-binding region, the heavy chain CH1 (e.g., of IgG1, IgG2, IgG3, or IgG4) has a substitution of one non-cysteine amino acid to one cysteine amino acid at position 126 (EU numbering), and the light chain CL has a substitution of one non-cysteine amino acid to one cysteine amino acid at position 124 (EU numbering). In some embodiments, the disulfide bond remodeling mutation includes 126C (e.g., F126C) in the CH1 and 124C (e.g., Q124C) in the CL in the antigen-binding region (EU numbering). In some embodiments, the disulfide bond remodeling mutation further includes a substitution of native cysteine to non-cysteine. In some embodiments, the CH1 in the antigen-binding region has a substitution of one cysteine amino acid to one non-cysteine amino acid at position 220 (EU numbering). In some embodiments, the light chain CL in the antigen-binding region has a substitution of one cysteine amino acid to one non-cysteine amino acid at position 214 (EU numbering). In some embodiments, the disulfide bond remodeling mutation includes C220S/A/V (IgG1 subtype, or C131S/A/V at the corresponding position of IgG2, IgG3, or IgG4) in the CH1, and includes C214S/A/V in the CL in the antigen-binding region (EU numbering). In some embodiments, the Fab comprises a CH1 and a CL, wherein the CH1 comprises an F126C mutation, and the CL comprises a Q124C mutation (EU numbering). In some embodiments, the antigen-binding region comprises a CH1 and a CL, wherein the CH1 comprises F126C and C220S mutations (or corresponding C131S), and the CL comprises Q124C and C214S mutations (EU numbering).

In some embodiments, an Fab fragment comprising a disulfide bond remodeling mutation comprises a CH1, wherein the CH1 comprises an amino acid sequence having at least 90%, 91%, 92%, 93%, 94%, 95%, 96%, 97%, 98%, or 99% identity to an amino acid sequence set forth in SEQ ID NO: 85, and comprises F126C. In some embodiments, the CH1 comprises or consists of an amino acid sequence set forth in SEQ ID NO: 85. In some embodiments, the CH1 comprises an amino acid sequence having at least 90%, 91%, 92%, 93%, 94%, 95%, 96%, 97%, 98%, or 99% identity to an amino acid sequence set forth in SEQ ID NO: 86, and comprises F126C and C220S. In some embodiments, the CH1 comprises or consists of an amino acid sequence set forth in SEQ ID NO: 86.

In some embodiments, an Fab fragment comprising a disulfide bond remodeling mutation comprises a CL, wherein the CL comprises an amino acid sequence having at least 90%, 91%, 92%, 93%, 94%, 95%, 96%, 97%, 98%, or 99% identity to an amino acid sequence set forth in SEQ ID NO: 97, and comprises Q124C. In some embodiments, the CL comprises or consists of an amino acid sequence set forth in SEQ ID NO: 97. In some embodiments, the CL comprises an amino acid sequence having at least 90%, 91%, 92%, 93%, 94%, 95%, 96%, 97%, 98%, or 99% identity to an amino acid sequence set forth in SEQ ID NO: 98, and comprises Q124C and C124S. In some embodiments, the CL comprises or consists of an amino acid sequence set forth in SEQ ID NO: 98.

### Charge mutation:

In some embodiments, the charge mutation is a mutation of an amino acid pair on the interface of the heavy chain and the light chain of at least one antigen-binding region, wherein two amino acids in the amino acid pair are mutated into amino acids with opposite charges. In some embodiments, the charge mutation includes a mutation of two amino acids into amino acids with opposite charges on the amino acid pair at the following positions, respectively: position 39 of the heavy chain variable region and position 38 of the light chain variable region (Kabat numbering). In some embodiments, the charge mutation includes a mutation of two amino acids into amino acids with opposite charges on the amino acid pair at the following positions, respectively: Q39 of the heavy chain variable region and Q38 of the light chain variable region (kabat numbering).

In some embodiments, in one antigen-binding region, the amino acid at position 39 of the heavy chain variable region is substituted with K, and the amino acid at position 38 of the light chain variable region is substituted with D. In some embodiments, in one antigen-binding region, the amino acid at position 39 of the heavy chain variable region is substituted with D, and the amino acid at position 38 of the light chain variable region is substituted with K.

As used herein, two antigen-binding regions "comprising opposite charge mutations" means that the first antigen-binding region and the second antigen-binding region comprise charge mutations at positions of one or more identical amino acid pairs, but the charge mutations at the amino acid pairs in one antigen-binding region are different from the charge mutations at the amino acid pairs in the other antigen-binding region, for example, the charge of the mutated amino acids in the first antigen-binding region is opposite to the charge of the mutated amino acids in the second antigen-binding region at the same position. For example, in the first antigen-binding region, the heavy chain amino acid at position X1 is mutated into a positively charged amino acid, and the light chain amino acid at position X2 is mutated into a negatively charged amino acid. Also, in the second antigen-binding region, the heavy chain amino acid at position X1 is mutated into a negatively charged amino acid, and the light chain amino acid at position X2 is mutated into a positively charged amino acid, wherein the positively charged amino acid in the first antigen-binding region may be identical to or different from, e.g., identical to, the positively charged amino acid in the second antigen-binding region; and the negatively charged amino acid in the first antigen-binding region may be identical to or different from, e.g., identical to, the negatively charged amino acid in the second antigen-binding region.

In some embodiments, in the first antigen-binding region, the amino acid at position 39 of the heavy chain variable region is substituted with a positively charged amino acid, and the amino acid at position 38 of the light chain variable region is substituted with a negatively charged amino acid; and/or in the second antigen-binding region, the amino acid at position 39 of the heavy chain variable region is substituted with a negatively charged amino acid, and the amino acid at position 38 of the light chain variable region is substituted with a positively charged amino acid.

In one embodiment, the trispecific antibody of the present invention comprises two antigen-binding regions, wherein
in one antigen-binding region, the amino acid at position 39 of the heavy chain variable region is substituted with K, and the amino acid at position 38 of the light chain variable region is substituted with D. Also, in the other antigen-binding region, the amino acid at position 39 of the heavy chain variable region is substituted with D, and the amino acid at position 38 of the light chain variable region is substituted with K.

### ➢ Antigen-binding region

In some embodiments, the trispecific antibody of the present invention comprises an antigen-binding region specifically binding to BCMA, an antigen-binding region specifically binding to CD3, and an antigen-binding region specifically binding to GPRC5D.

### ➢ Antigen-binding region specifically binding to BCMA

In some embodiments, the antigen-binding region specifically binding to BCMA comprises 3 complementarity determining regions from the heavy chain variable region (HCDRs): HCDR1, HCDR2, and HCDR3. In some embodiments, the antigen-binding region specifically binding to CD3 comprises 3 complementarity determining regions from the light chain variable region (LCDRs): LCDR1, LCDR2, and LCDR3. In some embodiments, the antigen-binding region specifically binding to CD3 comprises 3 complementarity determining regions from the heavy chain variable region (HCDRs) and 3 complementarity determining regions from the light chain variable region (LCDRs).

In some aspects, the antigen-binding region specifically binding to BCMA comprises a heavy chain variable region (VH). In some aspects, the antigen-binding region specifically binding to CD3 comprises a light chain variable region (VL). In some aspects, the antigen-binding region specifically binding to BCMA comprises a heavy chain variable region (VH) and a light chain variable region (VL). In some embodiments, the heavy chain variable region comprises 3 complementarity determining regions (CDRs) from the heavy chain variable region: HCDR1, HCDR2, and HCDR3. In some embodiments, the light chain variable region comprises 3 complementarity determining regions (CDRs) from the light chain variable region: LCDR1, LCDR2, and LCDR3. In some embodiments, the HCDRs and LCDRs are determined by the Kabat scheme (the Kabat numbering scheme described in Sequences of Proteins of Immunological Interest, 5th Ed., Public Health Service, National Institutes of Health, Bethesda, MD (1991)).

In some embodiments, the heavy chain variable region VH of the antigen-binding region specifically binding to BCMA
(i) comprises or consists of an amino acid sequence having at least 90%, 91%, 92%, 93%, 94%, 95%, 96%, 97%, 98%, or 99% identity to an amino acid sequence set forth in SEQ ID NO: 56; or
(ii) comprises or consists of an amino acid sequence set forth in SEQ ID NO: 56; or
(iii) comprises or consists of an amino acid sequence having one or more (preferably no more than 10, and more preferably no more than 5, 4, 3, 2, or 1) amino acid alterations (preferably amino acid substitutions, and more preferably conservative amino acid substitutions) compared with an amino acid sequence set forth in SEQ ID NO: 56, wherein preferably, the amino acid alterations do not occur in the CDRs.

In some embodiments, the light chain variable region VL of the antigen-binding region specifically binding to BCMA
(i) comprises or consists of an amino acid sequence having at least 90%, 91%, 92%, 93%, 94%, 95%, 96%, 97%, 98%, or 99% identity to an amino acid sequence set forth in SEQ ID NO: 60; or
(ii) comprises or consists of an amino acid sequence set forth in SEQ ID NO: 60; or
(iii) comprises or consists of an amino acid sequence having one or more (preferably no more than 10, and more preferably no more than 5, 4, 3, 2, or 1) amino acid alterations (preferably amino acid substitutions, and more preferably conservative amino acid substitutions) compared with an amino acid sequence set forth in SEQ ID NO: 60, wherein preferably, the amino acid alterations do not occur in the CDRs.

In some embodiments, the 3 complementarity determining regions from the heavy chain variable region (HCDRs), HCDR1, HCDR2, and HCDR3, of the antigen-binding region specifically binding to BCMA are three complementarity determining regions contained in the VH, HCDR1, HCDR2, and HCDR3, set forth in SEQ ID NO: 56.

In some embodiments, the 3 complementarity determining regions from the light chain variable region (LCDRs), LCDR1, LCDR2, and LCDR3, of the antigen-binding region specifically binding to BCMA are three complementarity determining regions contained in the VL, LCDR1, LCDR2, and LCDR3, set forth in SEQ ID NO: 60.

In some embodiments, in the antigen-binding region specifically binding to BCMA of the present invention, the HCDR1 comprises or consists of an amino acid sequence set forth in SEQ ID NO: 57; the HCDR2 comprises or consists of an amino acid sequence set forth in SEQ ID NO: 58; the HCDR3 comprises or consists of an amino acid sequence set forth in SEQ ID NO: 59; the LCDR1 comprises or consists of an amino acid sequence set forth in SEQ ID NO: 61; the LCDR2 comprises or consists of an amino acid sequence set forth in SEQ ID NO: 62; and/or the LCDR3 comprises or consists of an amino acid sequence set forth in SEQ ID NO: 63.

In some specific embodiments of the present invention, the antigen-binding region specifically binding to BCMA comprises a VH and a VL, wherein
the VH comprises or consists of an amino acid sequence set forth in SEQ ID NO: 56 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 60 or an amino acid sequence having at least 90%, 91%, 92%, 93%, 94%, 95%, 96%, 97%, 98%, or 99% identity thereto.

In some specific embodiments of the present invention, the antigen-binding region specifically binding to BCMA comprises three complementarity determining regions contained in the VH, HCDR1, HCDR2, and HCDR3, set forth in SEQ ID NO: 56, and three complementarity determining regions contained in the VL, LCDR1, LCDR2, and LCDR3, set forth in SEQ ID NO: 60.

In some specific embodiments of the present invention, the antigen-binding region specifically binding to BCMA comprises: HCDR1 set forth in SEQ ID NO: 57, HCDR2 set forth in SEQ ID NO: 58, HCDR3 set forth in SEQ ID NO: 59, LCDR1 set forth in SEQ ID NO: 61, LCDR2 set forth in SEQ ID NO: 62, and LCDR3 set forth in SEQ ID NO: 63.

In some specific embodiments of the present invention, the antigen-binding region specifically binding to BCMA comprises a VH and a VL, wherein the VH comprises an amino acid sequence set forth in SEQ ID NO: 56, and the VL comprises an amino acid sequence set forth in SEQ ID NO: 60. In some specific embodiments of the present invention, the antigen-binding region specifically binding to BCMA comprises a VH and a VL, wherein the VH consists of an amino acid sequence set forth in SEQ ID NO: 56, and the VL consists of an amino acid sequence set forth in SEQ ID NO: 60.

In one embodiment, the antigen-binding region specifically binding to BCMA is an antigen-binding fragment of an anti-BCMA antibody selected from: an Fab, an Fab', an Fab'-SH, an Fv, a single-chain antibody (e.g., scFv), an (Fab')₂, a single-domain antibody (e.g., VHH), a domain antibody (dAb), and a linear antibody. Preferably, the antigen-binding region specifically binding to BCMA is an Fab.

In some embodiments, the first antigen-binding region contained in the trispecific antibody of the present invention is an Fab fragment specifically binding to BCMA. In some embodiments, the Fab fragment specifically binding to BCMA contained in the trispecific antibody of the present invention is derived from an anti-BCMA antibody.

In some embodiments, the Fab fragment specifically binding to BCMA suitable for use in the trispecific antibody of the present invention comprises a disulfide bond remodeling mutation, for example, the fragment comprises F126C or F126C and C220S in the CH1, and comprises Q124C or Q124C and C214S in the CL.

In some embodiments, the Fab fragment specifically binding to BCMA suitable for use in the trispecific antibody of the present invention comprises a charge mutation, for example, the fragment comprises Q39K in the heavy chain variable region and Q38D in the light chain variable region; or the fragment comprises Q38K in the light chain variable region and Q39D in the heavy chain variable region.

The Fab fragment specifically binding to BCMA suitable for use in the trispecific antibody of the present invention comprises a disulfide bond remodeling mutation and a charge mutation, for example, the fragment comprises Q39K in the heavy chain variable region and F126C or F126C and C220S in the CH1; and the fragment comprises Q38D in the light chain variable region and Q124C or Q124C and C214S in the CL.

The Fab fragment specifically binding to BCMA suitable for use in the trispecific antibody of the present invention comprises a disulfide bond remodeling mutation and a charge mutation, for example, the fragment comprises Q39D in the heavy chain variable region and F126C or F126C and C220S in the CH1; and the fragment comprises Q38K in the light chain variable region and Q124C or Q124C and C214S in the CL.

In some embodiments, the Fab specifically binding to BCMA suitable for use in the trispecific antibody of the present invention comprises an Fab heavy chain comprising a heavy chain variable region and an Fab light chain comprising a light chain variable region,
wherein the heavy chain variable region of the Fab heavy chain
   (i) comprises or consists of an amino acid sequence set forth in SEQ ID NO: 76, or
   (ii) comprises an amino acid sequence having at least 90%, 91%, 92%, 93%, 94%, 95%, 96%, 97%, 98%, or 99% identity to an amino acid sequence set forth in SEQ ID NO: 76, and comprises a Q39K mutation; and/or
the light chain variable region of the Fab light chain
   (i) comprises or consists of an amino acid sequence set forth in SEQ ID NO: 75, or
   (ii) comprises an amino acid sequence having at least 90%, 91%, 92%, 93%, 94%, 95%, 96%, 97%, 98%, or 99% identity to an amino acid sequence set forth in SEQ ID NO: 75, and comprises a Q38D mutation.

### ➢ Antigen-binding region specifically binding to GPRC5D

In some embodiments, the antigen-binding region specifically binding to GPRC5D comprises 3 complementarity determining regions from the heavy chain variable region (HCDRs): HCDR1, HCDR2, and HCDR3. In some embodiments, the antigen-binding region specifically binding to CD3 comprises 3 complementarity determining regions from the light chain variable region (LCDRs): LCDR1, LCDR2, and LCDR3. In some embodiments, the antigen-binding region specifically binding to CD3 comprises 3 complementarity determining regions from the heavy chain variable region (HCDRs) and 3 complementarity determining regions from the light chain variable region (LCDRs).

In some aspects, the antigen-binding region specifically binding to GPRC5D comprises a heavy chain variable region (VH). In some aspects, the antigen-binding region specifically binding to GPRC5D comprises a light chain variable region (VL). In some aspects, the antigen-binding region specifically binding to GPRC5D comprises a heavy chain variable region (VH) and a light chain variable region (VL). In some embodiments, the heavy chain variable region comprises 3 complementarity determining regions (CDRs) from the heavy chain variable region: HCDR1, HCDR2, and HCDR3. In some embodiments, the light chain variable region comprises 3 complementarity determining regions (CDRs) from the light chain variable region: LCDR1, LCDR2, and LCDR3. In some embodiments, the HCDRs and LCDRs are determined by the Kabat scheme (the Kabat numbering scheme described in Sequences of Proteins of Immunological Interest, 5th Ed., Public Health Service, National Institutes of Health, Bethesda, MD (1991)).

In some embodiments, the antigen-binding region is derived from an antibody specifically binding to GPRC5D, such as the antibody specifically binding to GPRC5D as disclosed in PCT/CN2022/076832. In some embodiments, the antigen-binding region comprises 1, 2, 3, 4, 5, or 6 CDRs of the known antibody specifically binding to GPRC5D. In some embodiments, the antigen-binding region comprises 1, 2, and 3 heavy chain variable region CDRs, i.e., HCDR1, HCDR2, and HCDR3, of the known antibody specifically binding to GPRC5D. In some embodiments, the antigen-binding region comprises 1, 2, and 3 light chain variable region CDRs, i.e., LCDR1, LCDR2, and LCDR3, of the known antibody specifically binding to GPRC5D. In some embodiments, the antigen-binding region comprises 3 heavy chain variable region CDRs and 3 light chain variable region CDRs of the known antibody specifically binding to GPRC5D. In some embodiments, the antigen-binding region comprises a heavy chain variable region and a light chain variable region of the known antibody specifically binding to GPRC5D.

In some embodiments, the heavy chain variable region VH of the antigen-binding region specifically binding to GPRC5D
(i) comprises or consists of an amino acid sequence having at least 90%, 91%, 92%, 93%, 94%, 95%, 96%, 97%, 98%, or 99% identity to an amino acid sequence set forth in SEQ ID NO: 40 or 48; or
(ii) comprises or consists of an amino acid sequence set forth in SEQ ID NO: 40 or 48; or
(iii) comprises or consists of an amino acid sequence having one or more (preferably no more than 10, and more preferably no more than 5, 4, 3, 2, or 1) amino acid alterations (preferably amino acid substitutions, and more preferably conservative amino acid substitutions) compared with an amino acid sequence set forth in SEQ ID NO: 40 or 48, wherein preferably, the amino acid alterations do not occur in the CDRs.

In some embodiments, the light chain variable region VL of the antigen-binding region specifically binding to GPRC5D
(i) comprises or consists of an amino acid sequence having at least 90%, 91%, 92%, 93%, 94%, 95%, 96%, 97%, 98%, or 99% identity to an amino acid sequence set forth in SEQ ID NO: 44 or 52; or
(ii) comprises or consists of an amino acid sequence set forth in SEQ ID NO: 44 or 52; or
(iii) comprises or consists of an amino acid sequence having one or more (preferably no more than 10, and more preferably no more than 5, 4, 3, 2, or 1) amino acid alterations (preferably amino acid substitutions, and more preferably conservative amino acid substitutions) compared with an amino acid sequence set forth in SEQ ID NO: 44 or 52, wherein preferably, the amino acid alterations do not occur in the CDRs.

In some embodiments, the 3 complementarity determining regions from the heavy chain variable region (HCDRs), HCDR1, HCDR2, and HCDR3, of the antigen-binding region specifically binding to GPRC5D are three complementarity determining regions contained in the VH, HCDR1, HCDR2, and HCDR3, set forth in any one of SEQ ID NOs: 40 and 48.

In some embodiments, the 3 complementarity determining regions from the light chain variable region (LCDRs), LCDR1, LCDR2, and LCDR3, of the antigen-binding region specifically binding to GPRC5D are three complementarity determining regions contained in the VL, LCDR1, LCDR2, and LCDR3, set forth in any one of SEQ ID NOs: 44 and 52.

In some embodiments, in the antigen-binding region specifically binding to GPRC5D of the present invention, the HCDR1 comprises or consists of an amino acid sequence set forth in SEQ ID NO: 41; the HCDR2 comprises or consists of an amino acid sequence set forth in SEQ ID NO: 42; the HCDR3 comprises or consists of an amino acid sequence set forth in SEQ ID NO: 43; the LCDR1 comprises or consists of an amino acid sequence set forth in SEQ ID NO: 45; the LCDR2 comprises or consists of an amino acid sequence set forth in SEQ ID NO: 46; and/or the LCDR3 comprises or consists of an amino acid sequence set forth in SEQ ID NO: 47; or
the HCDR1 comprises or consists of an amino acid sequence set forth in SEQ ID NO: 49; the HCDR2 comprises or consists of an amino acid sequence set forth in SEQ ID NO: 50; the HCDR3 comprises or consists of an amino acid sequence set forth in SEQ ID NO: 51; the LCDR1 comprises or consists of an amino acid sequence set forth in SEQ ID NO: 53; the LCDR2 comprises or consists of an amino acid sequence set forth in SEQ ID NO: 54; and/or the LCDR3 comprises or consists of an amino acid sequence set forth in SEQ ID NO: 55.

In some specific embodiments of the present invention, the antigen-binding region specifically binding to GPRC5D comprises a VH and a VL, wherein
the VH comprises or consists of an amino acid sequence set forth in SEQ ID NO: 40 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 44 or an amino acid sequence having at least 90%, 91%, 92%, 93%, 94%, 95%, 96%, 97%, 98%, or 99% identity thereto; or
the VH comprises or consists of an amino acid sequence set forth in SEQ ID NO: 48 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 52 or an amino acid sequence having at least 90%, 91%, 92%, 93%, 94%, 95%, 96%, 97%, 98%, or 99% identity thereto.

In some specific embodiments of the present invention, the antigen-binding region specifically binding to GPRC5D comprises three complementarity determining regions contained in the VH, HCDR1, HCDR2, and HCDR3, set forth in SEQ ID NO: 40, and three complementarity determining regions contained in the VL, LCDR1, LCDR2, and LCDR3, set forth in SEQ ID NO: 44. In some specific embodiments of the present invention, the antigen-binding region specifically binding to GPRC5D comprises three complementarity determining regions contained in the VH, HCDR1, HCDR2, and HCDR3, set forth in SEQ ID NO: 48, and three complementarity determining regions contained in the VL, LCDR1, LCDR2, and LCDR3, set forth in SEQ ID NO: 52.

In some specific embodiments of the present invention, the antigen-binding region specifically binding to GPRC5D comprises: HCDR1 set forth in SEQ ID NO: 41, HCDR2 set forth in SEQ ID NO: 42, HCDR3 set forth in SEQ ID NO: 43, LCDR1 set forth in SEQ ID NO: 45, LCDR2 set forth in SEQ ID NO: 46, and LCDR3 set forth in SEQ ID NO: 47. In some specific embodiments of the present invention, the antigen-binding region specifically binding to GPRC5D comprises: HCDR1 set forth in SEQ ID NO: 49, HCDR2 set forth in SEQ ID NO: 50, HCDR3 set forth in SEQ ID NO: 51, LCDR1 set forth in SEQ ID NO: 53, LCDR2 set forth in SEQ ID NO: 54, and LCDR3 set forth in SEQ ID NO: 55.

In some specific embodiments of the present invention, the antigen-binding region specifically binding to GPRC5D comprises a VH and a VL, wherein the VH comprises an amino acid sequence set forth in SEQ ID NO: 40, and the VL comprises an amino acid sequence set forth in SEQ ID NO: 44. In some specific embodiments of the present invention, the antigen-binding region specifically binding to GPRC5D comprises a VH and a VL, wherein the VH consists of an amino acid sequence set forth in SEQ ID NO: 40, and the VL consists of an amino acid sequence set forth in SEQ ID NO: 44.

In some specific embodiments of the present invention, the antigen-binding region specifically binding to GPRC5D comprises a VH and a VL, wherein the VH comprises an amino acid sequence set forth in SEQ ID NO: 48, and the VL comprises an amino acid sequence set forth in SEQ ID NO: 52. In some specific embodiments of the present invention, the antigen-binding region specifically binding to GPRC5D comprises a VH and a VL, wherein the VH consists of an amino acid sequence set forth in SEQ ID NO: 48, and the VL consists of an amino acid sequence set forth in SEQ ID NO: 52.

In one embodiment, the antigen-binding region specifically binding to GPRC5D is an antigen-binding fragment of an anti-GPRC5D antibody selected from: an Fab, an Fab', an Fab'-SH, an Fv, a single-chain antibody (e.g., scFv), an (Fab')₂, a single-domain antibody (e.g., VHH), a domain antibody (dAb), and a linear antibody. Preferably, the antigen-binding region specifically binding to GPRC5D is an Fab.

In some embodiments, the third antigen-binding region contained in the trispecific antibody of the present invention is an Fab fragment specifically binding to GPRC5D. In some embodiments, the Fab fragment specifically binding to GPRC5D contained in the trispecific antibody of the present invention is derived from an anti-GPRC5D antibody.

In some embodiments, the Fab fragment specifically binding to GPRC5D suitable for use in the trispecific antibody of the present invention comprises a disulfide bond remodeling mutation, for example, the fragment comprises F126C or F126C and C220S in the CH1, and comprises Q124C or Q124C and C214S in the CL.

In some embodiments, the Fab fragment specifically binding to GPRC5D suitable for use in the trispecific antibody of the present invention comprises a charge mutation, for example, the fragment comprises Q39K in the heavy chain variable region and Q38D in the light chain variable region; or the fragment comprises Q38K in the light chain variable region and Q39D in the heavy chain variable region.

The Fab fragment specifically binding to GPRC5D suitable for use in the trispecific antibody of the present invention comprises a disulfide bond remodeling mutation and a charge mutation, for example, the fragment comprises Q39K in the heavy chain variable region and F126C or F126C and C220S in the CH1; and the fragment comprises Q38D in the light chain variable region and Q124C or Q124C and C214S in the CL.

The Fab fragment specifically binding to GPRC5D suitable for use in the trispecific antibody of the present invention comprises a disulfide bond remodeling mutation and a charge mutation, for example, the fragment comprises Q39D in the heavy chain variable region and F126C or F126C and C220S in the CH1; and the fragment comprises Q38K in the light chain variable region and Q124C or Q124C and C214S in the CL.

In some embodiments, the Fab specifically binding to GPRC5D suitable for use in the trispecific antibody of the present invention comprises an Fab heavy chain comprising a heavy chain variable region and an Fab light chain comprising a light chain variable region,
wherein the heavy chain variable region of the Fab heavy chain
   (i) comprises or consists of an amino acid sequence set forth in SEQ ID NO: 80, or
   (ii) comprises an amino acid sequence having at least 90%, 91%, 92%, 93%, 94%, 95%, 96%, 97%, 98%, or 99% identity to an amino acid sequence set forth in SEQ ID NO: 80, and comprises a Q39K mutation; and/or
the light chain variable region of the Fab light chain
   (i) comprises or consists of an amino acid sequence set forth in SEQ ID NO: 79, or
   (ii) comprises an amino acid sequence having at least 90%, 91%, 92%, 93%, 94%, 95%, 96%, 97%, 98%, or 99% identity to an amino acid sequence set forth in SEQ ID NO: 79, and comprises a Q38D mutation.

### ➢ Antigen-binding region specifically binding to CD3

In some embodiments, the antigen-binding region specifically binding to CD3 comprises 3 complementarity determining regions from the heavy chain variable region (HCDRs): HCDR1, HCDR2, and HCDR3. In some embodiments, the antigen-binding region specifically binding to CD3 comprises 3 complementarity determining regions from the light chain variable region (LCDRs): LCDR1, LCDR2, and LCDR3. In some embodiments, the antigen-binding region specifically binding to CD3 comprises 3 complementarity determining regions from the heavy chain variable region (HCDRs) and 3 complementarity determining regions from the light chain variable region (LCDRs).

In some aspects, the antigen-binding region specifically binding to CD3 comprises a heavy chain variable region (VH). In some aspects, the antigen-binding region specifically binding to CD3 comprises a light chain variable region (VL). In some aspects, the antigen-binding region specifically binding to CD3 comprises a heavy chain variable region (VH) and a light chain variable region (VL). In some embodiments, the heavy chain variable region comprises 3 complementarity determining regions (CDRs) from the heavy chain variable region: HCDR1, HCDR2, and HCDR3. In some embodiments, the light chain variable region comprises 3 complementarity determining regions (CDRs) from the light chain variable region: LCDR1, LCDR2, and LCDR3. In some embodiments, the HCDRs and LCDRs are determined by the Kabat scheme (the Kabat numbering scheme described in Sequences of Proteins of Immunological Interest, 5th Ed., Public Health Service, National Institutes of Health, Bethesda, MD (1991)).

In some embodiments, the antigen-binding region is derived from an antibody specifically binding to CD3, e.g., the CD3 antibody disclosed in WO2022068809, such as sp34.24 or sp34.87 therein.

In some embodiments, the antigen-binding region comprises 1, 2, 3, 4, 5, or 6 CDRs of the known antibody specifically binding to CD3, e.g., the CD3 antibody disclosed in WO2022068809, such as sp34.24 or sp34.87 therein.

In some embodiments, the antigen-binding region comprises 1, 2, and 3 heavy chain variable region CDRs, i.e., HCDR1, HCDR2, and HCDR3, of the known antibody specifically binding to CD3, e.g., the CD3 antibody disclosed in WO2022068809, such as sp34.24 or sp34.87 therein.

In some embodiments, the antigen-binding region comprises 1, 2, and 3 light chain variable region CDRs, i.e., LCDR1, LCDR2, and LCDR3, of the known antibody specifically binding to CD3, e.g., the CD3 antibody disclosed in WO2022068809, such as sp34.24 or sp34.87 therein.

In some embodiments, the antigen-binding region comprises 3 heavy chain variable region CDRs and 3 light chain variable region CDRs of the known antibody specifically binding to CD3, e.g., the CD3 antibody disclosed in WO2022068809, such as sp34.24 or sp34.87 therein.

In some embodiments, the antigen-binding region comprises a heavy chain variable region and a light chain variable region of the known antibody specifically binding to CD3, e.g., the CD3 antibody disclosed in WO2022068809, such as sp34.24 or sp34.87 therein, and mutations described herein.

In some embodiments, the heavy chain variable region VH of the antigen-binding region specifically binding to CD3
(i) comprises or consists of an amino acid sequence having at least 90%, 91%, 92%, 93%, 94%, 95%, 96%, 97%, 98%, or 99% identity to an amino acid sequence set forth in SEQ ID NO: 64 or 72; or
(ii) comprises or consists of an amino acid sequence set forth in SEQ ID NO: 64 or 72; or
(iii) comprises or consists of an amino acid sequence having one or more (preferably no more than 10, and more preferably no more than 5, 4, 3, 2, or 1) amino acid alterations (preferably amino acid substitutions, and more preferably conservative amino acid substitutions) compared with an amino acid sequence set forth in SEQ ID NO: 64 or 72, wherein preferably, the amino acid alterations do not occur in the CDRs.

In some embodiments, the light chain variable region VL of the antigen-binding region specifically binding to CD3
(i) comprises or consists of an amino acid sequence having at least 90%, 91%, 92%, 93%, 94%, 95%, 96%, 97%, 98%, or 99% identity to an amino acid sequence set forth in SEQ ID NO: 68; or
(ii) comprises or consists of an amino acid sequence set forth in SEQ ID NO: 68; or
(iii) comprises or consists of an amino acid sequence having one or more (preferably no more than 10, and more preferably no more than 5, 4, 3, 2, or 1) amino acid alterations (preferably amino acid substitutions, and more preferably conservative amino acid substitutions) compared with an amino acid sequence set forth in SEQ ID NO: 68, wherein preferably, the amino acid alterations do not occur in the CDRs.

In some embodiments, the 3 complementarity determining regions from the heavy chain variable region (HCDRs), HCDR1, HCDR2, and HCDR3, of the antigen-binding region specifically binding to CD3 are three complementarity determining regions contained in the VH, HCDR1, HCDR2, and HCDR3, set forth in any one of SEQ ID NOs: 64 and 72.

In some embodiments, the 3 complementarity determining regions from the light chain variable region (LCDRs), LCDR1, LCDR2, and LCDR3, of the antigen-binding region specifically binding to CD3 are three complementarity determining regions contained in the VL, LCDR1, LCDR2, and LCDR3, set forth in SEQ ID NO: 68.

In some embodiments, in the antigen-binding region specifically binding to CD3 of the present invention,
the HCDR1 comprises or consists of an amino acid sequence set forth in SEQ ID NO: 65; the HCDR2 comprises or consists of an amino acid sequence set forth in SEQ ID NO: 66; the HCDR3 comprises or consists of an amino acid sequence set forth in SEQ ID NO: 67 or 73; the LCDR1 comprises or consists of an amino acid sequence set forth in SEQ ID NO: 69; the LCDR2 comprises or consists of an amino acid sequence set forth in SEQ ID NO: 70; and/or the LCDR3 comprises or consists of an amino acid sequence set forth in SEQ ID NO: 71.

In some specific embodiments of the present invention, the antigen-binding region specifically binding to CD3 comprises a VH and a VL, wherein
the VH comprises or consists of an amino acid sequence set forth in SEQ ID NO: 64 or 72 or an amino acid sequence having at least 90%, 91%, 92%, 93%, 94%, 95%, 96%, 97%, 98%, or 99% identity thereto, and the VL comprises or consists of an amino acid sequence set forth in SEQ ID NO: 68 or an amino acid sequence having at least 90%, 91%, 92%, 93%, 94%, 95%, 96%, 97%, 98%, or 99% identity thereto.

In some specific embodiments of the present invention, the antigen-binding region specifically binding to CD3 comprises three complementarity determining regions contained in the VH, HCDR1, HCDR2, and HCDR3, set forth in SEQ ID NO: 64 or 72, and three complementarity determining regions contained in the VL, LCDR1, LCDR2, and LCDR3, set forth in SEQ ID NO: 68.

In some specific embodiments of the present invention, the antigen-binding region specifically binding to CD3 comprises: HCDR1 set forth in SEQ ID NO: 65, HCDR2 set forth in SEQ ID NO: 66, HCDR3 set forth in SEQ ID NO: 67 or 73, LCDR1 set forth in SEQ ID NO: 69, LCDR2 set forth in SEQ ID NO: 70, and LCDR3 set forth in SEQ ID NO: 71.

In some specific embodiments of the present invention, the antigen-binding region specifically binding to CD3 comprises a VH and a VL, wherein the VH comprises an amino acid sequence set forth in SEQ ID NO: 64 or 72, and the VL comprises an amino acid sequence set forth in SEQ ID NO: 68. In some specific embodiments of the present invention, the antigen-binding region specifically binding to CD3 comprises a VH and a VL, wherein the VH consists of an amino acid sequence set forth in SEQ ID NO: 64 or 72, and the VL consists of an amino acid sequence set forth in SEQ ID NO: 68.

In one embodiment, the antigen-binding region specifically binding to CD3 is an antigen-binding fragment of an anti-CD3 antibody selected from: an Fab, an Fab', an Fab'-SH, an Fv, a single-chain antibody (e.g., scFv), an (Fab')₂, a single-domain antibody (e.g., VHH), a domain antibody (dAb), and a linear antibody. Preferably, the antigen-binding region specifically binding to CD3 is an Fab.

In some embodiments, the second antigen-binding region contained in the trispecific antibody of the present invention is an Fab fragment specifically binding to CD3. In some embodiments, the Fab fragment specifically binding to CD3 contained in the trispecific antibody of the present invention is derived from an anti-CD3 antibody. In some embodiments, the Fab fragment specifically binding to CD3 suitable for use in the trispecific antibody of the present invention comprises a disulfide bond remodeling mutation, for example, the fragment comprises F126C or F126C and C220S in the CH1, and comprises Q124C or Q124C and C214S in the CL.

In some embodiments, the Fab fragment specifically binding to CD3 suitable for use in the trispecific antibody of the present invention comprises a charge mutation, for example, the fragment comprises Q39K in the heavy chain variable region and Q38D in the light chain variable region; or the fragment comprises Q38K in the light chain variable region and Q39D in the heavy chain variable region.

The Fab fragment specifically binding to CD3 suitable for use in the trispecific antibody of the present invention comprises a disulfide bond remodeling mutation and a charge mutation, for example, the fragment comprises Q39K in the heavy chain variable region and F126C or F126C and C220S in the CH1; and the fragment comprises Q38D in the light chain variable region and Q124C or Q124C and C214S in the CL.

The Fab fragment specifically binding to CD3 suitable for use in the trispecific antibody of the present invention comprises a disulfide bond remodeling mutation and a charge mutation, for example, the fragment comprises Q39D in the heavy chain variable region and F126C or F126C and C220S in the CH1; and the fragment comprises Q38K in the light chain variable region and Q124C or Q124C and C214S in the CL.

In some embodiments, the Fab specifically binding to CD3 suitable for use in the trispecific antibody of the present invention comprises an Fab heavy chain comprising a heavy chain variable region and an Fab light chain comprising a light chain variable region,
wherein the heavy chain variable region of the Fab heavy chain
   (i) comprises or consists of an amino acid sequence set forth in SEQ ID NO: 77 or 78, or
   (ii) comprises an amino acid sequence having at least 90%, 91%, 92%, 93%, 94%, 95%, 96%, 97%, 98%, or 99% identity to an amino acid sequence set forth in SEQ ID NO: 77 or 78, and comprises a Q39D mutation; and/or
the light chain variable region of the Fab light chain
   (i) comprises or consists of an amino acid sequence set forth in SEQ ID NO: 74, or
   (ii) comprises an amino acid sequence having at least 90%, 91%, 92%, 93%, 94%, 95%, 96%, 97%, 98%, or 99% identity to an amino acid sequence set forth in SEQ ID NO: 74, and comprises a Q38K mutation.

### ➢ Fc dimer suitable for use in trispecific antibody of the present invention

In one embodiment, two Fc regions in the trispecific antibody of the present invention form a dimeric Fc by dimerization. Preferably, the two Fc regions form a heterodimeric Fc by heterodimerization.

In some embodiments, the first Fc region and the second Fc region are different and can form a heterodimeric Fc scaffold by dimerization.

In some embodiments, the Fc region encompasses Fc regions of native sequences and variant Fc regions. The Fc regions of native sequences encompass naturally occurring Fc sequences of various immunoglobulins, such as Fc regions of various Ig subtypes and allotypes thereof (Gestur Vidarsson et al., IgG subclasses and allotypes: from structure to effector functions, 20 October 2014, doi: 10.3389/fimmu.2014.00520). In some embodiments, the Fc region is a human IgG Fc, such as human IgG1 Fc, human IgG2 Fc, human IgG3 Fc, or human IgG4 Fc. In one embodiment, the Fc region comprises or consists of an amino acid sequence set forth in SEQ ID NO: 87 or 88, or an amino acid sequence having at least 90% identity, e.g., 95%, 96%, 97%, 99% or more identity, thereto.

In some embodiments, the Fc region of the present invention comprises a CH2 and a CH3 of the antibody. In some embodiments, the antibody Fc region may also bear an IgG hinge region or a portion of the IgG hinge region, such as an IgG1 hinge region or a portion of the IgG1 hinge region, at the N-terminus. The hinge region may comprise mutations.

As understood by those skilled in the art, to facilitate the formation of the multispecific antibody of the present invention as a heterodimer, the Fc region contained in the multispecific antibody of the present invention may comprise mutations that favor the heterodimerization of the first Fc region and the second Fc region. In one embodiment, mutations are introduced into the CH3 regions of the two Fc regions.

Methods for facilitating heterodimerization of Fc regions are known in the art. For example, the CH3 region of the first Fc region and the CH3 region of the second Fc region are engineered in a complementary manner, such that each CH3 region (or the heavy chain comprising it) can be no longer homodimerized with itself but forced to be heterodimerized with other CH3 regions that are complementarily engineered (such that the CH3 regions of the first and second Fc regions are heterodimerized and no homodimers are formed between the two first CH3 regions or the two second CH3 regions). Preferably, corresponding Knob and Hole mutations are introduced into the first Fc region and the second Fc region based on the Knob-in-Hole technique.

In a particular embodiment, in the CH3 region of one Fc region, the threonine residue at position 366 is substituted with a tryptophan residue (T366W) (knob mutation); while in the CH3 region of the other Fc region, the tyrosine residue at position 407 is substituted with a valine residue (Y407V) (hole mutation), optionally the threonine residue at position 366 is substituted with a serine residue (T366S), and/or the leucine residue at position 368 is substituted with an alanine residue (L368A) (numbering according to EU index).

In yet another embodiment, in the CH3 region of one Fc region, the threonine residue at position 366 is substituted with a tryptophan residue (T366W) and the serine residue at position 354 is substituted with a cysteine residue (S354C) or the glutamic acid residue at position 356 is substituted with a cysteine residue (E356C) (in particular, the serine residue at position 354 is substituted with a cysteine residue); while in the CH3 region of the other Fc region, the tyrosine residue at position 407 is substituted with a valine residue (Y407V) (hole mutation), optionally the threonine residue at position 366 is substituted with a serine residue (T366S) and the leucine residue at position 368 is substituted with an alanine residue (L368A) (numbering according to EU index), and optionally the tyrosine residue at position 349 is substituted with a cysteine residue (Y349C) (numbering according to EU index).

In a specific embodiment, one Fc region comprises an amino acid substitution T366W, and the other Fc region comprises amino acid substitutions T366S, L368A, and Y407V (numbering according to EU index).

In a specific embodiment, one Fc region comprises amino acid substitutions S354C and T366W, and the other Fc region comprises amino acid substitutions Y349C, T366S, L368A, and Y407V (numbering according to EU index). Accordingly, in a specific embodiment, the trispecific antibody of the present invention comprises two Fc regions which are heterodimerized, wherein one Fc region polypeptide comprises or consists of an amino acid sequence set forth in SEQ ID NO: 91, and the other Fc region polypeptide comprises or consists of an amino acid sequence set forth in SEQ ID NO: 92.

Accordingly, in a specific embodiment, the trispecific antibody of the present invention comprises two Fc regions which are heterodimerized, wherein one Fc region polypeptide comprises an amino acid sequence having at least 85%, 90%, 95%, 96%, 97%, 98%, or 99% identity to an amino acid sequence set forth in SEQ ID NO: 91, and the other Fc region polypeptide comprises an amino acid sequence having at least 85%, 90%, 95%, 96%, 97%, 98%, or 99% identity to an amino acid sequence set forth in SEQ ID NO: 92.

Accordingly, in a specific embodiment, the trispecific antibody of the present invention comprises two Fc regions which are heterodimerized, wherein one Fc region polypeptide comprises an amino acid sequence having at least 85%, 90%, 95%, 96%, 97%, 98%, or 99% identity to an amino acid sequence set forth in SEQ ID NO: 92 and comprises a mutation T366W, and the other Fc region polypeptide comprises an amino acid sequence having at least 85%, 90%, 95%, 96%, 97%, 98%, or 99% identity to an amino acid sequence set forth in SEQ ID NO: 91 and comprises mutations T366S, L368A, and Y407V.

In some embodiments, the Fc region further comprises additional mutations that favor the purification of the heterodimer.

The binding molecule of the present invention, such as the Fc region of the antibody, may also be mutated to obtain desired properties. Mutations for the Fc region are known in the art.

In one embodiment, the Fc region is modified in properties of the effector function of the Fc region (e.g., complement activation function of the Fc region). In one embodiment, the effector function has been reduced or eliminated relative to a wild-type isotype Fc region. In one embodiment, the effector function is reduced or eliminated by using a method selected from: using an Fc isotype that naturally has a reduced or an eliminated effector function, and performing Fc region modification.

In a preferred embodiment, the Fc region has reduced effector functions mediated by the Fc region, such as reduced or eliminated ADCC or ADCP or CDC effector functions, for example, comprising mutations for achieving the above functions.

As understood by those skilled in the art, binding molecules, e.g., antibody molecules, of the present invention can further comprise modifications in the Fc domain that alter binding affinity for one or more Fc receptors, according to the desired use of the binding molecules, e.g., antibody molecules, of the present invention. In one embodiment, the Fc receptor is an Fcγ receptor, in particular a human Fcγ receptor. In some embodiments, the Fc region comprises a mutation that reduces binding to the Fcγ receptor. For example, in some embodiments, the Fc region for use in the present invention has an L234A/L235A mutation that reduces binding to the Fcγ receptor. In yet another preferred embodiment, the Fc fragment may have a mutation that leads to an increased serum half-life, e.g., a mutation that improves binding of the Fc fragment to FcRn.

In some embodiments, the Fc region comprising a mutation that reduces binding to the Fcγ receptor comprises or consists of an amino acid sequence set forth in SEQ ID NO: 89 or 90 or an amino acid sequence having at least 90% identity, e.g., 95%, 96%, 97%, 99% or more identity, thereto.

In some embodiments, the Fc region comprises an amino acid sequence having at least 90% identity, e.g., 95%, 96%, 97%, 99% or more identity, to an amino acid sequence set forth in SEQ ID NO: 89 or 90 and comprises an L234A/L235A mutation.

In a preferred embodiment, the heterodimeric Fc suitable for use in the trispecific antibody of the present invention comprises two Fc regions, wherein
one Fc region comprises or consists of an amino acid sequence set forth in SEQ ID NO: 93, and the other Fc region comprises or consists of an amino acid sequence set forth in SEQ ID NO: 94; or
one Fc region polypeptide comprises an amino acid sequence having at least 85%, 90%, 95%, 96%, 97%, 98%, or 99% identity to an amino acid sequence set forth in SEQ ID NO: 94 and comprises mutations L234A/L235A and T366W, and the other Fc region polypeptide comprises an amino acid sequence having at least 85%, 90%, 95%, 96%, 97%, 98%, or 99% identity to an amino acid sequence set forth in SEQ ID NO: 93 and comprises mutations L234A/L235A, T366S, L368A and Y407V.

### ➢ Trispecific antibody structures and exemplary trispecific antibodies of the present invention

In some embodiments, the present invention provides a trispecific antibody comprising a first Fab specifically binding to BCMA, an Fab specifically binding to CD3, an Fab specifically binding to GPRC5D, and an Fc heterodimer.

In some embodiments, the present invention provides a trispecific antibody comprising a first Fab specifically binding to BCMA, an Fab specifically binding to CD3, an Fab specifically binding to GPRC5D, and an Fc heterodimer.

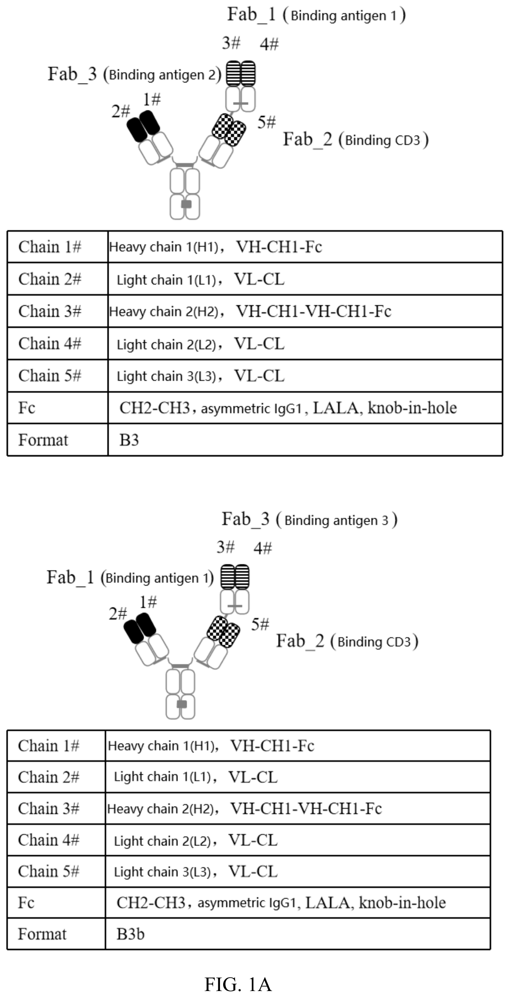

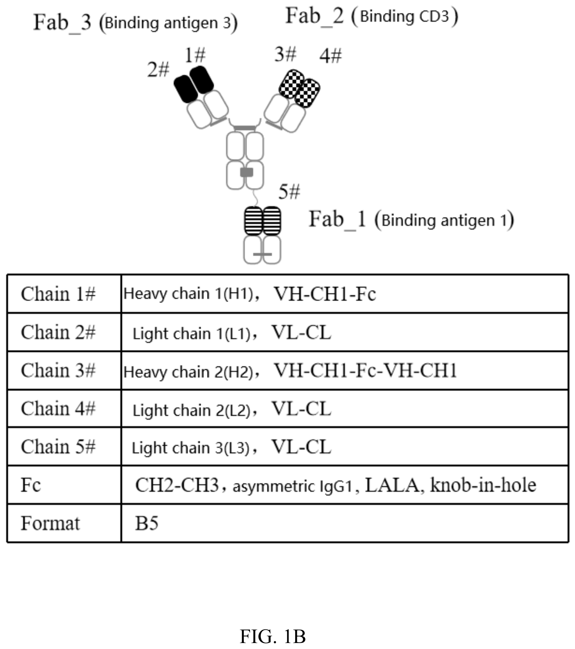

In some preferred embodiments, the trispecific antibody of the present invention comprises a first Fab specifically binding to a first antigen, a second Fab specifically binding to a second antigen, a third Fab specifically binding to a third antigen, and an Fc heterodimer, wherein the first antigen is selected from one of BCMA, CD3, and GPRC5D, the second antigen is selected from one of BCMA, CD3, and GPRC5D, the third antigen is selected from one of BCMA, CD3, and GPRC5D, and the first, second, and third antigens are different, wherein
(1) the first Fab specifically binding to the first antigen is fused to a CH2 or a hinge region of one of Fc regions of the Fc heterodimer (a first Fc region, e.g., an Fc region comprising a Knob mutation or an Fc region comprising a hole mutation) at the C-terminus of a CH1 of an Fab heavy chain;
   the second Fab specifically binding to the second antigen is fused to a CH2 or a hinge region of another Fc region of the Fc heterodimer (a second Fc region, e.g., an Fc region comprising a hole mutation or an Fc region comprising a knob) at the C-terminus of a CH1 of an Fab heavy chain; and
   the third Fab specifically binding to the third antigen is fused to the N-terminus of a VH of the Fab heavy chain of the second Fab at the C-terminus of a CH1 of an Fab heavy chain; or
   the third Fab specifically binding to the third antigen is fused to a CH2 or a hinge region of one of Fc regions of the Fc heterodimer (a first Fc region, e.g., an Fc region comprising a Knob mutation or an Fc region comprising a hole mutation) at the C-terminus of a CH1 of an Fab heavy chain;
   the second Fab specifically binding to the second antigen is fused to a CH2 or a hinge region of another Fc region of the Fc heterodimer (a second Fc region, e.g., an Fc region comprising a hole mutation or an Fc region comprising a knob) at the C-terminus of a CH1 of an Fab heavy chain; and
   the first Fab specifically binding to the first antigen is fused to the N-terminus of a VH of the Fab heavy chain of the second Fab at the C-terminus of a CH1 of an Fab heavy chain;
   such as the structure shown in FIG. 1A;
(2) the first Fab specifically binding to the first antigen is fused to a CH2 or a hinge region of one of Fc regions of the Fc heterodimer (a first Fc region, e.g., an Fc region comprising a Knob mutation or an Fc region comprising a hole) at the C-terminus of a CH1 of an Fab heavy chain;
   the second Fab specifically binding to the second antigen is fused to a CH2 or a hinge region of another Fc region of the Fc heterodimer (a second Fc region, e.g., an Fc region comprising a hole mutation or an Fc region comprising a knob) at the C-terminus of a CH1 of an Fab heavy chain; and
   the third Fab specifically binding to the third antigen is fused to the C-terminus of the Fc region fused to the second Fab at the N-terminus of a VH of an Fab heavy chain; or
   the third Fab specifically binding to the third antigen is fused to a CH2 or a hinge region of one of Fc regions of the Fc heterodimer (a first Fc region, e.g., an Fc region comprising a Knob mutation or an Fc region comprising a hole) at the C-terminus of a CH1 of an Fab heavy chain;
   the second Fab specifically binding to the second antigen is fused to a CH2 or a hinge region of another Fc region of the Fc heterodimer (a second Fc region, e.g., an Fc region comprising a hole mutation or an Fc region comprising a knob) at the C-terminus of a CH1 of an Fab heavy chain; and
   the first Fab specifically binding to the first antigen is fused to the C-terminus of the Fc region fused to the second Fab at the N-terminus of a VH of an Fab heavy chain;
   such as the structure shown in FIG. 1B.

In some embodiments, the second antigen is CD3. In some embodiments, the first and third antigens are GPRC5D or BCMA, respectively, or the third and first antigens are GPRC5D or BCMA, respectively.

In some embodiments, the first antigen is BCMA, the second antigen is CD3, and the third antigen is GPRC5D; or the first antigen is GPRC5D, the second antigen is CD3, or the third antigen is BCMA.

In some embodiments, the fusion includes direct fusion or fusion through a linker. In some embodiments, the linker is (GGGGS)n, wherein n = 1, 2, 3, or 4.

In some embodiments, the first Fc region comprises a knob mutation, and the second Fc region comprises a hole mutation. In some embodiments, the first Fc region comprises a hole mutation, and the second Fc region comprises a knob mutation. In some embodiments, one or both of the first and second Fc regions comprise an L234A/L235A mutation. In some embodiments, the first Fc region comprises a knob mutation and L234A/L235A mutation, and the second Fc region comprises a hole mutation and L234A/L235A mutation. In some embodiments, the first Fc region comprises a hole mutation and L234A/L235A mutation, and the second Fc region comprises a knob mutation and L234A/L235A mutation.

In some embodiments, the second Fab comprises a charge mutation. In some embodiments, one or both of the first Fab or the third Fab comprise a charge mutation and a disulfide bond remodeling mutation.

In some embodiments, the second Fab comprises a charge mutation, and one of the first or third Fab comprises a charge mutation and a disulfide bond remodeling mutation. In some embodiments, the two Fabs comprising a charge mutation comprise opposite charge mutations.

In some embodiments, the second Fab comprises a charge mutation comprising a Q38K mutation in the Fab light chain and a Q39D mutation in the Fab heavy chain. In some embodiments, the first or third Fab comprises a charge mutation and a disulfide bond remodeling mutation comprising Q39K and F126C mutations in the Fab heavy chain and Q38D and Q124C mutations in the Fab light chain. In some embodiments, the first or third Fab comprises a charge mutation and a disulfide bond remodeling mutation comprising Q39K, F126C, and C220S mutations in the Fab heavy chain and Q38D, Q124C, and C214S mutations in the Fab light chain.

In some embodiments, the second Fab comprises a charge mutation comprising a Q38D mutation in the Fab light chain and a Q39K mutation in the Fab heavy chain. In some embodiments, the first or third Fab comprises a charge mutation and a disulfide bond remodeling mutation comprising Q39D and F126C mutations in the Fab heavy chain and Q38K and Q124C mutations in the Fab light chain. In some embodiments, the first or third Fab comprises a charge mutation and a disulfide bond remodeling mutation comprising Q39D, F126C, and C220S mutations in the Fab heavy chain and Q38K, Q124C, and C214S mutations in the Fab light chain.

In some embodiments, the first or third Fab comprising a charge mutation and a disulfide bond remodeling mutation is fused to the second Fab, or to the second Fab through the Fc region.

In some specific embodiments, the trispecific antibody of the present invention is a left-right asymmetric IgG-like pentamer consisting of 5 polypeptide chains and consists of the following peptide chains:
a peptide chain 1# sequentially comprising an Fab heavy chain specifically binding to GPRC5D and an Fc domain,
a peptide chain 2# comprising an Fab light chain specifically binding to GPRC5D,
a peptide chain 3# sequentially comprising an Fab heavy chain specifically binding to BCMA, an Fab heavy chain specifically binding to CD3, and an Fc domain,
a peptide chain 4# comprising an Fab light chain specifically binding to BCMA, and
a peptide chain 5# comprising an Fab light chain specifically binding to CD3,
wherein the Fab heavy chain of the peptide chain 1# is paired with the Fab light chain of the peptide chain 2# to form the third Fab, and the two Fab heavy chains of the peptide chain 3# are paired with the Fab light chains of the peptide chain 4# and the peptide chain 5# to form the first Fab and the second Fab, respectively, for example, as shown in FIG. 1A.

In some specific embodiments, the peptide chain 1# comprises or consists of an amino acid sequence set forth in SEQ ID NO: 5, or an amino acid sequence having at least 85%, 90%, 91%, 92%, 93%, 94%, 95%, 96%, 97%, 98%, or 99% identity to an amino acid sequence set forth in SEQ ID NO: 5;
the peptide chain 2# comprises or consists of an amino acid sequence set forth in SEQ ID NO: 4, or an amino acid sequence having at least 85%, 90%, 91%, 92%, 93%, 94%, 95%, 96%, 97%, 98%, or 99% identity to an amino acid sequence set forth in SEQ ID NO: 4;
the peptide chain 3# comprises or consists of an amino acid sequence set forth in SEQ ID NO: 3 or 6, or an amino acid sequence having at least 85%, 90%, 91%, 92%, 93%, 94%, 95%, 96%, 97%, 98%, or 99% identity to an amino acid sequence set forth in SEQ ID NO: 3 or 6;
the peptide chain 4# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2; and/or
the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.

In some specific embodiments, the peptide chain 1# comprises or consists of an amino acid sequence set forth in SEQ ID NO: 15, or an amino acid sequence having at least 85%, 90%, 91%, 92%, 93%, 94%, 95%, 96%, 97%, 98%, or 99% identity to an amino acid sequence set forth in SEQ ID NO: 15;
the peptide chain 2# comprises or consists of an amino acid sequence set forth in SEQ ID NO: 14, or an amino acid sequence having at least 85%, 90%, 91%, 92%, 93%, 94%, 95%, 96%, 97%, 98%, or 99% identity to an amino acid sequence set forth in SEQ ID NO: 14;
the peptide chain 3# comprises or consists of an amino acid sequence set forth in SEQ ID NO: 3 or 6, or an amino acid sequence having at least 85%, 90%, 91%, 92%, 93%, 94%, 95%, 96%, 97%, 98%, or 99% identity to an amino acid sequence set forth in SEQ ID NO: 3 or 6;
the peptide chain 4# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2; and/or
the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.

In some specific embodiments, the trispecific antibody of the present invention is a left-right asymmetric IgG-like pentamer consisting of 5 polypeptide chains and consists of the following peptide chains:
a peptide chain 1# sequentially comprising an Fab heavy chain specifically binding to BCMA and an Fc domain,
a peptide chain 2# comprising an Fab light chain specifically binding to BCMA,
a peptide chain 3# sequentially comprising an Fab heavy chain specifically binding to GPRC5D, an Fab heavy chain specifically binding to CD3, and an Fc domain,
a peptide chain 4# comprising an Fab light chain specifically binding to GPRC5D, and
a peptide chain 5# comprising an Fab light chain specifically binding to CD3,
wherein the Fab heavy chain of the peptide chain 1# is paired with the Fab light chain of the peptide chain 2# to form the first Fab, and the two Fab heavy chains of the peptide chain 3# are paired with the Fab light chains of the peptide chain 4# and the peptide chain 5# to form the third Fab and the second Fab, respectively, for example, as shown in FIG. 1A.

In some specific embodiments, the peptide chain 1# comprises or consists of an amino acid sequence set forth in SEQ ID NO: 11, or an amino acid sequence having at least 85%, 90%, 91%, 92%, 93%, 94%, 95%, 96%, 97%, 98%, or 99% identity to an amino acid sequence set forth in SEQ ID NO: 11;
the peptide chain 2# comprises or consists of an amino acid sequence set forth in SEQ ID NO: 10, or an amino acid sequence having at least 85%, 90%, 91%, 92%, 93%, 94%, 95%, 96%, 97%, 98%, or 99% identity to an amino acid sequence set forth in SEQ ID NO: 10;
the peptide chain 3# comprises or consists of an amino acid sequence set forth in SEQ ID NO: 9, or an amino acid sequence having at least 85%, 90%, 91%, 92%, 93%, 94%, 95%, 96%, 97%, 98%, or 99% identity to an amino acid sequence set forth in SEQ ID NO: 9;
the peptide chain 4# comprises or consists of an amino acid sequence set forth in SEQ ID NO: 8, or an amino acid sequence having at least 85%, 90%, 91%, 92%, 93%, 94%, 95%, 96%, 97%, 98%, or 99% identity to an amino acid sequence set forth in SEQ ID NO: 8; and
the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.

In some specific embodiments, the trispecific antibody of the present invention is a left-right asymmetric IgG-like pentamer consisting of 5 polypeptide chains and consists of the following peptide chains:
a peptide chain 1# sequentially comprising an Fab heavy chain specifically binding to GPRC5D and an Fc domain, a peptide chain 2# comprising an Fab light chain specifically binding to GPRC5D,
a peptide chain 3# sequentially comprising an Fab heavy chain specifically binding to CD3, an Fc domain, and an Fab heavy chain of the antigen-binding region specifically binding to BCMA,
a peptide chain 4# comprising an Fab light chain specifically binding to CD3, and
a peptide chain 5# comprising an Fab light chain of the antigen-binding region specifically binding to BCMA,
wherein the Fab heavy chain of the peptide chain 1# is paired with the Fab light chain of the peptide chain 2# to form the third Fab, and the two Fab heavy chains of the peptide chain 3# are paired with the Fab light chains of the peptide chain 4# and the peptide chain 5# to form the second Fab and the first Fab, respectively, for example, as shown in FIG. 1B.

In some specific embodiments, the peptide chain 1# comprises or consists of an amino acid sequence set forth in SEQ ID NO: 5, or an amino acid sequence having at least 85%, 90%, 91%, 92%, 93%, 94%, 95%, 96%, 97%, 98%, or 99% identity to an amino acid sequence set forth in SEQ ID NO: 5;
the peptide chain 2# comprises or consists of an amino acid sequence set forth in SEQ ID NO: 4, or an amino acid sequence having at least 85%, 90%, 91%, 92%, 93%, 94%, 95%, 96%, 97%, 98%, or 99% identity to an amino acid sequence set forth in SEQ ID NO: 4;
the peptide chain 3# comprises or consists of an amino acid sequence set forth in SEQ ID NO: 7, or an amino acid sequence having at least 85%, 90%, 91%, 92%, 93%, 94%, 95%, 96%, 97%, 98%, or 99% identity to an amino acid sequence set forth in SEQ ID NO: 7;
the peptide chain 4# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1; and/or
the peptide chain 5# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2.

### II. Production and purification of multispecific antibody

In one embodiment, the present invention provides a method for preparing the antibody molecule or the fragment or the chain thereof of the present invention, wherein the method comprises culturing the host cell under conditions suitable for expressing the nucleic acid encoding the antibody molecule or the fragment or the chain thereof of the present invention, and optionally isolating the antibody or the fragment or the chain thereof. In a certain embodiment, the method further comprises recovering the antibody molecule or the fragment or the chain thereof of the present invention from the host cell.

Polynucleotides encoding the polypeptide chain of the antibody of the present invention can be inserted into one or more vectors for further cloning and/or expression in host cells. Methods known to those skilled in the art can be used to construct expression vectors. Once the expression vector comprising one or more nucleic acid molecules of the present invention has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, liposome-based transfection, or other conventional techniques.

The antibody molecule prepared as described herein can be purified by known prior art such as high performance liquid chromatography, ion exchange chromatography, gel electrophoresis, affinity chromatography, and size exclusion chromatography. The actual conditions used to purify a particular protein also depend on factors such as net charge, hydrophobicity, and hydrophilicity, and these will be apparent to those skilled in the art. The purity of the antibody molecule of the present invention can be determined by any one of a variety of well-known analytical methods including size exclusion chromatography, gel electrophoresis, high performance liquid chromatography, and the like.

### III. Immunoconjugate

In some embodiments, the present invention provides an immunoconjugate comprising any of the antibodies provided herein and other substances, such as a therapeutic agent or a label. In some embodiments, the therapeutic agent may be a therapeutic agent suitable for forming an immunoconjugate with an antibody. In some embodiments, the therapeutic agent is a therapeutic agent selected from any one of the following categories (i)-(iii): (i) drugs that enhance antigen presentation (e.g., tumor antigen presentation); (ii) drugs that enhance effector cell responses (e.g., B cell and/or T cell activation and/or mobilization); (iii) drugs that reduce immunosuppression; and (iv) drugs that have anti-tumor effects. In some embodiments, the therapeutic agent is a chemotherapeutic agent.

In some embodiments, the immunoconjugate is antibody-drug conjugates (ADC).

### IV. Nucleic acid and host cell

The present invention provides a nucleic acid encoding any chain or any monomer or domain or antigen-binding region of the multispecific antibody, e.g., trispecific antibody, of the present invention. Polynucleotide sequences encoding each chain can be generated using methods well known in the art. In addition, the polynucleotide and the nucleic acid of the present invention can comprise a segment encoding a secretion signal peptide and are operably linked to an antibody or a protein encoding the multispecific antibody, e.g., trispecific antibody, of the present invention so that secretory expression of the multispecific antibody, e.g., trispecific antibody, and each chain thereof of the present invention can be directed.

The present invention also provides a vector comprising the nucleic acid disclosed herein. In one embodiment, the vector is an expression vector, such as a eukaryotic expression vector. The vector includes, but is not limited to, a virus, a plasmid, a cosmid, a λ phage, or a yeast artificial chromosome (YAC). In a preferred embodiment, the expression vector of the present invention is a pCNDA vector, such as a pCDNA3.1 expression vector.

The present invention further provides a host cell comprising the nucleic acid or the vector. Host cells suitable for replicating and supporting the expression of the multispecific antibody, e.g., the trispecific antibody, of the present invention are well known in the art. Such cells can be transfected or transduced with a particular expression vector, and a large number of vector-containing cells can be cultivated for inoculation in large-scale fermenters, so as to obtain sufficient multispecific antibodies, e.g., trispecific antibodies, for clinical application.

In one embodiment, the host cell is eukaryotic. In another embodiment, the host cell is selected from a yeast cell and a mammalian cell (e.g., a CHO cell or a 293 cell, such as an Expi293 cell or an HEK293 cell). Examples of available mammalian host cell lines include SV40 transformed monkey kidney CV1 lines (COS-7), human embryonic kidney lines (293 or 293T cells, as described, for example, in Graham et al., JGenVirol 36,59 (1977)), baby hamster kidney cells (BHK), mouse Sertoli cells (TM4 cells, as described, for example, in Mather, BiolReprod 23,243-251 (1980)), monkey kidney cells (CV1), African green monkey kidney cells (VERO-76), human cervical cancer cells (HELA), canine kidney cells (MDCK), buffalo rat liver cells (BRL3A), human lung cells (W138), human liver cells (HepG2), mouse mammary tumor cells (MMT060562), TRI cells (as described, for example, in Mather et al., AnnalsN.Y.AcadSci 383,44-68 (1982)), MRC5 cells, and FS4 cells. Other available mammalian host cell lines include Chinese hamster ovary (CHO) cells, including dhfr-CHO cells (Urlaub et al., Proc. Natl. Acad. Sci. USA 77,4216 (1980)), and myeloma cell lines such as YO, NS0, P3X63, and Sp2/0. In one embodiment, the host cell is a eukaryotic cell, preferably a mammalian cell such as a Chinese hamster ovary (CHO) cell, a human embryonic kidney (HEK) cell, or a lymphocyte (e.g., Y0, NS0, and Sp20 cells).

### V. Pharmaceutical composition, pharmaceutical combination, and kit

In one aspect, the present invention provides a composition or a drug or a formulation, e.g. a pharmaceutical composition comprising the trispecific antibody molecule of the present invention, and optionally a pharmaceutical supplementary material. In some embodiments, the pharmaceutical supplementary material is a pharmaceutically acceptable carrier.

The composition of the present invention may be in a variety of forms. These forms include, for example, liquid, semi-solid and solid dosage forms, such as liquid solutions (e.g., injectable solutions and infusible solutions), dispersions or suspensions, liposomes, and suppositories. The preferred form depends on the intended mode of administration and therapeutic use. Commonly preferred compositions are in the form of injectable solutions or infusible solutions. The preferred mode of administration is parenteral injection or infusion. As used herein, the phrases "parenteral administration" and "administered parenterally" mean modes of administration other than enteral and topical administration, typically by injection, and include, but are not limited to, intravenous, intramuscular, intra-arterial, intradermal, intraperitoneal, transtracheal, subcutaneous injection and infusion.

In one preferred embodiment, the antibody molecule is administered by intravenous infusion or injection. In another preferred embodiment, the antibody molecule is administered by intramuscular, intraperitoneal or subcutaneous injection.

In some embodiments, the antibody molecule of the present invention is the only active ingredient in the pharmaceutical composition. In other embodiments, the pharmaceutical composition may comprise the antibody molecule described herein and one or more additional therapeutic agents.

The pharmaceutical composition of the present invention may comprise a "therapeutically effective amount" or a "prophylactically effective amount" of the antibody molecule of the present invention.

A kit comprising the antibody molecule described herein is also within the scope of the present invention. The kit may include one or more other elements, including, for example, a package insert; other reagents, such as a marker or a reagent for coupling; a pharmaceutically acceptable carrier; and a device or other materials for administration to a subject.

In another aspect, the present invention also provides a pharmaceutical combination or a pharmaceutical combination product comprising the antibody molecule described herein and one or more additional therapeutic agents.

The present invention further provides a kit of parts comprising the pharmaceutical combination, wherein, for example, the kit of parts comprises in the same package:
- a first container containing a pharmaceutical composition comprising the trispecific antibody of the present invention; and
- a second container containing a pharmaceutical composition comprising additional therapeutic agents.

Additional therapeutic agents suitable for use in the pharmaceutical composition and the pharmaceutical combination of the present invention may be selected from any one of the following categories (i)-(iii): (i) drugs that enhance antigen presentation (e.g., tumor antigen presentation); (ii) drugs that enhance effector cell responses (e.g., B cell and/or T cell activation and/or mobilization); (iii) drugs that reduce immunosuppression; and (iv) drugs that have anti-tumor effects. In some embodiments, the additional therapeutic agent is a chemotherapeutic agent.

### VI. Use and method of the molecule of the present invention

In one aspect, the present invention provides *in vivo* and *in vitro* use and method for the use of the antibody molecule of the present invention.

In some embodiments, the use and the method of the present invention involve the application of the antibody molecule of the present invention *in vivo* and/or *in vitro* in:
- binding to GPRC5D antigen, including GPRC5D antigen expressed on the surface of cells, with a high affinity;
- targeting T cells (e.g., CD4+ and/or CD8+ T cells) to cells that express GPRC5D on the surface, in particular GPRC5D-positive tumor cells;
- targeting T cells (e.g., CD4+ and/or CD8+ T cells) to cells that express BCMA on the surface, in particular BCMA-positive tumor cells;
- activating the CD3 downstream signaling pathway of T cells;
- mediating the killing effect of T cells on GPRC5D-positive tumor cells and/or BCMA-positive tumor cells;
- inducing T cells to release cytokines such as TNF-α, IFN-γ, and IL-2;
- inhibiting or killing GPRC5D-positive and/or BCMA-positive tumor cells; or
- treating GPRC5D-positive and/or BCMA-positive tumors such as multiple myeloma, for example, treating a BCMA-positive patient population, a GPRC5D-positive patient population, or a GPRC5D-positive patient population in which BCMA is lost upon binding of an anti-BCMA molecule; or
- avoiding tumor recurrence mediated by BCMA escape, such as recurrence of multiple myeloma.

In some embodiments, the present invention provides a method for preventing or treating a GPRC5D- and/or BCMA-related disease in a subject, comprising administering to the subject the trispecific antibody, or the immunoconjugate, the composition or the drug or the formulation comprising the same of the present invention. In some embodiments, the present invention provides a method for the use described above in a subject, comprising administering to the subject the trispecific antibody, or the immunoconjugate, the composition or the drug or the formulation comprising the same of the present invention.

In some embodiments, the disease is, for example, a tumor, e.g., a cancer. The cancer may be at an early, intermediate or advanced stage, or may be a metastatic cancer. In some embodiments, the cancer is a GPRC5D-single-positive cancer, a BCMA-single-positive cancer, a GPRC5D/BCMA-double-positive cancer, or a cancer with double low expression of BCMA and GPRC5D, such as a cancer with low expression of GPRC5D and/or low expression of BCMA (e.g., a cancer that recurs after anti-BCMA therapy or anti-GPRC5D therapy). In some embodiments, the cancer may be a solid tumor or a hematological tumor. In some embodiments, the cancer is multiple myeloma. By having antigen-binding specificity against BCMA and GPRC5D, the trispecific antibody molecule of the present invention can have a broader patient population for cancer treatment compared to BCMA/CD3-targeting bispecific antibodies and GPRC5D/CD3-targeting bispecific antibodies. In some embodiments, the present invention provides use of the trispecific antibody molecule of the present invention in the treatment of a GPRC5D-single-positive cancer. In some embodiments, the present invention provides use of the trispecific antibody molecule of the present invention in the treatment of a BCMA-single-positive cancer. In some embodiments, the present invention provides use of the trispecific antibody molecule of the present invention in the treatment of a GPRC5D/BCMA-double-positive cancer. In some embodiments, the present invention also provides a method for treating a cancer with low expression of BCMA and/or GPRC5D, such as a cancer with low expression of GPRC5D and/or low expression of BCMA or double low expression of BCMA and GPRC5D (e.g., a cancer that recurs after anti-BCMA therapy and/or anti-GPRC5D therapy), using the trispecific antibody molecule of the present invention.

The "GPRC5D-single-positive cancer" described herein refers to that only GPRC5D expression is present in the cancer cells, or only GPRC5D expression is elevated (i.e., overexpressed) in the cancer cells than in healthy cells (e.g., cells of the same tissue of a healthy subject or healthy cells adjacent to the cancer cells). The " a cancer with low expression of GPRC5D" described herein refers to that GPRC5D is expressed in the cancer cells at a lower level, e.g., at a slightly higher level than in a healthy cell of normal tissue.

The "BCMA-single-positive cancer" described herein refers to that only BCMA expression is present in the cancer cells, or only BCMA expression is elevated (i.e., overexpressed) in the cancer cells than in healthy cells (e.g., cells of the same tissue of a healthy subject or healthy cells adjacent to the cancer cells). The "a cancer with low expression of BCMA" refers to that BCMA is expressed in the cancer cells at a lower level, e.g., at a slightly higher level than in a healthy cell of normal tissue.

The "GPRC5D/BCMA-double-positive cancer" described herein refers to that GPRC5D expression and BCMA expression are present in the cancer cells, or GPRC5D expression and BCMA expression are elevated in the cancer cells than in healthy cells (e.g., cells of the same tissue of a healthy subject or healthy cells adjacent to the cancer cells). The "GPRC5D/BCMA-double-positive cancer" also encompasses cancers with BCMA expression and GPRC5D expression but low expression of either or both, e.g., a cancer with low expression of BCMA and/or GPRC5D, such as a cancer with low expression of GPRC5D and/or low expression of BCMA or a cancer with double low expression of BCMA and/or GPRC5D (e.g., a cancer that recurs after anti-BCMA therapy and/or anti-GPRC5D therapy).

When referring to "BCMA-positive", it encompasses not only cases where wild-type BCMA is expressed, but also cases where BCMA variants are expressed. BCMA variants may be variants obtained due to mutations such as deletions or amino acid substitutions. In some embodiments, BCMA mutations lead to drug resistance to immunotherapy targeting BCMA in individuals. Therefore, the "BCMA-positive cancer" of the present invention also encompasses cancers in which BCMA variants expression is presented, or cancers in which BCMA variants expression is elevated (i.e., overexpressed) in cancer cells than in healthy cells (e.g., cells of the same tissue of a healthy subject or healthy cells adjacent to the cancer cells).

In some embodiments, the BCMA variant comprises one or more or all of the following mutations:
(i) a proline (P) deletion at position 34 (P34del);
(ii) a serine (S) deletion at position 30 (S30del);
(iii) an amino acid substitution at position 39, e.g., a substitution of arginine (R) to alanine (A) (R39A); and
(iv) an amino acid substitution at position 27, e.g., a substitution of arginine (R) to proline (P) (R27P).

In some embodiments, the present invention provides a method for inhibiting or killing GPRC5D-positive and/or BCMA-positive cells.

In some embodiments, the GPRC5D-positive cells are cells with GPRC5D expression or overexpression in the cells. In some embodiments, the BCMA-positive cells are cells with BCMA expression or overexpression in the cells.

In some embodiments, the BCMA variant in the BCMA-positive cells comprises one or more or all of the following mutations:
(i) a proline (P) deletion at position 34 (P34del);
(ii) a serine (S) deletion at position 30 (S30del);
(iii) an amino acid substitution at position 39, e.g., a substitution of arginine (R) to alanine (A) (R39A); and
(iv) an amino acid substitution at position 27, e.g., a substitution of arginine (R) to proline (P) (R27P).

In some embodiments, the BCMA-positive cells with BCMA variants expression or overexpression of the present invention are resistant to immunotherapy targeting BCMA (e.g., Elranatamab or Teclistamab treatment). In some embodiments, the BCMA-positive cells with BCMA variants expression or overexpression of the present invention are resistant to immunotherapy targeting BCMA and GPRC5D.

In some embodiments, in the BCMA-positive cells with BCMA variants expression or overexpression of the present invention, BCMA has a P34del, S30del, R39A, or R27P mutation. In some embodiments, in the BCMA-positive cells with BCMA variants expression or overexpression of the present invention, the expression of BCMA having a P34del, S30del, R39A, or R27P mutation is presented, or its expression is elevated (i.e., overexpressed) than in healthy cells (e.g., cells of the same tissue of a healthy subject or healthy cells adjacent to cancer cells).

In some embodiments, the BCMA-positive cells with BCMA variants expression or overexpression of the present invention have expression or overexpression of BCMA variant containing a P34del or R27P mutation and are resistant to Elranatamab.

In some embodiments, the BCMA-positive cells with BCMA variants expression or overexpression of the present invention have expression or overexpression of BCMA variant containing a P34del, S30del, R39A, or R27P mutation and are resistant to Teclistamab.

In some embodiments, the antibody molecule of the present invention or the pharmaceutical composition comprising the antibody molecule of the present invention is used as a drug for the treatment and/or prevention of a GPRC5D- and/or BCMA-related disease or for killing or inhibiting GPRC5D-positive and/or BCMA-positive cells in an individual or as a diagnostic tool for a GPRC5D- and/or BCMA-related disease, wherein, preferably, the individual is a mammal, more preferably a human.

In other aspects, the present invention provides use of the trispecific antibody, or the immunoconjugate, the composition, or the combination product comprising the same of the present invention in the manufacture or preparation of a medicament for the use described herein, e.g., for use in preventing or treating the related disease or condition mentioned herein or for killing or inhibiting GPRC5D-positive and/or BCMA-positive cells.

In some embodiments, the trispecific antibody (and the immunoconjugate, the composition, the pharmaceutical composition, the formulation, and the like comprising the same) of the present invention can also be administered in combination with one or more additional therapies, e.g., therapeutic modalities and/or additional therapeutic agents, for the use described herein, e.g., for use in preventing and/or treating the related disease or condition mentioned herein or for killing or inhibiting GPRC5D-positive and/or BCMA-positive cells.

In some embodiments, the therapeutic modality is, for example, surgical therapy or radiotherapy.

In one aspect, the present invention provides a diagnostic method for detecting the presence of associated antigens in a biological sample, such as a serum, semen, urine or tissue biopsy sample (e.g., from a hyperproliferative or cancerous lesion), *in vitro* or *in vivo.* The diagnostic method comprises: (i) contacting a sample (and optionally a control sample) with the antibody molecule as described herein or administering the antibody molecule to a subject under conditions that allow interactions, and (ii) detecting the formation of a complex by the antibody molecule and the sample (and optionally the control sample). The formation of a complex indicates the presence of the associated antigen and may show the suitability of or need for the treatment and/or prevention described herein.

In some embodiments, the associated antigens, such as GPRC5D and/or BCMA, are detected prior to the treatment, e.g., prior to the initial treatment or prior to a certain treatment after a treatment interval. Detection methods that can be used include immunohistochemistry, immunocytochemistry, FACS, ELISA assays, PCR techniques (e.g., RT-PCR), or *in vivo* imaging techniques. Generally, antibody molecules used in *in vivo* and *in vitro* detection methods are directly or indirectly labeled with a detectable substance to facilitate the detection of bound or unbound conjugates. Suitable detectable substances include a variety of biologically active enzymes, prosthetic groups, fluorescent substances, luminescent substances, paramagnetic (e.g., nuclear magnetic resonance active) substances, and radioactive substances.

In some embodiments, the level and/or distribution of the associated antigens, such as GPRC5D and/or BCMA, are/is determined *in vivo.* For example, the antibody molecule of the present invention labeled with a detectable substance is detected in a non-invasive manner, e.g., by using appropriate imaging techniques such as positron emission tomography (PET) scanning. In one embodiment, for example, the level and/or distribution of the associated antigens are/is determined *in vivo* by detecting the antibody molecule of the present invention that is detectably labeled with a PET reagent (e.g., 18F-fluorodeoxyglucose (FDG)).

In one embodiment, the present invention provides a diagnostic kit comprising the antibody molecule described herein and a package insert.

### VII. Specific embodiments

In one aspect of the present invention, the present invention relates to the following specific embodiments:
1. A trispecific antibody specifically binding to BCMA, comprising a first antigen-binding region specifically binding to BCMA, and second and third antigen-binding regions specifically binding to other antigens.
2. The trispecific antibody according to embodiment 1, wherein the second antigen-binding region specifically binds to CD3, and/or the third antigen-binding region specifically binds to GPRC5D.
3. The trispecific antibody according to embodiment 1 or 2, wherein the first antigen-binding region, the second antigen-binding region, and the third antigen-binding region are a first Fab, a second Fab, and a third Fab, respectively.
4. The trispecific antibody according to embodiment 3, wherein
   (1) the first Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the third Fab is fused to N-terminus of a VH of the Fab heavy chain of the second Fab fragment at C-terminus of a CH1 of an Fab heavy chain;
      or
      the third Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the first Fab is fused to N-terminus of a VH of the Fab heavy chain of the second Fab fragment at C-terminus of a CH1 of an Fab heavy chain;
      or
   (2) the first Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the third Fab is fused to C-terminus of the Fc region fused to the second Fab at N-terminus of a VH of an Fab heavy chain; or
      the third Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the first Fab is fused to C-terminus of the Fc region fused to the second Fab at N-terminus of a VH of an Fab heavy chain.
5. The trispecific antibody according to embodiment 4, wherein the fusion comprises direct fusion or fusion through a linker.
6. The trispecific antibody according to embodiment 5, wherein the linker is (GGGGS)n, wherein n = 1, 2, 3, or 4.
7. The trispecific antibody according to any one of embodiments 3 to 6, wherein the second Fab comprises a charge mutation, and the first or third Fab fused to the second Fab or to the second Fab through the Fc region comprises a charge mutation and a disulfide bond remodeling mutation.
8. The trispecific antibody according to embodiment 7, wherein the charge mutation is a mutation to D or K at position 39 of a VH of an Fab and to K or D at position 38 of a VL of the Fab, for example,
   the second Fab comprises a VH comprising 39D and a VL comprising 38K, and
   the first or third Fab fused to the second Fab through the Fc region comprises a VH comprising 39K and a VL comprising 38D; or
   the second Fab comprises a VH comprising 39K and a VL comprising 38D, and
   the first or third Fab fused to the second Fab through the Fc region comprises a VH comprising 39D and a VL comprising 38K.
9. The trispecific antibody according to embodiment 7 or 8, wherein the disulfide bond remodeling mutation comprises F126C in a CH1 of an Fab and comprises Q124C in a CL of the Fab.
10. The trispecific antibody according to embodiment 9, wherein the Fab comprising a disulfide bond remodeling mutation comprises a CH1, wherein the CH1
   (i) comprises an amino acid sequence having at least 90% identity to an amino acid sequence set forth in SEQ ID NO: 85, and comprises F126C; or
   (ii) comprises or consists of an amino acid sequence set forth in SEQ ID NO: 85.
11. The trispecific antibody according to embodiment 9 or 10, wherein the Fab comprising a disulfide bond remodeling mutation comprises a CL, wherein the CL
   (i) comprises an amino acid sequence having at least 90% identity to an amino acid sequence set forth in SEQ ID NO: 97, and comprises Q124C; or
   (ii) comprises or consists of an amino acid sequence set forth in SEQ ID NO: 97.
12. The trispecific antibody according to embodiment 9, wherein the Fab comprising a disulfide bond remodeling mutation comprises a CH1 and a CL, wherein the CH1 comprises or consists of an amino acid sequence set forth in SEQ ID NO: 85, and the CL comprises or consists of an amino acid sequence set forth in SEQ ID NO: 97.
13. The trispecific antibody according to embodiment 7 or 8, wherein the disulfide bond remodeling mutation comprises F126C and C220S in a CH1 of an Fab and comprises Q124C and C214S in a CL of the Fab.
14. The trispecific antibody according to embodiment 13, wherein the Fab comprising a disulfide bond remodeling mutation comprises a CH1, wherein the CH1
   (i) comprises an amino acid sequence having at least 90% identity to an amino acid sequence set forth in SEQ ID NO: 86, and comprises F126C and C220S; or
   (ii) comprises or consists of an amino acid sequence set forth in SEQ ID NO: 86.
15. The trispecific antibody according to embodiment 13 or 14, wherein the Fab comprising a disulfide bond remodeling mutation comprises a CL, wherein the CL
   (i) comprises an amino acid sequence having at least 90% identity to an amino acid sequence set forth in SEQ ID NO: 98, and comprises Q124C and C214S; or
   (ii) comprises or consists of an amino acid sequence set forth in SEQ ID NO: 98.
16. The trispecific antibody according to embodiment 13, wherein the Fab comprising a disulfide bond remodeling mutation comprises a CH1 and a CL, wherein the CH1 comprises or consists of an amino acid sequence set forth in SEQ ID NO: 86, and the CL comprises or consists of an amino acid sequence set forth in SEQ ID NO: 98.
17. The trispecific antibody according to any one of embodiments 1 to 16, wherein the trispecific antibody comprises a first Fc region and a second Fc region, wherein the first Fc region and the second Fc region are identical or different.
18. The trispecific antibody according to embodiment 17, wherein the first Fc region and the second Fc region are human IgG Fcs, such as human IgG1 Fc, human IgG2 Fc, human IgG3 Fc, or human IgG4 Fc, for example, comprising or consisting of an amino acid sequence set forth in SEQ ID NO: 87 or 88, or an amino acid sequence having at least 90% identity, e.g., 95%, 96%, 97%, 99% or more identity, thereto.
19. The trispecific antibody according to embodiment 18, wherein the first and/or second Fc region comprises an L234A/L235A mutation.
20. The trispecific antibody according to embodiment 19, wherein the first and/or second Fc region comprises an amino acid sequence set forth in SEQ ID NO: 89 or 90, or comprises an amino acid sequence having at least 90% identity, e.g., 95%, 96%, 97%, 99% or more identity, to an amino acid sequence set forth in SEQ ID NO: 89 or 90 and comprises an amino acid substitution L234A/L235A.
21. The trispecific antibody according to any one of embodiments 17 to 20, wherein one of the first and second Fc regions comprises a Knob mutation and the other comprises a Hole mutation.
22. The trispecific antibody according to embodiment 21, wherein the one Fc region comprises an amino acid substitution T366W, and the other Fc region comprises amino acid substitutions T366S, L368A and Y407V (numbering according to EU index).
23. The trispecific antibody according to embodiment 22, wherein one Fc region polypeptide comprises an amino acid sequence having at least 85%, 90%, 95%, 96%, 97%, 98%, or 99% identity to an amino acid sequence set forth in SEQ ID NO: 91, and the other Fc region polypeptide comprises an amino acid sequence having at least 85%, 90%, 95%, 96%, 97%, 98%, or 99% identity to an amino acid sequence set forth in SEQ ID NO: 92.
24. The trispecific antibody according to embodiment 23, wherein the one Fc region polypeptide comprises or consists of an amino acid sequence set forth in SEQ ID NO: 91, and the other Fc region polypeptide comprises or consists of an amino acid sequence set forth in SEQ ID NO: 92.
25. The trispecific antibody according to embodiment 17, wherein the one Fc region comprises amino acid substitutions L234A/L235A and T366W, and the other Fc region comprises amino acid substitutions L234A/L235A, T366S, L368A and Y407V (numbering according to EU index).
26. The trispecific antibody according to embodiment 25, wherein one Fc region polypeptide comprises an amino acid sequence having at least 85%, 90%, 95%, 96%, 97%, 98%, or 99% identity to an amino acid sequence set forth in SEQ ID NO: 94, and the other Fc region polypeptide comprises an amino acid sequence having at least 85%, 90%, 95%, 96%, 97%, 98%, or 99% identity to an amino acid sequence set forth in SEQ ID NO: 93.
27. The trispecific antibody according to embodiment 26, wherein the one Fc region polypeptide comprises or consists of an amino acid sequence set forth in SEQ ID NO: 94, and the other Fc region polypeptide comprises or consists of an amino acid sequence set forth in SEQ ID NO: 93.
28. The trispecific antibody according to any one of embodiments 1 to 27, wherein the first antigen-binding region specifically binding to BCMA comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein
   the HCDR1 comprises or consists of an amino acid sequence set forth in SEQ ID NO: 57; the HCDR2 comprises or consists of an amino acid sequence set forth in SEQ ID NO: 58; the HCDR3 comprises or consists of an amino acid sequence set forth in SEQ ID NO: 59; the LCDR1 comprises or consists of an amino acid sequence set forth in SEQ ID NO: 61; the LCDR2 comprises or consists of an amino acid sequence set forth in SEQ ID NO: 62; and the LCDR3 comprises or consists of an amino acid sequence set forth in SEQ ID NO: 63.
29. The trispecific antibody according to embodiment 28, wherein the VH of the first antigen-binding region specifically binding to BCMA comprises or consists of an amino acid sequence set forth in SEQ ID NO: 56, or an amino acid sequence having at least 90%, 91%, 92%, 93%, 94%, 95%, 96%, 97%, 98%, or 99% identity thereto.
30. The trispecific antibody according to embodiment 28 or 29, wherein the VL of the first antigen-binding region specifically binding to BCMA comprises or consists of an amino acid sequence set forth in SEQ ID NO: 60, or an amino acid sequence having at least 90%, 91%, 92%, 93%, 94%, 95%, 96%, 97%, 98%, or 99% identity thereto.
31. The trispecific antibody according to embodiment 30, wherein the first antigen-binding region specifically binding to BCMA comprises a VH and a VL, wherein the VH consists of an amino acid sequence set forth in SEQ ID NO: 56, and the VL of the first antigen-binding region specifically binding to BCMA consists of an amino acid sequence set forth in SEQ ID NO: 60.
32. The trispecific antibody according to embodiment 28, wherein the first antigen-binding region specifically binding to BCMA comprises a VH and a VL, wherein the VH comprises 39K and the VL comprises 38D; or the VH comprises 39D and the VL comprises 38K,
   for example,
   the VH of the first antigen-binding region
      (i) comprises or consists of an amino acid sequence set forth in SEQ ID NO: 76, or
      (ii) comprises an amino acid sequence having at least 90%, 91%, 92%, 93%, 94%, 95%, 96%, 97%, 98%, or 99% identity to an amino acid sequence set forth in SEQ ID NO: 76, and comprises a Q39K mutation;
   and/or the VL of the first antigen-binding region
      (i) comprises or consists of an amino acid sequence set forth in SEQ ID NO: 75, or
      (ii) comprises an amino acid sequence having at least 90%, 91%, 92%, 93%, 94%, 95%, 96%, 97%, 98%, or 99% identity to an amino acid sequence set forth in SEQ ID NO: 75, and comprises a Q38D mutation.
33. The trispecific antibody according to any one of embodiments 1 to 32, wherein the third antigen-binding region specifically binds to GPRC5D and comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein
   the HCDR1 comprises or consists of an amino acid sequence set forth in SEQ ID NO: 41; the HCDR2 comprises or consists of an amino acid sequence set forth in SEQ ID NO: 42; the HCDR3 comprises or consists of an amino acid sequence set forth in SEQ ID NO: 43; the LCDR1 comprises or consists of an amino acid sequence set forth in SEQ ID NO: 45; the LCDR2 comprises or consists of an amino acid sequence set forth in SEQ ID NO: 46; and the LCDR3 comprises or consists of an amino acid sequence set forth in SEQ ID NO: 47; or
   the HCDR1 comprises or consists of an amino acid sequence set forth in SEQ ID NO: 49; the HCDR2 comprises or consists of an amino acid sequence set forth in SEQ ID NO: 50; the HCDR3 comprises or consists of an amino acid sequence set forth in SEQ ID NO: 51; the LCDR1 comprises or consists of an amino acid sequence set forth in SEQ ID NO: 53; the LCDR2 comprises or consists of an amino acid sequence set forth in SEQ ID NO: 54; and the LCDR3 comprises or consists of an amino acid sequence set forth in SEQ ID NO: 55.
34. The trispecific antibody according to embodiment 33, wherein the VH of the third antigen-binding region specifically binding to GPRC5D
   (i) comprises or consists of an amino acid sequence set forth in SEQ ID NO: 40, or an amino acid sequence having at least 90%, 91%, 92%, 93%, 94%, 95%, 96%, 97%, 98%, or 99% identity thereto; or
   (ii) comprises or consists of an amino acid sequence set forth in SEQ ID NO: 48, or an amino acid sequence having at least 90%, 91%, 92%, 93%, 94%, 95%, 96%, 97%, 98%, or 99% identity thereto.
35. The trispecific antibody according to embodiment 33 or 34, wherein the VL of the third antigen-binding region specifically binding to GPRC5D
   (i) comprises or consists of an amino acid sequence set forth in SEQ ID NO: 44, or an amino acid sequence having at least 90%, 91%, 92%, 93%, 94%, 95%, 96%, 97%, 98%, or 99% identity thereto; or
   (ii) comprises or consists of an amino acid sequence set forth in SEQ ID NO: 52, or an amino acid sequence having at least 90%, 91%, 92%, 93%, 94%, 95%, 96%, 97%, 98%, or 99% identity thereto.
36. The trispecific antibody according to embodiment 35, wherein the third antigen-binding region specifically binding to GPRC5D comprises a VH and a VL, wherein the VH consists of an amino acid sequence set forth in SEQ ID NO: 48, and the VL consists of an amino acid sequence set forth in SEQ ID NO: 52; or the VH consists of an amino acid sequence set forth in SEQ ID NO: 40, and the VL consists of an amino acid sequence set forth in SEQ ID NO: 44.
37. The trispecific antibody according to any one of embodiments 33 to 36, wherein the third antigen-binding region specifically binding to GPRC5D comprises a VH and a VL, wherein the VH comprises 39K and the VL comprises 38D; or the VH comprises 39D and the VL comprises 38K, for example,
   the VH of the third antigen-binding region specifically binding to GPRC5D
      (i) comprises or consists of an amino acid sequence set forth in SEQ ID NO: 80, or
      (ii) comprises an amino acid sequence having at least 90%, 91%, 92%, 93%, 94%, 95%, 96%, 97%, 98%, or 99% identity to an amino acid sequence set forth in SEQ ID NO: 80, and comprises a Q39K mutation;
   and the VL of the third antigen-binding region specifically binding to GPRC5D
      (i) comprises or consists of an amino acid sequence set forth in SEQ ID NO: 79, or
      (ii) comprises an amino acid sequence having at least 90%, 91%, 92%, 93%, 94%, 95%, 96%, 97%, 98%, or 99% identity to an amino acid sequence set forth in SEQ ID NO: 79, and comprises a Q38D mutation.
38. The trispecific antibody according to any one of embodiments 1 to 37, wherein the second antigen-binding region specifically binds to CD3 and comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein
   the HCDR1 comprises or consists of an amino acid sequence set forth in SEQ ID NO: 65; the HCDR2 comprises or consists of an amino acid sequence set forth in SEQ ID NO: 66; the HCDR3 comprises or consists of an amino acid sequence set forth in SEQ ID NO: 67 or 73; the LCDR1 comprises or consists of an amino acid sequence set forth in SEQ ID NO: 69; the LCDR2 comprises or consists of an amino acid sequence set forth in SEQ ID NO: 70; and the LCDR3 comprises or consists of an amino acid sequence set forth in SEQ ID NO: 71.
39. The trispecific antibody according to embodiment 38, wherein the VH of the second antigen-binding region specifically binding to CD3 comprises or consists of an amino acid sequence set forth in SEQ ID NO: 64 or 72, or an amino acid sequence having at least 90%, 91%, 92%, 93%, 94%, 95%, 96%, 97%, 98%, or 99% identity thereto.
40. The trispecific antibody according to embodiment 38 or 39, wherein the VL of the second antigen-binding region specifically binding to CD3 comprises or consists of an amino acid sequence set forth in SEQ ID NO: 68, or an amino acid sequence having at least 90%, 91%, 92%, 93%, 94%, 95%, 96%, 97%, 98%, or 99% identity thereto.
41. The trispecific antibody according to embodiment 40, wherein the second antigen-binding region specifically binding to CD3 comprises a VH and a VL, wherein the VH consists of an amino acid sequence set forth in SEQ ID NO: 64 or 72, and the VL of the second antigen-binding region consists of an amino acid sequence set forth in SEQ ID NO: 68.
42. The trispecific antibody according to any one of embodiments 38 to 41, wherein the second antigen-binding region specifically binding to CD3 comprises a VH and a VL, wherein the VH comprises 39K and the VL comprises 38D; or the VH comprises 39D and the VL comprises 38K.
43. The trispecific antibody according to embodiment 42, wherein the VH of the second antigen-binding region specifically binding to CD3
   (i) comprises or consists of an amino acid sequence set forth in SEQ ID NO: 77 or 78, or
   (ii) comprises an amino acid sequence having at least 90%, 91%, 92%, 93%, 94%, 95%, 96%, 97%, 98%, or 99% identity to an amino acid sequence set forth in SEQ ID NO: 77 or 78, and comprises a Q39D mutation;
   and the VL of the second antigen-binding region specifically binding to CD3
   (i) comprises or consists of an amino acid sequence set forth in SEQ ID NO: 74, or
   (ii) comprises an amino acid sequence having at least 90%, 91%, 92%, 93%, 94%, 95%, 96%, 97%, 98%, or 99% identity to an amino acid sequence set forth in SEQ ID NO: 74, and comprises a Q38K mutation.
44. The trispecific antibody according to any one of embodiments 3 to 43, wherein the trispecific antibody is a left-right asymmetric IgG-like pentamer consisting of 5 polypeptide chains and consists of the following peptide chains:
   a peptide chain 1# sequentially comprising an Fab heavy chain specifically binding to GPRC5D and an Fc domain,
   a peptide chain 2# comprising an Fab light chain specifically binding to GPRC5D,
   a peptide chain 3# sequentially comprising an Fab heavy chain specifically binding to BCMA, an Fab heavy chain specifically binding to CD3, and an Fc domain,
   a peptide chain 4# comprising an Fab light chain specifically binding to BCMA, and
   a peptide chain 5# comprising an Fab light chain specifically binding to CD3,
   wherein the Fab heavy chain of the peptide chain 1# is paired with the Fab light chain of the peptide chain 2# to form the third Fab, and the two Fab heavy chains of the peptide chain 3# are paired with the Fab light chains of the peptide chain 4# and the peptide chain 5# to form the first Fab and the second Fab, respectively.
45. The trispecific antibody according to embodiment 44, wherein
   the peptide chain 1# comprises or consists of an amino acid sequence set forth in SEQ ID NO: 5, or an amino acid sequence having at least 85%, 90%, 91%, 92%, 93%, 94%, 95%, 96%, 97%, 98%, or 99% identity to an amino acid sequence set forth in SEQ ID NO: 5;
   the peptide chain 2# comprises or consists of an amino acid sequence set forth in SEQ ID NO: 4, or an amino acid sequence having at least 85%, 90%, 91%, 92%, 93%, 94%, 95%, 96%, 97%, 98%, or 99% identity to an amino acid sequence set forth in SEQ ID NO: 4;
   the peptide chain 3# comprises or consists of an amino acid sequence set forth in SEQ ID NO: 3 or 6, or an amino acid sequence having at least 85%, 90%, 91%, 92%, 93%, 94%, 95%, 96%, 97%, 98%, or 99% identity to an amino acid sequence set forth in SEQ ID NO: 3 or 6;
   the peptide chain 4# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2; and/or
   the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.
46. The trispecific antibody according to embodiment 44, wherein
   the peptide chain 1# comprises or consists of an amino acid sequence set forth in SEQ ID NO: 15, or an amino acid sequence having at least 85%, 90%, 91%, 92%, 93%, 94%, 95%, 96%, 97%, 98%, or 99% identity to an amino acid sequence set forth in SEQ ID NO: 15;
   the peptide chain 2# comprises or consists of an amino acid sequence set forth in SEQ ID NO: 14, or an amino acid sequence having at least 85%, 90%, 91%, 92%, 93%, 94%, 95%, 96%, 97%, 98%, or 99% identity to an amino acid sequence set forth in SEQ ID NO: 14;
   the peptide chain 3# comprises or consists of an amino acid sequence set forth in SEQ ID NO: 3 or 6, or an amino acid sequence having at least 85%, 90%, 91%, 92%, 93%, 94%, 95%, 96%, 97%, 98%, or 99% identity to an amino acid sequence set forth in SEQ ID NO: 3 or 6;
   the peptide chain 4# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2; and/or
   the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.
47. The trispecific antibody according to any one of embodiments 1 to 43, wherein the trispecific antibody is a left-right asymmetric IgG-like pentamer consisting of 5 polypeptide chains and consists of the following peptide chains:
   a peptide chain 1# sequentially comprising an Fab heavy chain specifically binding to BCMA and an Fc domain,
   a peptide chain 2# comprising an Fab light chain specifically binding to BCMA,
   a peptide chain 3# sequentially comprising an Fab heavy chain specifically binding to GPRC5D, an Fab heavy chain specifically binding to CD3, and an Fc domain,
   a peptide chain 4# comprising an Fab light chain specifically binding to GPRC5D, and
   a peptide chain 5# comprising an Fab light chain specifically binding to CD3,
   wherein the Fab heavy chain of the peptide chain 1# is paired with the Fab light chain of the peptide chain 2# to form the first Fab, and the two Fab heavy chains of the peptide chain 3# are paired with the Fab light chains of the peptide chain 4# and the peptide chain 5# to form the third Fab and the second Fab, respectively.
48. The trispecific antibody according to embodiment 47, wherein
   the peptide chain 1# comprises or consists of an amino acid sequence set forth in SEQ ID NO: 11, or an amino acid sequence having at least 85%, 90%, 91%, 92%, 93%, 94%, 95%, 96%, 97%, 98%, or 99% identity to an amino acid sequence set forth in SEQ ID NO: 11;
   the peptide chain 2# comprises or consists of an amino acid sequence set forth in SEQ ID NO: 10, or an amino acid sequence having at least 85%, 90%, 91%, 92%, 93%, 94%, 95%, 96%, 97%, 98%, or 99% identity to an amino acid sequence set forth in SEQ ID NO: 10;
   the peptide chain 3# comprises or consists of an amino acid sequence set forth in SEQ ID NO: 9, or an amino acid sequence having at least 85%, 90%, 91%, 92%, 93%, 94%, 95%, 96%, 97%, 98%, or 99% identity to an amino acid sequence set forth in SEQ ID NO: 9;
   the peptide chain 4# comprises or consists of an amino acid sequence set forth in SEQ ID NO: 8, or an amino acid sequence having at least 85%, 90%, 91%, 92%, 93%, 94%, 95%, 96%, 97%, 98%, or 99% identity to an amino acid sequence set forth in SEQ ID NO: 8; and/or
   the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.
49. The trispecific antibody according to any one of embodiments 1 to 43, wherein the trispecific antibody is a left-right asymmetric IgG-like pentamer consisting of 5 polypeptide chains and consists of the following peptide chains:
   a peptide chain 1# sequentially comprising an Fab heavy chain specifically binding to GPRC5D and an Fc domain,
   a peptide chain 2# comprising an Fab light chain specifically binding to GPRC5D,
   a peptide chain 3# sequentially comprising an Fab heavy chain specifically binding to CD3, an Fc domain, and an Fab heavy chain of the antigen-binding region specifically binding to BCMA,
   a peptide chain 4# comprising an Fab light chain specifically binding to CD3, and
   a peptide chain 5# comprising an Fab light chain of the antigen-binding region specifically binding to BCMA,
   wherein the Fab heavy chain of the peptide chain 1# is paired with the Fab light chain of the peptide chain 2# to form the third Fab, and the two Fab heavy chains of the peptide chain 3# are paired with the Fab light chains of the peptide chain 4# and the peptide chain 5# to form the second Fab and the first Fab, respectively.
50. The trispecific antibody according to embodiment 49, wherein
   the peptide chain 1# comprises or consists of an amino acid sequence set forth in SEQ ID NO: 5, or an amino acid sequence having at least 85%, 90%, 91%, 92%, 93%, 94%, 95%, 96%, 97%, 98%, or 99% identity to an amino acid sequence set forth in SEQ ID NO: 5;
   the peptide chain 2# comprises or consists of an amino acid sequence set forth in SEQ ID NO: 4, or an amino acid sequence having at least 85%, 90%, 91%, 92%, 93%, 94%, 95%, 96%, 97%, 98%, or 99% identity to an amino acid sequence set forth in SEQ ID NO: 4;
   the peptide chain 3# comprises or consists of an amino acid sequence set forth in SEQ ID NO: 7, or an amino acid sequence having at least 85%, 90%, 91%, 92%, 93%, 94%, 95%, 96%, 97%, 98%, or 99% identity to an amino acid sequence set forth in SEQ ID NO: 7;
   the peptide chain 4# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1; and/or
   the peptide chain 5# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2.
51. A nucleic acid molecule encoding any one of the chains of the trispecific antibody according to any one of embodiments 1 to 50, or consisting of a nucleic acid sequence.
52. An expression vector comprising the nucleic acid molecule according to embodiment 51, wherein, for example, the expression vector is a pCNDA vector, such as a pCDNA3.1 expression vector.
53. A host cell comprising the nucleic acid molecule according to embodiment 51 or the expression vector according to embodiment 52, wherein, preferably, the host cell is prokaryotic or eukaryotic, e.g., a 293 cell or a CHO cell, such as an HEK293 cell.
54. A method for preparing the trispecific antibody according to any one of embodiments 1 to 50, wherein the method comprises culturing the host cell comprising the nucleic acid molecule according to embodiment 51 or the expression vector according to embodiment 52 under a condition suitable for expressing the chains of the antibody, and optionally recovering the antibody from the host cell (or a host cell medium).
55. An immunoconjugate comprising the trispecific antibody according to any one of embodiments 1 to 50.
56. A pharmaceutical composition or a drug or a formulation comprising the trispecific antibody according to any one of embodiments 1 to 50 or the immunoconjugate according to embodiment 55, and optionally a pharmaceutical supplementary material.
57. A pharmaceutical combination comprising the trispecific antibody according to any one of embodiments 1 to 50 or the immunoconjugate according to embodiment 55, and one or more additional therapeutic agents, such as a chemotherapeutic agent.
58. A method for preventing or treating a cancer in a subject, comprising administering to the subject an effective amount of the trispecific antibody according to any one of embodiments 1 to 50, or the immunoconjugate according to embodiment 55, or the pharmaceutical composition or the formulation according to embodiment 56, or the pharmaceutical combination according to embodiment 57.
59. The method according to embodiment 58, wherein the cancer is a GPRC5D-single-positive cancer, a BCMA-single-positive cancer, a GPRC5D/BCMA-double-positive cancer, or a cancer with double low expression of BCMA and GPRC5D, such as a cancer with low expression of GPRC5D and/or low expression of BCMA (e.g., a cancer that recurs after anti-BCMA therapy or anti-GPRC5D therapy).
60. The method according to embodiment 58 or 59, wherein the cancer is a solid tumor or a hematological tumor, for example, the cancer is multiple myeloma.
61. The method according to any one of embodiments 58 to 60, wherein the method further comprises administering in combination with additional therapies, such as therapeutic modalities (e.g., surgical therapy or radiotherapy) and/or additional therapeutic agents (e.g., a chemotherapeutic agent).

In one aspect of the present invention, the present invention further relates to the following specific embodiments:
1. A trispecific antibody specifically binding to GPRC5D, comprising a first antigen-binding region, a second antigen-binding region, and a third antigen-binding region specifically binding to BCMA, wherein the first antigen-binding region comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein
   the HCDR1 comprises or consists of an amino acid sequence set forth in SEQ ID NO: 57; the HCDR2 comprises or consists of an amino acid sequence set forth in SEQ ID NO: 58; the HCDR3 comprises or consists of an amino acid sequence set forth in SEQ ID NO: 59; the LCDR1 comprises or consists of an amino acid sequence set forth in SEQ ID NO: 61; the LCDR2 comprises or consists of an amino acid sequence set forth in SEQ ID NO: 62; and/or the LCDR3 comprises or consists of an amino acid sequence set forth in SEQ ID NO: 63;
   preferably, the second antigen-binding region specifically binds to CD3, and/or the third antigen-binding region specifically binds to GPRC5D.
2. The trispecific antibody according to embodiment 1, wherein the VH of the first antigen-binding region specifically binding to BCMA comprises or consists of an amino acid sequence set forth in SEQ ID NO: 56, or an amino acid sequence having at least 90%, 91%, 92%, 93%, 94%, 95%, 96%, 97%, 98%, or 99% identity thereto.
3. The trispecific antibody according to embodiment 1 or 2, wherein the VL of the first antigen-binding region specifically binding to BCMA comprises or consists of an amino acid sequence set forth in SEQ ID NO: 60, or an amino acid sequence having at least 90%, 91%, 92%, 93%, 94%, 95%, 96%, 97%, 98%, or 99% identity thereto.
4. The trispecific antibody according to embodiment 1, wherein the first antigen-binding region specifically binding to BCMA comprises a VH and a VL, wherein the VH consists of an amino acid sequence set forth in SEQ ID NO: 56, and the VL of the first antigen-binding region consists of an amino acid sequence set forth in SEQ ID NO: 60.
5. The trispecific antibody according to any one of embodiments 1 to 4, wherein the first antigen-binding region specifically binding to BCMA comprises a VH and a VL, wherein the VH comprises 39K and the VL comprises 38D; or the VH comprises 39D and the VL comprises 38K.
6. The trispecific antibody according to embodiment 5, wherein the VH of the first antigen-binding region specifically binding to BCMA
   (i) comprises or consists of an amino acid sequence set forth in SEQ ID NO: 76, or
   (ii) comprises an amino acid sequence having at least 90%, 91%, 92%, 93%, 94%, 95%, 96%, 97%, 98%, or 99% identity to an amino acid sequence set forth in SEQ ID NO: 76, and comprises a Q39K mutation;
   and the VL of the first antigen-binding region specifically binding to BCMA
   (i) comprises or consists of an amino acid sequence set forth in SEQ ID NO: 75, or
   (ii) comprises an amino acid sequence having at least 90%, 91%, 92%, 93%, 94%, 95%, 96%, 97%, 98%, or 99% identity to an amino acid sequence set forth in SEQ ID NO: 75, and comprises a Q38D mutation.
7. The trispecific antibody according to any one of embodiments 1 to 6, wherein the first antigen-binding region specifically binding to BCMA is an Fab.
8. The trispecific antibody according to any one of embodiments 1 to 7, wherein the third antigen-binding region specifically binding to GPRC5D comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein
   the HCDR1 comprises or consists of an amino acid sequence set forth in SEQ ID NO: 41; the HCDR2 comprises or consists of an amino acid sequence set forth in SEQ ID NO: 42; the HCDR3 comprises or consists of an amino acid sequence set forth in SEQ ID NO: 43; the LCDR1 comprises or consists of an amino acid sequence set forth in SEQ ID NO: 45; the LCDR2 comprises or consists of an amino acid sequence set forth in SEQ ID NO: 46; and/or the LCDR3 comprises or consists of an amino acid sequence set forth in SEQ ID NO: 47; or
   the HCDR1 comprises or consists of an amino acid sequence set forth in SEQ ID NO: 49; the HCDR2 comprises or consists of an amino acid sequence set forth in SEQ ID NO: 50; the HCDR3 comprises or consists of an amino acid sequence set forth in SEQ ID NO: 51; the LCDR1 comprises or consists of an amino acid sequence set forth in SEQ ID NO: 53; the LCDR2 comprises or consists of an amino acid sequence set forth in SEQ ID NO: 54; and/or the LCDR3 comprises or consists of an amino acid sequence set forth in SEQ ID NO: 55.
9. The trispecific antibody according to embodiment 8, wherein the VH of the third antigen-binding region specifically binding to GPRC5D
   (i) comprises or consists of an amino acid sequence set forth in SEQ ID NO: 40, or an amino acid sequence having at least 90%, 91%, 92%, 93%, 94%, 95%, 96%, 97%, 98%, or 99% identity thereto; or
   (ii) comprises or consists of an amino acid sequence set forth in SEQ ID NO: 48, or an amino acid sequence having at least 90%, 91%, 92%, 93%, 94%, 95%, 96%, 97%, 98%, or 99% identity thereto.
10. The trispecific antibody according to embodiment 8 or 9, wherein the VL of the third antigen-binding region specifically binding to GPRC5D
   (i) comprises or consists of an amino acid sequence set forth in SEQ ID NO: 44, or an amino acid sequence having at least 90%, 91%, 92%, 93%, 94%, 95%, 96%, 97%, 98%, or 99% identity thereto; or
   (ii) comprises or consists of an amino acid sequence set forth in SEQ ID NO: 52, or an amino acid sequence having at least 90%, 91%, 92%, 93%, 94%, 95%, 96%, 97%, 98%, or 99% identity thereto.
11. The trispecific antibody according to embodiment 8, wherein the third antigen-binding region specifically binding to GPRC5D comprises a VH and a VL, wherein the VH consists of an amino acid sequence set forth in SEQ ID NO: 48, and the VL consists of an amino acid sequence set forth in SEQ ID NO: 52; or the VH consists of an amino acid sequence set forth in SEQ ID NO: 40, and the VL consists of an amino acid sequence set forth in SEQ ID NO: 44.
12. The trispecific antibody according to any one of embodiments 1 to 11, wherein the third antigen-binding region specifically binding to GPRC5D comprises a VH and a VL, wherein the VH comprises 39K and the VL comprises 38D; or the VH comprises 39D and the VL comprises 38K.
13. The trispecific antibody according to embodiment 12, wherein the VH of the third antigen-binding region specifically binding to GPRC5D
   (i) comprises or consists of an amino acid sequence set forth in SEQ ID NO: 80, or
   (ii) comprises an amino acid sequence having at least 90%, 91%, 92%, 93%, 94%, 95%, 96%, 97%, 98%, or 99% identity to an amino acid sequence set forth in SEQ ID NO: 80, and comprises a Q39K mutation;
   and the VL of the third antigen-binding region
   (i) comprises or consists of an amino acid sequence set forth in SEQ ID NO: 79, or
   (ii) comprises an amino acid sequence having at least 90%, 91%, 92%, 93%, 94%, 95%, 96%, 97%, 98%, or 99% identity to an amino acid sequence set forth in SEQ ID NO: 79, and comprises a Q38D mutation.
14. The trispecific antibody according to any one of embodiments 1 to 13, wherein the third antigen-binding region specifically binding to GPRC5D is an Fab.
15. The trispecific antibody according to any one of embodiments 1 to 14, wherein the second antigen-binding region specifically binding to CD3 comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein
   the HCDR1 comprises or consists of an amino acid sequence set forth in SEQ ID NO: 65; the HCDR2 comprises or consists of an amino acid sequence set forth in SEQ ID NO: 66; the HCDR3 comprises or consists of an amino acid sequence set forth in SEQ ID NO: 67 or 73; the LCDR1 comprises or consists of an amino acid sequence set forth in SEQ ID NO: 69; the LCDR2 comprises or consists of an amino acid sequence set forth in SEQ ID NO: 70; and/or the LCDR3 comprises or consists of an amino acid sequence set forth in SEQ ID NO: 71.
16. The trispecific antibody according to embodiment 15, wherein the VH of the second antigen-binding region specifically binding to CD3 comprises or consists of an amino acid sequence set forth in SEQ ID NO: 64 or 72, or an amino acid sequence having at least 90%, 91%, 92%, 93%, 94%, 95%, 96%, 97%, 98%, or 99% identity thereto.
17. The trispecific antibody according to embodiment 15 or 16, wherein the VL of the second antigen-binding region specifically binding to CD3 comprises or consists of an amino acid sequence set forth in SEQ ID NO: 68, or an amino acid sequence having at least 90%, 91%, 92%, 93%, 94%, 95%, 96%, 97%, 98%, or 99% identity thereto.
18. The trispecific antibody according to embodiment 15, wherein the second antigen-binding region specifically binding to CD3 comprises a VH and a VL, wherein the VH consists of an amino acid sequence set forth in SEQ ID NO: 64 or 72, and the VL of the second antigen-binding region consists of an amino acid sequence set forth in SEQ ID NO: 68.
19. The trispecific antibody according to any one of embodiments 15 to 18, wherein the second antigen-binding region specifically binding to CD3 comprises a VH and a VL, wherein the VH comprises 39K and the VL comprises 38D; or the VH comprises 39D and the VL comprises 38K.
20. The trispecific antibody according to embodiment 19, wherein the VH of the second antigen-binding region specifically binding to CD3
   (i) comprises or consists of an amino acid sequence set forth in SEQ ID NO: 77 or 78, or
   (ii) comprises an amino acid sequence having at least 90%, 91%, 92%, 93%, 94%, 95%, 96%, 97%, 98%, or 99% identity to an amino acid sequence set forth in SEQ ID NO: 77 or 78, and comprises a Q39D mutation;
   and the VL of the second antigen-binding region specifically binding to CD3
   (i) comprises or consists of an amino acid sequence set forth in SEQ ID NO: 74, or
   (ii) comprises an amino acid sequence having at least 90%, 91%, 92%, 93%, 94%, 95%, 96%, 97%, 98%, or 99% identity to an amino acid sequence set forth in SEQ ID NO: 74, and comprises a Q38K mutation.
21. The trispecific antibody according to any one of embodiments 1 to 20, wherein the second antigen-binding region specifically binding to CD3 is an Fab.
22. The trispecific antibody according to any one of embodiments 1 to 21, wherein the first antigen-binding region, the second antigen-binding region, and the third antigen-binding region are a first Fab, a second Fab, and a third Fab, respectively.
23. The trispecific antibody according to embodiment 22, wherein the first Fab or the third Fab further comprises a disulfide bond remodeling mutation.
24. The trispecific antibody according to embodiment 23, wherein the disulfide bond remodeling mutation comprises F126C in a CH1 of an Fab and comprises Q124C in a CL of the Fab.
25. The trispecific antibody according to embodiment 24, wherein the Fab comprising a disulfide bond remodeling mutation comprises a CH1, wherein the CH1
   (i) comprises an amino acid sequence having at least 90% identity to an amino acid sequence set forth in SEQ ID NO: 85, and comprises F126C; or
   (ii) comprises or consists of an amino acid sequence set forth in SEQ ID NO: 85.
26. The trispecific antibody according to embodiment 24 or 25, wherein the Fab comprising a disulfide bond remodeling mutation comprises a CL, wherein the CL
   (i) comprises an amino acid sequence having at least 90% identity to an amino acid sequence set forth in SEQ ID NO: 97, and comprises Q124C; or
   (ii) comprises or consists of an amino acid sequence set forth in SEQ ID NO: 97.
27. The trispecific antibody according to embodiment 24, wherein the Fab comprising a disulfide bond remodeling mutation comprises a CH1 and a CL, wherein the CH1 comprises or consists of an amino acid sequence set forth in SEQ ID NO: 85, and the CL comprises or consists of an amino acid sequence set forth in SEQ ID NO: 97.
28. The trispecific antibody according to embodiment 24, wherein the disulfide bond remodeling mutation comprises F126C and C220S in a CH1 of an Fab and comprises Q124C and C214S in a CL of the Fab.
29. The trispecific antibody according to embodiment 28, wherein the Fab comprising a disulfide bond remodeling mutation comprises a CH1, wherein the CH1
   (i) comprises an amino acid sequence having at least 90% identity to an amino acid sequence set forth in SEQ ID NO: 86, and comprises F126C and C220S; or
   (ii) comprises or consists of an amino acid sequence set forth in SEQ ID NO: 86.
30. The trispecific antibody according to embodiment 28 or 29, wherein the Fab comprising a disulfide bond remodeling mutation comprises a CL, wherein the CL
   (i) comprises an amino acid sequence having at least 90% identity to an amino acid sequence set forth in SEQ ID NO: 98, and comprises Q124C; or
   (ii) comprises or consists of an amino acid sequence set forth in SEQ ID NO: 98.
31. The trispecific antibody according to embodiment 28, wherein the Fab comprising a disulfide bond remodeling mutation comprises a CH1 and a CL, wherein the CH1 comprises or consists of an amino acid sequence set forth in SEQ ID NO: 86, and the CL comprises or consists of an amino acid sequence set forth in SEQ ID NO: 98.
32. The trispecific antibody according to any one of embodiments 1 to 31, wherein the trispecific antibody comprises a first Fc region and a second Fc region, wherein the first Fc region and the second Fc region are identical or different.
33. The trispecific antibody according to embodiment 32, wherein the first Fc region and the second Fc region are human IgG Fcs, such as human IgG1 Fc, human IgG2 Fc, human IgG3 Fc, or human IgG4 Fc, for example, comprising or consisting of an amino acid sequence set forth in SEQ ID NO: 87 or 88, or an amino acid sequence having at least 90% identity, e.g., 95%, 96%, 97%, 99% or more identity, thereto.
34. The trispecific antibody according to embodiment 33, wherein the first and/or second Fc region comprises an L234A/L235A mutation.
35. The trispecific antibody according to embodiment 34, wherein the first and/or second Fc region comprises an amino acid sequence set forth in SEQ ID NO: 89 or 90, or comprises an amino acid sequence having at least 90% identity, e.g., 95%, 96%, 97%, 99% or more identity, to an amino acid sequence set forth in SEQ ID NO: 89 or 90 and comprises an amino acid substitution L234A/L235A.
36. The trispecific antibody according to any one of embodiments 32 to 35, wherein one of the first and second Fc regions comprises a Knob mutation and the other comprises a Hole mutation.
37. The trispecific antibody according to embodiment 36, wherein the one Fc region comprises an amino acid substitution T366W, and the other Fc region comprises amino acid substitutions T366S, L368A and Y407V (numbering according to EU index).
38. The trispecific antibody according to embodiment 37, wherein one Fc region polypeptide comprises an amino acid sequence having at least 85%, 90%, 95%, 96%, 97%, 98%, or 99% identity to an amino acid sequence set forth in SEQ ID NO: 91, and the other Fc region polypeptide comprises an amino acid sequence having at least 85%, 90%, 95%, 96%, 97%, 98%, or 99% identity to an amino acid sequence set forth in SEQ ID NO: 92.
39. The trispecific antibody according to embodiment 32, wherein the one Fc region comprises amino acid substitutions L234A/L235A and T366W, and the other Fc region comprises amino acid substitutions L234A/L235A, T366S, L368A and Y407V (numbering according to EU index).
40. The trispecific antibody according to embodiment 39, wherein one Fc region polypeptide comprises an amino acid sequence having at least 85%, 90%, 95%, 96%, 97%, 98%, or 99% identity to an amino acid sequence set forth in SEQ ID NO: 94, and the other Fc region polypeptide comprises an amino acid sequence having at least 85%, 90%, 95%, 96%, 97%, 98%, or 99% identity to an amino acid sequence set forth in SEQ ID NO: 93.
41. The trispecific antibody according to any one of embodiments 1 to 40, wherein the first antigen-binding region, the second antigen-binding region, and the third antigen-binding region are a first Fab, a second Fab, and a third Fab, respectively, wherein
   (1) the first Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the third Fab is fused to N-terminus of a VH of the Fab heavy chain of the second Fab fragment at C-terminus of a CH1 of an Fab heavy chain;
      or
      the third Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the first Fab is fused to N-terminus of a VH of the Fab heavy chain of the second Fab fragment at C-terminus of a CH1 of an Fab heavy chain;
      or
   (2) the first Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the third Fab is fused to C-terminus of the Fc region fused to the second Fab at N-terminus of a VH of an Fab heavy chain; or
      the third Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the first Fab is fused to C-terminus of the Fc region fused to the second Fab at N-terminus of a VH of an Fab heavy chain.
42. The trispecific antibody according to embodiment 41, wherein the fusion comprises direct fusion or fusion through a linker.
43. The trispecific antibody according to embodiment 42, wherein the linker is (GGGGS)n, wherein n = 1, 2, 3, or 4.
44. The trispecific antibody according to any one of embodiments 41 to 43, wherein the second Fab comprises a charge mutation comprising a Q38K or Q38D mutation in the Fab light chain and a Q39D or Q39K mutation in the Fab heavy chain.
45. The trispecific antibody according to any one of embodiments 41 to 44, wherein in the first or third Fab fused to the second Fab or to the second Fab through the Fc region, the Fab heavy chain comprises Q39K or Q39D and F126C mutations, and the Fab light chain comprises Q38D or Q38K and Q124C mutations; or
   in the first or third Fab fused to the second Fab or to the second Fab through the Fc region, the Fab heavy chain comprises Q39K or Q39D and F126C mutations and C220S, and the Fab light chain comprises Q38D or Q38K and Q124C mutations and C214S. 46. The trispecific antibody according to any one of embodiments 41 to 43, wherein the second Fab comprises a charge mutation comprising a Q38K mutation in the Fab light chain and a Q39D mutation in the Fab heavy chain; and in the first or third Fab fused to the second Fab or to the second Fab through the Fc region, the Fab heavy chain comprises Q39K and F126C mutations, and the Fab light chain comprises Q38D and Q124C mutations; or
   the second Fab comprises a charge mutation comprising a Q38K mutation in the Fab light chain and a Q39D mutation in the Fab heavy chain; and in the first or third Fab fused to the second Fab or to the second Fab through the Fc region, the Fab heavy chain comprises Q39K and F126C mutations and C220S, and the Fab light chain comprises Q38D and Q124C mutations and C214S; or
   the second Fab comprises a charge mutation comprising a Q38D mutation in the Fab light chain and a Q39K mutation in the Fab heavy chain; and in the first or third Fab fused to the second Fab or to the second Fab through the Fc region, the Fab heavy chain comprises Q39D and F126C mutations, and the Fab light chain comprises Q38K and Q124C mutations; or
   the second Fab comprises a charge mutation comprising a Q38D mutation in the Fab light chain and a Q39K mutation in the Fab heavy chain; and in the first or third Fab fused to the second Fab or to the second Fab through the Fc region, the Fab heavy chain comprises Q39D and F126C mutations and C220S, and the Fab light chain comprises Q38K and Q124C mutations and C214S.
47. The trispecific antibody according to any one of embodiments 1 to 46, wherein the trispecific antibody is a left-right asymmetric IgG-like pentamer consisting of 5 polypeptide chains and consists of the following peptide chains:
   a peptide chain 1# sequentially comprising an Fab heavy chain specifically binding to GPRC5D and an Fc domain,
   a peptide chain 2# comprising an Fab light chain specifically binding to GPRC5D,
   a peptide chain 3# sequentially comprising an Fab heavy chain specifically binding to BCMA, an Fab heavy chain specifically binding to CD3, and an Fc domain,
   a peptide chain 4# comprising an Fab light chain specifically binding to BCMA, and
   a peptide chain 5# comprising an Fab light chain specifically binding to CD3,
   wherein the Fab heavy chain of the peptide chain 1# is paired with the Fab light chain of the peptide chain 2# to form the third Fab, and the two Fab heavy chains of the peptide chain 3# are paired with the Fab light chains of the peptide chain 4# and the peptide chain 5# to form the first Fab and the second Fab, respectively.
48. The trispecific antibody according to embodiment 47, wherein
   the peptide chain 1# comprises or consists of an amino acid sequence set forth in SEQ ID NO: 5, or an amino acid sequence having at least 85%, 90%, 91%, 92%, 93%, 94%, 95%, 96%, 97%, 98%, or 99% identity to an amino acid sequence set forth in SEQ ID NO: 5;
   the peptide chain 2# comprises or consists of an amino acid sequence set forth in SEQ ID NO: 4, or an amino acid sequence having at least 85%, 90%, 91%, 92%, 93%, 94%, 95%, 96%, 97%, 98%, or 99% identity to an amino acid sequence set forth in SEQ ID NO: 4;
   the peptide chain 3# comprises or consists of an amino acid sequence set forth in SEQ ID NO: 3 or 6, or an amino acid sequence having at least 85%, 90%, 91%, 92%, 93%, 94%, 95%, 96%, 97%, 98%, or 99% identity to an amino acid sequence set forth in SEQ ID NO: 3 or 6;
   the peptide chain 4# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2; and/or
   the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.
49. The trispecific antibody according to embodiment 47, wherein
   the peptide chain 1# comprises or consists of an amino acid sequence set forth in SEQ ID NO: 15, or an amino acid sequence having at least 85%, 90%, 91%, 92%, 93%, 94%, 95%, 96%, 97%, 98%, or 99% identity to an amino acid sequence set forth in SEQ ID NO: 15;
   the peptide chain 2# comprises or consists of an amino acid sequence set forth in SEQ ID NO: 14, or an amino acid sequence having at least 85%, 90%, 91%, 92%, 93%, 94%, 95%, 96%, 97%, 98%, or 99% identity to an amino acid sequence set forth in SEQ ID NO: 14;
   the peptide chain 3# comprises or consists of an amino acid sequence set forth in SEQ ID NO: 3 or 6, or an amino acid sequence having at least 85%, 90%, 91%, 92%, 93%, 94%, 95%, 96%, 97%, 98%, or 99% identity to an amino acid sequence set forth in SEQ ID NO: 3 or 6;
   the peptide chain 4# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2; and/or
   the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.
50. The trispecific antibody according to any one of embodiments 1 to 46, wherein the trispecific antibody is a left-right asymmetric IgG-like pentamer consisting of 5 polypeptide chains and consists of the following peptide chains:
   a peptide chain 1# sequentially comprising an Fab heavy chain specifically binding to BCMA and an Fc domain,
   a peptide chain 2# comprising an Fab light chain specifically binding to BCMA,
   a peptide chain 3# sequentially comprising an Fab heavy chain specifically binding to GPRC5D, an Fab heavy chain specifically binding to CD3, and an Fc domain,
   a peptide chain 4# comprising an Fab light chain specifically binding to GPRC5D, and
   a peptide chain 5# comprising an Fab light chain specifically binding to CD3,
   wherein the Fab heavy chain of the peptide chain 1# is paired with the Fab light chain of the peptide chain 2# to form the first Fab, and the two Fab heavy chains of the peptide chain 3# are paired with the Fab light chains of the peptide chain 4# and the peptide chain 5# to form the third Fab and the second Fab, respectively.
51. The trispecific antibody according to embodiment 50, wherein
   the peptide chain 1# comprises or consists of an amino acid sequence set forth in SEQ ID NO: 11, or an amino acid sequence having at least 85%, 90%, 91%, 92%, 93%, 94%, 95%, 96%, 97%, 98%, or 99% identity to an amino acid sequence set forth in SEQ ID NO: 11;
   the peptide chain 2# comprises or consists of an amino acid sequence set forth in SEQ ID NO: 10, or an amino acid sequence having at least 85%, 90%, 91%, 92%, 93%, 94%, 95%, 96%, 97%, 98%, or 99% identity to an amino acid sequence set forth in SEQ ID NO: 10;
   the peptide chain 3# comprises or consists of an amino acid sequence set forth in SEQ ID NO: 9, or an amino acid sequence having at least 85%, 90%, 91%, 92%, 93%, 94%, 95%, 96%, 97%, 98%, or 99% identity to an amino acid sequence set forth in SEQ ID NO: 9;
   the peptide chain 4# comprises or consists of an amino acid sequence set forth in SEQ ID NO: 8, or an amino acid sequence having at least 85%, 90%, 91%, 92%, 93%, 94%, 95%, 96%, 97%, 98%, or 99% identity to an amino acid sequence set forth in SEQ ID NO: 8; and/or
   the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.
52. The trispecific antibody according to any one of embodiments 1 to 46, wherein the trispecific antibody is a left-right asymmetric IgG-like pentamer consisting of 5 polypeptide chains and consists of the following peptide chains:
   a peptide chain 1# sequentially comprising an Fab heavy chain specifically binding to GPRC5D and an Fc domain,
   a peptide chain 2# comprising an Fab light chain specifically binding to GPRC5D,
   a peptide chain 3# sequentially comprising an Fab heavy chain specifically binding to CD3, an Fc domain, and an Fab heavy chain of the antigen-binding region specifically binding to BCMA,
   a peptide chain 4# comprising an Fab light chain specifically binding to CD3, and
   a peptide chain 5# comprising an Fab light chain of the antigen-binding region specifically binding to BCMA, wherein the Fab heavy chain of the peptide chain 1# is paired with the Fab light chain of the peptide chain 2# to form the third Fab, and the two Fab heavy chains of the peptide chain 3# are paired with the Fab light chains of the peptide chain 4# and the peptide chain 5# to form the second Fab and the first Fab, respectively.
53. The trispecific antibody according to embodiment 52, wherein
   the peptide chain 1# comprises or consists of an amino acid sequence set forth in SEQ ID NO: 5, or an amino acid sequence having at least 85%, 90%, 91%, 92%, 93%, 94%, 95%, 96%, 97%, 98%, or 99% identity to an amino acid sequence set forth in SEQ ID NO: 5;
   the peptide chain 2# comprises or consists of an amino acid sequence set forth in SEQ ID NO: 4, or an amino acid sequence having at least 85%, 90%, 91%, 92%, 93%, 94%, 95%, 96%, 97%, 98%, or 99% identity to an amino acid sequence set forth in SEQ ID NO: 4;
   the peptide chain 3# comprises or consists of an amino acid sequence set forth in SEQ ID NO: 7, or an amino acid sequence having at least 85%, 90%, 91%, 92%, 93%, 94%, 95%, 96%, 97%, 98%, or 99% identity to an amino acid sequence set forth in SEQ ID NO: 7;
   the peptide chain 4# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1; and/or
   the peptide chain 5# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid
   sequence set forth in SEQ ID NO: 2.
54. A nucleic acid molecule encoding any one of the chains of the trispecific antibody according to any one of embodiments 1 to 53, or consisting of a nucleic acid sequence.
55. An expression vector comprising the nucleic acid molecule according to embodiment 54, wherein, for example, the expression vector is a pCNDA vector, such as a pCDNA3.1 expression vector.
56. A host cell comprising the nucleic acid molecule according to embodiment 54 or the expression vector according to embodiment 52, wherein, preferably, the host cell is prokaryotic or eukaryotic, e.g., a 293 cell or a CHO cell, such as an HEK293 cell.
57. A method for preparing the trispecific antibody according to any one of embodiments 1 to 53, wherein the method comprises culturing the host cell comprising the nucleic acid molecule according to embodiment 54 or the expression vector according to embodiment 55 under a condition suitable for expressing the chains of the antibody, and optionally recovering the antibody from the host cell (or a host cell medium).
58. An immunoconjugate comprising the trispecific antibody according to any one of embodiments 1 to 53.
59. A pharmaceutical composition or a drug or a formulation comprising the trispecific antibody according to any one of embodiments 1 to 53 or the immunoconjugate according to embodiment 58, and optionally a pharmaceutical supplementary material.
60. A pharmaceutical combination comprising the trispecific antibody according to any one of embodiments 1 to 53 or the immunoconjugate according to embodiment 55, and one or more additional therapeutic agents, such as a chemotherapeutic agent.
61. A method for preventing or treating a cancer in a subject, comprising administering to the subject an effective amount of the trispecific antibody according to any one of embodiments 1 to 53, or the immunoconjugate according to embodiment 58, or the pharmaceutical composition or the formulation according to embodiment 59, or the pharmaceutical combination according to embodiment 57.
62. The method according to embodiment 61, wherein the cancer is a GPRC5D-single-positive cancer, a BCMA-single-positive cancer, a GPRC5D/BCMA-double-positive cancer, or a cancer with double low expression of BCMA and GPRC5D, such as a cancer with low expression of GPRC5D and/or low expression of BCMA (e.g., a cancer that recurs after anti-BCMA therapy or anti-GPRC5D therapy).
63. The method according to embodiment 61 or 62, wherein the cancer is a solid tumor or a hematological tumor, for example, the cancer is multiple myeloma.
64. The method according to any one of embodiments 58 to 60, wherein the method further comprises administering in combination with additional therapies, such as therapeutic modalities (e.g., surgical therapy or radiotherapy) and/or additional therapeutic agents (e.g., a chemotherapeutic agent).

**It should be understood that the present invention also encompasses any combination of any technical features or any combination of any technical solutions described herein.**

### Examples

### Example 1. Molecular Structure Design and Construction of Trispecific Antibodies (Ts)

Exemplary antibodies were multispecific antibodies of T-cell engager class simultaneously targeting GPRC5D, BCMA, and CD3, which can simultaneously bind to two types of tumor-associated antigens (i.e., GPRC5D and BCMA) on the surface of multiple myeloma (MM) cells and CD3 receptors on the surface of T cells. Multispecific antibodies targeting GPRC5D, BCMA, and CD3 simultaneously and containing disulfide bond remodeling mutations and charge mutations were designed based on two anti-GPRC5D antibodies (hz7F5.3 and hz5E12.1.P1) with different affinities, one anti-BCMA antibody (ADI-38497), and two anti-CD3 antibodies (hzsp34.24 and hzsp34.87) with different affinities. The sequence numbers and specific sequences of the variable regions and CDR sequences of the three types of antibodies described above are shown in sequence information.

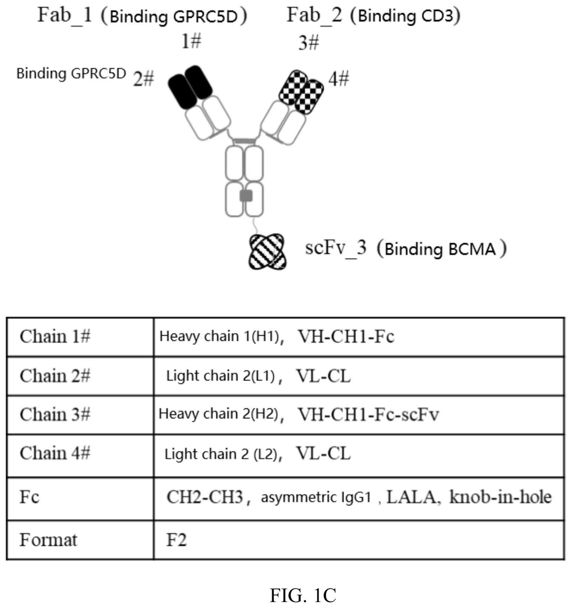

Specifically, in the present invention, 6 exemplary antibodies of the 1+1+1 structure (B3, B3b, B5) as described in FIG. 1 and Table 1 were designed, and a superior potency over bispecific antibodies was achieved. Further, in the exemplary antibody design, light chain mispairing of non-BCMA and CD3 Fab was resolved using specific mutation techniques (disulfide bond remodeling mutations and charge mutations, see sequence information table for specific mutation information), and heavy chain mispairing of asymmetric IgG-like bispecific antibody in Fc portion was resolved using a "knob-in-hole" technique, wherein the Fc is a heavy chain constant region of IgG1 with the introduction of amino acid mutations L234A and L235A ("EU" numbering) that weaken the effector function.

The design structures of the 6 exemplary antibodies described above are shown in FIG. 1 and Table 1.

**Table 1. Fragment numbers and descriptions of exemplary antibodies**

| | Exemplary antibodies | Structure |
|---|---|---|
| Exemplary antibody No. 1 | B3(24) | B3 |
| Exemplary antibody No. 2 | B3(87) | B3 |
| Exemplary antibody No. 3 | B5(87) | B5 |
| Exemplary antibody No. 4 | B3b (87) | B3b |
| Exemplary antibody No. 5 | B3-7F5.3 (87) | B3 |
| Exemplary antibody No. 6 | B3-7F5.3 (24) | B3 |

The structures of the 6 exemplary antibodies described above were in the B3, B3b, and B5 formats.

The B3 format (see FIG. 1A for specific information such as chain #) was a left-right asymmetric IgG-like pentamer consisting of 5 polypeptide chains and consisted of the following peptide chains:
a peptide chain 1# sequentially comprising an Fab heavy chain specifically binding to GPRC5D and an Fc domain,
a peptide chain 2# comprising an Fab light chain specifically binding to GPRC5D,
a peptide chain 3# sequentially comprising an Fab heavy chain specifically binding to BCMA, an Fab heavy chain specifically binding to CD3, and an Fc domain,
a peptide chain 4# comprising an Fab light chain specifically binding to BCMA, and
a peptide chain 5# comprising an Fab light chain specifically binding to CD3,
wherein the Fab heavy chain of the peptide chain 1# was paired with the Fab light chain of the peptide chain 2# to form the third Fab, and the two Fab heavy chains of the peptide chain 3# were paired with the Fab light chains of the peptide chain 4# and the peptide chain 5# to form the first Fab and the second Fab, respectively, for example, as shown in FIG. 1A-Format B3.

The B3b format (see FIG. 1A for specific information such as chain #) was a left-right asymmetric IgG-like pentamer consisting of 5 polypeptide chains and consisted of the following peptide chains:
a peptide chain 1# sequentially comprising an Fab heavy chain specifically binding to BCMA and an Fc domain,
a peptide chain 2# comprising an Fab light chain specifically binding to BCMA,
a peptide chain 3# sequentially comprising an Fab heavy chain specifically binding to GPRC5D, an Fab heavy chain specifically binding to CD3, and an Fc domain,
a peptide chain 4# comprising an Fab light chain specifically binding to GPRC5D, and
a peptide chain 5# comprising an Fab light chain specifically binding to CD3,
wherein the Fab heavy chain of the peptide chain 1# was paired with the Fab light chain of the peptide chain 2# to form the first Fab, and the two Fab heavy chains of the peptide chain 3# were paired with the Fab light chains of the peptide chain 4# and the peptide chain 5# to form the third Fab and the second Fab, respectively, for example, as shown in FIG. 1A-Format B3b.

The B5 format (see FIG. 1B for specific information such as chain #) was a left-right asymmetric IgG-like pentamer consisting of 5 polypeptide chains and consisted of the following peptide chains:
a peptide chain 1# sequentially comprising an Fab heavy chain specifically binding to GPRC5D and an Fc domain,
a peptide chain 2# comprising an Fab light chain specifically binding to GPRC5D,
a peptide chain 3# sequentially comprising an Fab heavy chain specifically binding to CD3, an Fc domain, and an Fab heavy chain of the antigen-binding region specifically binding to BCMA,
a peptide chain 4# comprising an Fab light chain specifically binding to CD3, and
a peptide chain 5# comprising an Fab light chain of the antigen-binding region specifically binding to BCMA,
wherein the Fab heavy chain of the peptide chain 1# was paired with the Fab light chain of the peptide chain 2# to form the third Fab, and the two Fab heavy chains of the peptide chain 3# were paired with the Fab light chains of the peptide chain 4# and the peptide chain 5# to form the second Fab and the first Fab, respectively, for example, as shown in FIG. 1B.

Also, in the present patent, the following bispecific or trispecific antibodies were expressed and purified: Inno-B (control Hel/BCMA/CD3, wherein Hel is hen egg lysozyme antibody, does not bind to any human-related protein, and was used as a control), Inno-G (control Hel/GPRC5D/CD3), F2 (24) (GPRC5D/BCMA/CD3, with a structure derived from WO2022174813A1 (BCMA antigen-binding region is scFv)), F2 (87) (GPRC5D/BCMA/CD3, with a structure derived from WO2022174813A1 (BCMA antigen-binding region is scFv)), JNJ-BCMA/CD3 (BCMA/CD3, derived from Patent No. WO2017031104A1, also known as Teclistamab), Roche- BCMA/CD3 (BCMA/CD3, derived from Patent No. WO2018083204A1, also known as Alnuctamab), REGN-BCMA/CD3 (BCMA/CD3, derived from Patent No. US20200024356A), JNJ-GPRC5D/CD3 (GPRC5D/CD3, derived from Patent No. WO2018017786A2), Roche- GPRC5D/CD3 (GPRC5D/CD3, derived from Patent No. WO 2019/154890 A1), Elranatamab (Pfizer BCMAxCD3, derived from Patent No. WO2016166629A1).

The following negative control antibody was also expressed and purified: anti-Hel antibody (denoted as IgG or hIgG1 in the drawings), with a heavy chain sequence of SEQ ID NO: 101 and a light chain sequence of SEQ ID NO: 100.

### Example 2. Preparation and Purification of Trispecific Antibodies

### Plasmid preparation

Nucleic acids encoding each chain of each antibody of the present invention were synthesized by GENEWIZ and separately constructed into pCDNA3.1 template plasmids. A certain amount of plasmids were prepared, filtered through a 0.22 µm filter head, and used for transient expression of cells.

### Transfection and expression

Expi293 cells (Invitrogen) were passaged according to a desired transfection volume. The cell density was adjusted to 3 × 10⁶ cells/mL the day before transfection. The cell density was adjusted again to 3 × 10⁶ cells/mL on the day of transfection. Each molecule was divided into two flasks A and B at the time of expression. Plasmids containing the peptide chains 1# and 2# were added into flask A at a ratio of 1:1, and plasmids containing the peptide chains 3#, 4#, and 5# were added into flask B at a ratio of 1:1:1. Opti-MEM medium (Gibco, Cat. No. 31985-070) with a final volume of 1/10 (v/v) was used as a transfection buffer to dilute the plasmids and the dilution was mixed well. An appropriate amount of polyethylenimine (PEI) (Polysciences, 23966) was added to the plasmids from the previous step (the mass ratio of plasmids to PEI was 1:3), mixed well and incubated at room temperature for 10 min to give a DNA/PEI mixture. The DNA/PEI mixture was gently poured into HEK293 cells, mixed well, and cultured at 37 °C, 8% CO₂ for 24 h, followed by the addition of VPA (Sigma, Cat. No. P4543-100G) to reach a final concentration of 2 mM. Then 2% (v/v) Feed (1 g/L Phytone Peptone + 1 g/L Difco Select Phytone) was added and the mixture was cultured for another 6 days.

### Purification

The culture was collected and centrifuged at 4000 rpm for 30 min. The cell supernatant was taken, filtered through a 0.45 µM filter membrane, purified by ProteinA affinity chromatography, further purified by KappaSelect affinity chromatography, and refined by LambdaFabSelect affinity chromatography. Finally, an *in vitro* recombination was performed and the intact molecules were purified by ion exchange chromatography.

The ProteinA affinity chromatography purification was specifically performed as follows: the supernatant was purified using a pre-packed column Hitrap Mabselect Sure (GE, 11-0034-95). The procedures were as follows: The packing column was equilibrated with 5 column volumes of equilibration buffer (20 mM Tris, 150 mM NaCl, pH 7.2) before purification; the collected supernatant was passed through the column, and then the column was washed with 10 column volumes of equilibration buffer to remove non-specific binding proteins; the packing was washed with 5 column volumes of eluent buffer (100 mM sodium citrate, pH 3.5), and the eluate was collected. 2 M Tris was added for neutralization to achieve a pH of 6.5. Quantitative determination of the correct pairing ratio of the purified antibody was performed by liquid chromatography-mass spectrometry (LC-MS).

The KappaSelect affinity chromatography purification was specifically performed as follows: the supernatant was purified using a pre-packed column HiTrap KappaSelect (GE, 17-5458-11). The procedures were as follows: The packing column was equilibrated with 5 column volumes of equilibration buffer (PBS, pH7.4) before purification; the collected supernatant was passed through the column, and then the column was washed with 10 column volumes of equilibration buffer to remove non-specific binding proteins; the packing was washed with 5 column volumes of eluent buffer (0.1 M glycine buffer, pH 2.5), and the eluate was collected. 2 M Tris was added for neutralization to achieve a pH of 6.5. Quantitative determination of the correct pairing ratio of the purified antibody was performed by liquid chromatography-mass spectrometry (LC-MS).

The LambdaFabSelect affinity chromatography purification was specifically performed as follows: the supernatant was purified using a pre-packed column HiTrap LambdaFabSelect (GE, 17-5482-11). The procedures were as follows: The packing column was equilibrated with 5 column volumes of equilibration buffer (PBS, pH7.4) before purification; the collected supernatant was passed through the column, and then the column was washed with 10 column volumes of equilibration buffer to remove non-specific binding proteins; the packing was washed with 5 column volumes of eluent buffer (0.1 M acetate buffer, pH 3.5), and the eluate was collected. 2 M Tris was added for neutralization to achieve a pH of 6.5. Quantitative determination of the correct pairing ratio of the purified antibody was performed by liquid chromatography-mass spectrometry (LC-MS).

### In vitro recombination

The *in vitro* recombination was specifically performed as follows: Molecule A obtained in flask A and molecule B obtained in flask B were mixed at a molar ratio of 1:1. GSH (Sigma Aldrich, G4251) at a final concentration of 5 mM and arginine (Sigma Aldrich, A5006) at a final concentration of 50 mM were added, and the mixture was incubated at 37 °C for 6 h.

### Fine purification

The ion exchange chromatography purification was specifically performed as follows: The product obtained from the *in vitro* recombination was exchanged into a pH 6.0 low-salt PB buffer (10 mM phosphate, pH 6.0) using an ultrafiltration concentration tube (MILLIPORE, Cat. No. UFC901096) and subjected to fine purification using Capto HiRes S 10/100 (GE, Cat. No. 29275879). The packing column was equilibrated with 5 column volumes of a pH 6.0 low-salt PB buffer before purification; the sample after buffer exchange was passed through the column, and then the packing column was washed with 10 column volumes of the pH 6.0 low-salt PB buffer to remove non-specific binding proteins; for elution, the concentration of pH 6.0 high-salt PB buffer (10 mM phosphate, pH 6.0, 1 M NaCl) was linearly increased (linear gradient: the ratio of pH 6.0 high-salt PB buffer was increased from 1% to 100% in 30 column volumes), and the elution main peak was collected. Buffer-exchange into PBS (Gibco, Cat. No. 70011-044) was performed using an ultrafiltration concentration tube (MILLIPORE, Cat. No. UFC901096).

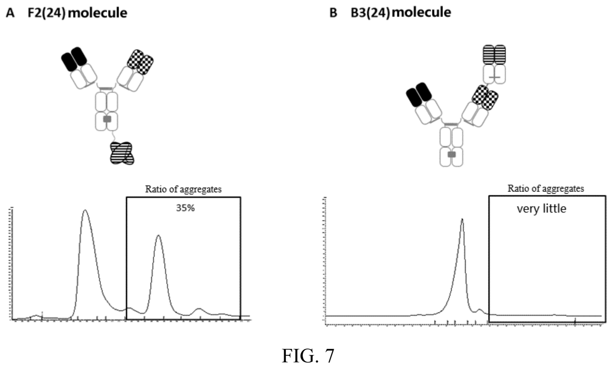

FIG. 7 shows the difference in aggregates between the B3 (24) molecule and the control molecule (F2 (24)).

The B3 (24) molecule of the present invention had a significant improvement in protein properties compared to the trispecific antibody (F2 (24)) in Patent Application No. WO2022174813A1. This is demonstrated by the fact that the ratio of aggregates produced during the cation exchange purification after the *in vitro* recombination was greatly reduced. As determined by the cation exchange chromatography, the trispecific antibody F2 (24) in Patent Application No. WO2022174813A1 showed about 35% aggregates (FIG. 7A) after the *in vitro* recombination, whereas the B3 (24) molecule produced very little or essentially no aggregates (FIG. 7B) after the *in vitro* recombination. Since aggregates are also one of the impurities to be removed in the purification process, a substantial reduction in the aggregates can lead to a greatly improved recovery rate in the purification process.

### Example 3. Determination of Affinity of Trispecific Antibodies for Antigen-Expressing Cells

### 3.1 Determination of the affinity of trispecific antibodies for antigen-expressing cells

With the exemplary trispecific antibodies, the affinity for human (h-) and cynomolgus monkey (cyno-) BCMA was assayed on GS-CHO-hBCMA and GS-CHO-cynoBCMA cell lines; the affinity for human (h-) and cynomolgus monkey (cyno-) GPRC5D was assayed on GS-CHO-hGPRC5D and GS-CHO-cynoGPRC5D cell lines; and the affinity for human CD3 was assayed on T cells in Jurkat cell line (Genomeditech, GM-C01459) and human cryopreserved PBMCs (Saily, SLB-HP050B).

The GS-CHO cell lines (GS-CHO-hGPRC5D and GS-CHO-cynoGPRCSD) over-expressing GPRC5D were obtained as follows: the nucleic acids encoding the full-length sequences of human and monkey GPRC5D (human GPRC5D (Q9NZD1, uniprot database); monkey GPRC5D (A0A2K5W6I7, uniprot database)) were separately inserted into PEE17.4 plasmids (Lonza, GS Xceed Expression System), and the constructed stably transfected cell lines were subjected to pressurized screening.

The GS-CHO cell lines (GS-CHO-hBCMA and GS-CHO-cynoBCMA) over-expressing BCMA were obtained as follows: the nucleic acids encoding the full-length sequences of human and monkey BCMA (human BCMA (Q02223, uniprot database); monkey BCMA (A0A2K5UD97, uniprot database)) were separately inserted into T2A-EGFP plasmids (GENEWIZ), and the constructed stably transfected cell lines were subjected to pressurized screening.

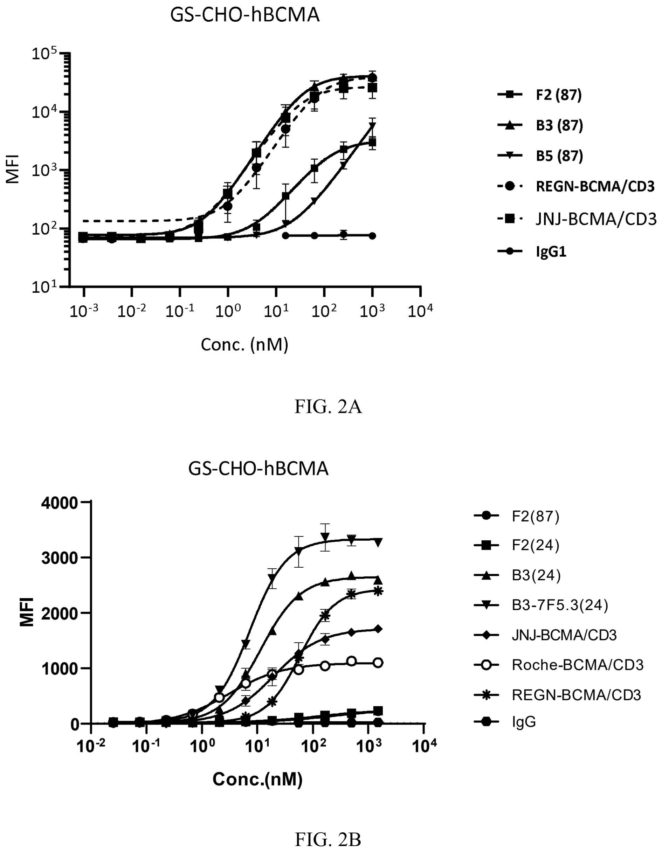

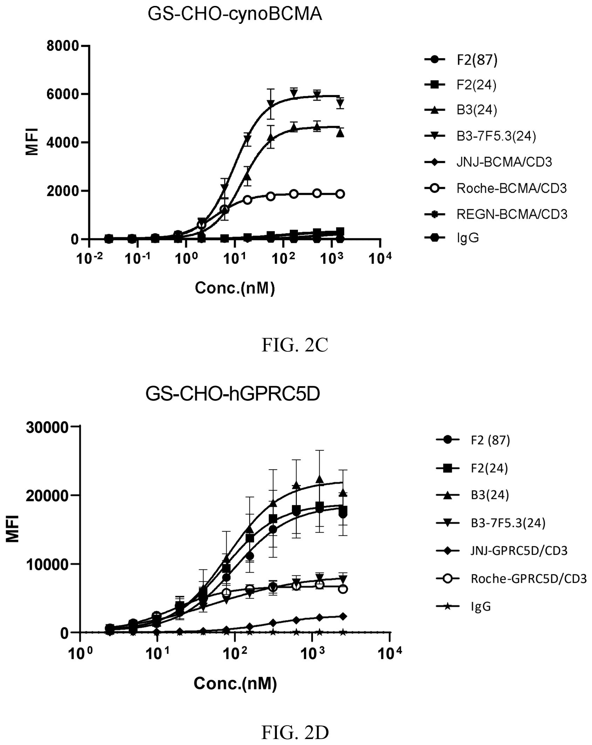

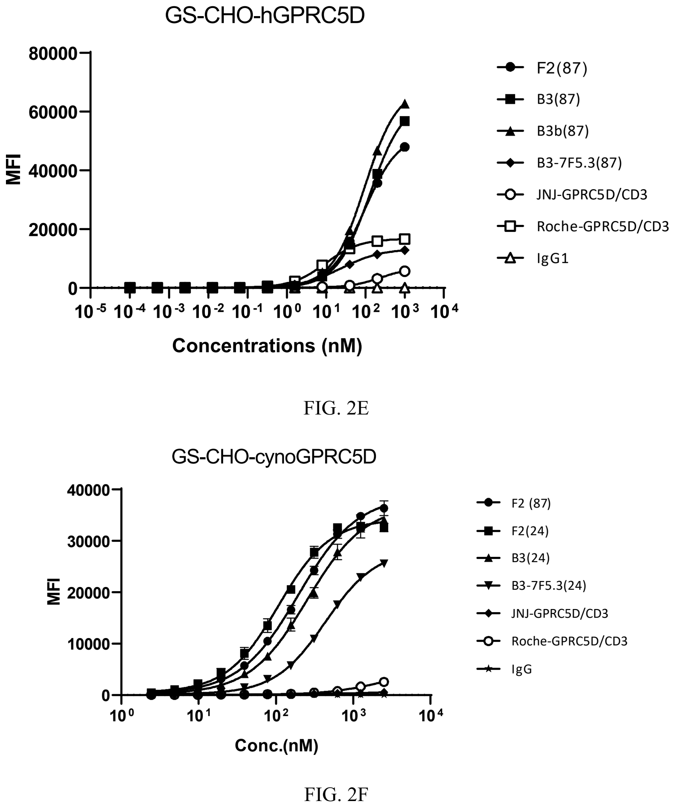

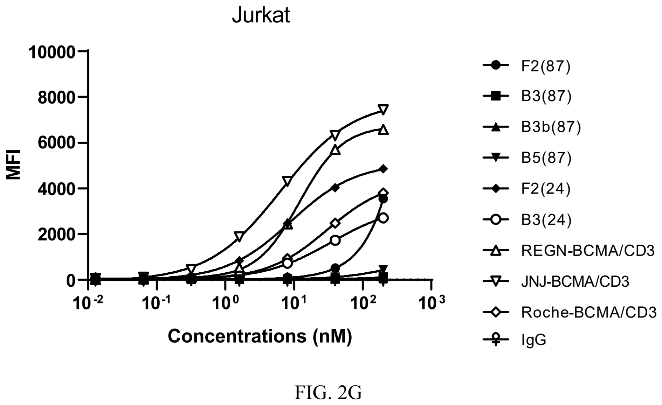

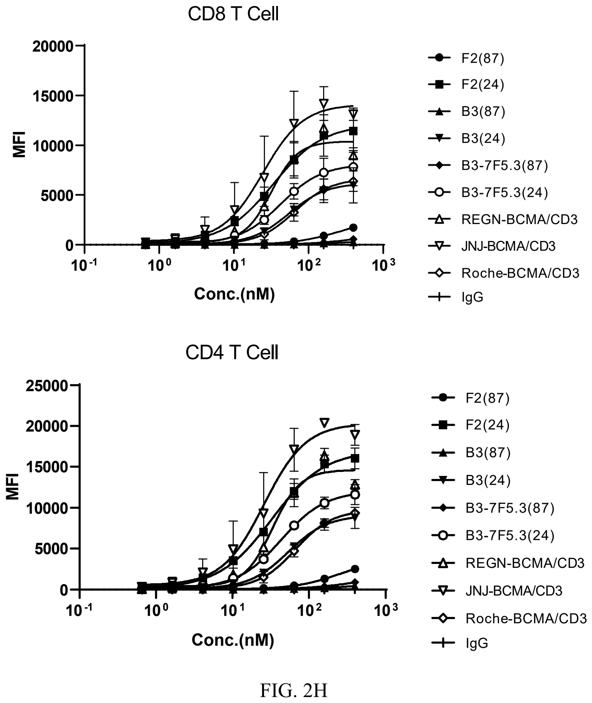

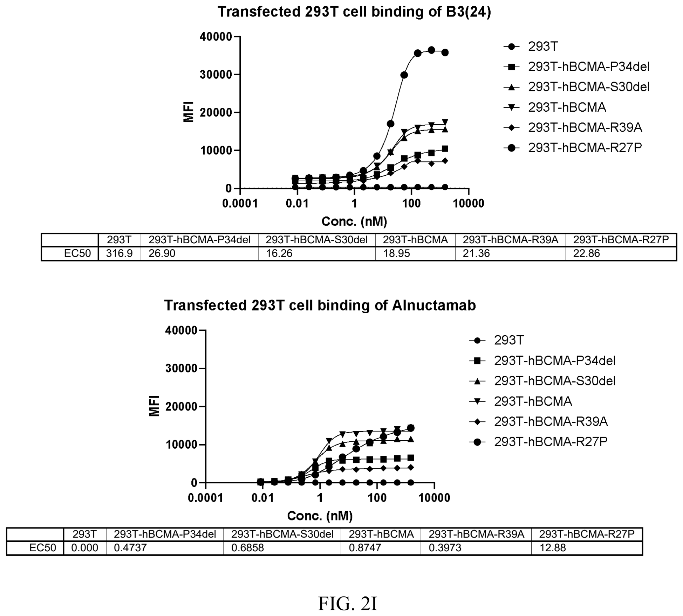

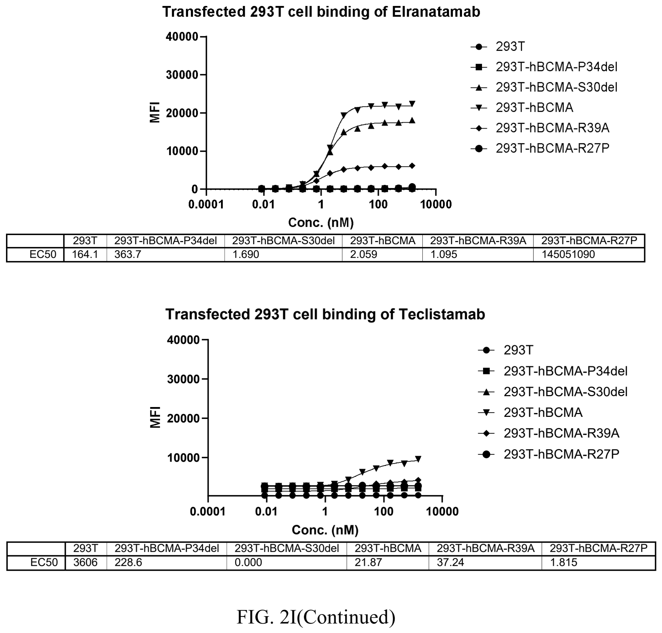

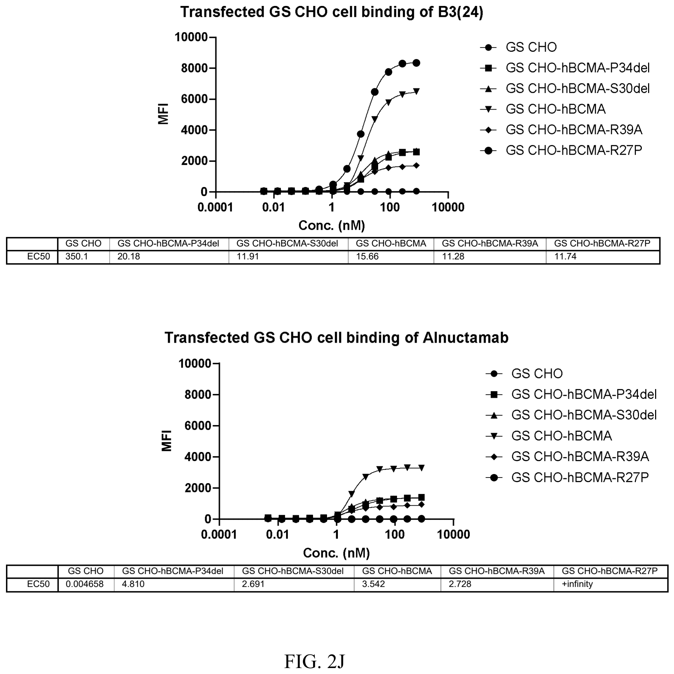

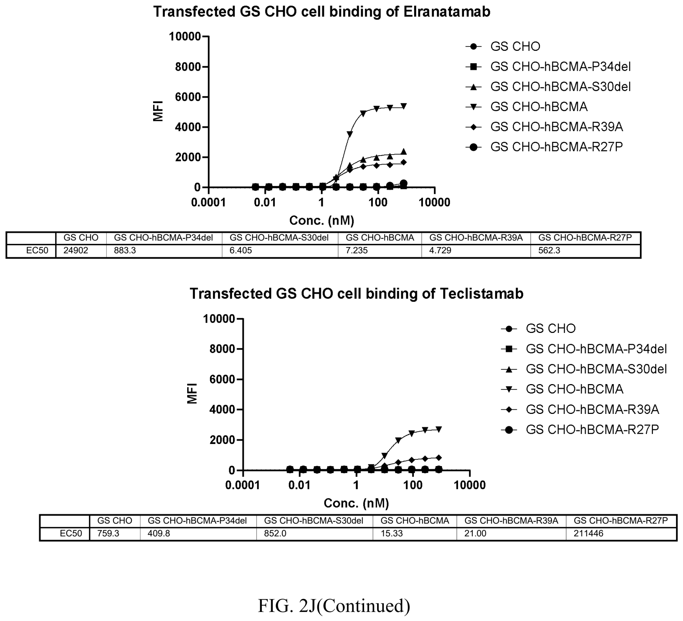

Specifically, cultured cells were obtained or T cells were isolated from human PBMCs according to the relevant instructions, and the cells were inoculated into a 96-well V-bottom plate at 1E5 cells/well. Then the molecules to be tested and the control molecules were subjected to serial dilution (see detail in FIG. 2) and then co-incubated with cells expressing the corresponding antigens at 4 °C for 40 min. A fluorescently labeled secondary antibody (diluted in a 1:200 ratio; also mixed with a staining agent DCM diluted in a 1:2000 ratio as a marker for cell viability; wherein CD4 and CD8 antibodies diluted in a 1:200 ratio were added to the isolated human T cells to label and distinguish the corresponding T cells) was then added. Finally, the fluorescence intensity of the cells was detected by a flow cytometer (BD), and then curve fitting was performed and EC₅₀ values were calculated using GraphPad Prism 8.0. FIGs. 2A-H and Tables 2-9 show the binding affinity of the antibodies of the present invention for the corresponding antigens. Reagents and materials

| Name | Cat. No. | Manufacturer |
|---|---|---|
| Pan T Cell Isolation Kit | 130-096-535 | Miltenyi Biotec |
| DCM-APC/CY7 | L34975 | Invitrogen |
| anti-human Fc-PE | ab98596 | Abcam |
| Anti-human CD4 | 612912 | BD |
| Anti-human CD8 | 612889 | BD |

The specific results are shown in FIG. 2 and Tables 2-10.

**Table 2. Affinity-1 of exemplary antibodies for human BCMA**

| Exemplary antibodies | EC50 (nM) |
|---|---|
| F2(87) | 119.6 |
| B3(87) | 37.7 |
| B5(87) | N.D. |
| REGN-BCMA/CD3 | 76.1 |
| JNJ-BCMA/CD3 | 32.1 |

| | |
|---|---|
| N.D.: not detected | |

**Table 3. Affinity-2 of exemplary antibodies for human BCMA**

| Exemplary antibodies | EC50 (nM) |
|---|---|
| F2(87) | 211.20 |
| F2(24) | 141.70 |
| B3 (24) | 11.45 |
| B3-7F5.3 (24) | 7.36 |
| JNJ-BCMA/CD3 | 19.79 |
| Roche-BCMA/CD3 | 3.08 |
| REGN-BCMA/CD3 | 59.19 |

**Table 4. Affinity-1 of exemplary antibodies for cynomolgus monkey BCMA**

| Exemplary antibodies | EC50 (nM) |
|---|---|
| F2(87) | 116.80 |
| F2(24) | 83.01 |
| B3 (24) | 14.20 |
| B3-7F5.3 (24) | 9.69 |
| JNJ-BCMA/CD3 | 829.30 |
| Roche-BCMA/CD3 | 3.81 |
| REGN-BCMA/CD3 | 1316.00 |

**Table 5. Affinity-1 of exemplary antibodies for human GPRC5D**

| Exemplary antibodies | EC50 (nM) |
|---|---|
| F2(87) | 98.56 |
| F2(24) | 73.27 |
| B3(24) | 80.73 |
| B3-7F5.3(24) | 53.40 |
| JNJ-GPRC5D/CD3 | 284.60 |
| Roche-GPRC5D/CD3 | 15.98 |

**Table 6. Affinity-2 of exemplary antibodies for human GPRC5D**

| Exemplary antibodies | EC50 (nM) |
|---|---|
| F2(87) | 94.06 |
| B3(87) | 131.70 |
| B3b (87) | 95.88 |
| B3-7F5.3 (87) | 24.40 |
| JNJ-GPRC5D/CD3 | 236.20 |
| Roche-GPRC5D/CD3 | 9.78 |

**Table 7. Affinity-1 of exemplary antibodies for cynomolgus monkey GPRC5D**

| Exemplary antibodies | EC₅₀ (nM) |
|---|---|
| F2(87) | 195.61 |
| F2(24) | 107.74 |
| B3 (24) | 255.53 |
| B3-7F5.3 (24) | 437.53 |
| JNJ-GPRC5D/CD3 | N.D. |
| Roche-GPRC5D/CD3 | N.D. |

**Table 8. Affinity-1 of exemplary antibodies for human CD3**

| Exemplary antibodies | EC₅₀ (nM) |
|---|---|
| F2(87) | N.D. |
| B3(87) | N.D. |
| B3b (87) | N.D. |
| B5(87) | N.D. |
| F2(24) | 8.94 |
| B3 (24) | 34.46 |
| REGN-BCMA/CD3 | 12.04 |
| JNJ-BCMA/CD3 | 6.39 |
| Roche-GPRC5D/CD3 | 30.43 |

**Table 9. Affinity of exemplary antibodies for human T cells**

| | EC₅₀ (nM) | |
|---|---|---|
| Exemplary antibodies | CD8 T cell | CD4 T cell |
| F2(87) | N.D. | N.D. |
| F2(24) | 33.72 | 32.49 |
| B3 (24) | 54.49 | 53.62 |
| B3(87) | N.D. | N.D. |
| B3-7F5.3 (24) | 42.42 | 43.37 |
| B3-7F5.3 (87) | N.D. | N.D. |
| REGN-BCMA/CD3 | 31.88 | 33.41 |
| JNJ-BCMA/CD3 | 24.59 | 25.88 |
| Roche-GPRC5D/CD3 | 65.82 | 66.58 |

### 3.2 Determination of the affinity of trispecific antibodies for mutant antigen-expressing cells

The method for obtaining 293T cell lines overexpressing BCMA mutants (293T-hBCMA-P34del, 293T-hBCMA-S30del, 293T-hBCMA-R39A, 293T-hBCMA-R27P) and GS CHO cell lines overexpressing BCMA mutants (GS CHO-hBCMA-P34del, GS CHO -hBCMA-S30del, GS CHO -hBCMA-R39A, GS CHO -hBCMA-R27P) was as follows: the full-length sequence of human BCMA (human BCMA (Q02223, Uniprot database) was subjected to corresponding point mutations or deletion mutations; the mutated encoding nucleic acids were inserted into the T2A-EGFP plasmids (GENEWIZ), respectively, followed by pressure screening for constructed stable cell lines. The amino acid sequences and encoding nucleic acid sequences of the full-length human BCMA sequence, hBCMA-P34del, hBCMA-S30del, hBCMA-R39A, and hBCMA-R27P are available in the sequence information.

### 1. Specific Construction Method:

293T cells were revived and passaged 2-3 times, the culture medium was discarded, the cells were then washed with 10 ml of PBS, and digested with 2 ml of trypsin; after stopping the digestion with medium, the cells were centrifuged at 400 xg for 3 min, and the supernatant was discarded. The cells were resuspended in medium, and 20 µl of the cell suspension was transferred to a 1.5 ml EP tube. The cells were counted and plated into T175 flasks at 1.5 × 10⁷ cells. The cells were shaken and placed horizontally in a 37°C incubator for overnight culture. The 293T cells that were plated in advance were removed, the culture medium was discarded, followed by adding 20 ml of DMEM culture medium containing 10% FBS and 1% PS to each flask, resulting in the transfection mixture 1:

| Reagent | Volumn |
|---|---|
| opti-MEM | 1500 µL |
| Lipo 3000 | 44.5 µL |

### Transfection mixture 2:

| Reagent | Volum |
|---|---|
| opti-MEM | 1500 µL |
| DNA plasmid | 27.42µg |
| packaging plasmid 1 | 13.72 µg |
| packaging plasmid 2 | 6.86 µg |
| P3000 | 96 µL |

Mixture 1 was slowly added dropwise to mixture 2 all at once, followed by mixing thoroughly, and standing at room temperature for 15 min.

The transfection mixture was added to the 293T cell culture medium, the flask was labeled, and shaked to evenly distribute the transfection mixture throughout the medium. The culture medium was discarded 4-6 hours after transfection and 20 mL of fresh DMEM supplemented with 2% FBS was added. The cell culture supernatants were collected 48 and 72 hours after transfection, centrifuged at 500 × g for 10 min at 4°C, and filtered through a 0.45 µm low-protein-binding filter membrane. After removing the supernatant, the cells were digested with 2 mL of trypsin; after stopping the digestion with medium, the cells were centrifuged at 400 × g for 3 min, the supernatant was discarded, the cells were resuspend in medium, and transferred to a culture flask, resulting in 293T cell lines overexpressing BCMA mutants (293T-hBCMA-P34del, 293T-hBCMA-S30del, 293T-hBCMA-R39A, 293T-hBCMA-R27P), which were placed in an incubator for continuous culture.

The viral supernatant : Takara virus concentrate =3:1 (v/v) was mixed, followed by sealing with parafilm, and incubating overnight at 4°C; the incubated mixture was centrifuged at 1500 × g for 45 min at 4°C and the supernatant was discarded; the lentiviral pellet was resuspended in 1 mL of CD CHO medium and incubated at 4°C until completely dissolved, followed by aliquoting and storing at -80°C.

Construction of a GS CHO cell line overexpressing a BCMA mutant: GS CHO cells were harvested by centrifugation at 400 × g for 4 min, the supernatant was discarded, and the cells were resuspended in 3 mL of complete medium. The cells were counted, and 1 × 10⁶ cells were plated in a 6-well plate at a ratio of virus supernatant : resuspended cells= 2:1, followed by supplementing with medium to 1 mL per well, and to 2 mL per well 4 h after infection. 24 h after infection, the cells were placed in a centrifuge tube and harvested by centrifugation at 400 × g for 4 min. The virus-containing medium was removed and the cells were resuspended in fresh CD CHO medium containing 10% FBS and 1% PS. The cells were plated into a T25 flask and cultured upright in a CO₂ incubator. The cells were supplemented with medium to 10 mL after 48 h culture, and were placed in the CO₂ incubator for continuous culture. The CHO cells cultured at 37°C were removed and harvested by centrifugation at 400 × g for 4 min. The supernatants were discarded and the cells were inoculated into a 125 mL Erlenmeyer flask and cultured in a 37°C constant temperature shaker, resulting in GS CHO cell lines overexpressing BCMA mutants (GS CHO-hBCMA-P34del, GS CHO -hBCMA-S30del, GS CHO-hBCMA-R39A, GS CHO -hBCMA-R27P).

### 2. Detection Method:

The cultured 293T cell lines overexpressing BCMA mutants (293T-hBCMA-P34del, 293T-hBCMA-S30del, 293T-hBCMA-R39A, 293T-hBCMA-R27P) were removed, followed by discarding the medium, the cells were then washed with 10 ml of PBS, and digested with 2 ml of trypsin; after stopping the digestion with medium, the cells were centrifuged at 400 × g for 5 min
, and the supernatant was discarded. The cells were resuspended in medium, and 20 µl of the cell suspension was transferred to a 1.5 ml EP tube. The cells were counted and plated into a 96-well U-bottom plate at 1E5 cells /well. The to-be-tested antibody and the control molecule were then serially diluted. The antibody was serially diluted 3-fold in PBS from an initial concentration of 1500 nM, with a total of 12 dilutions. 50 µl of the serially diluted drug was incubated with the plated cells at 4°C for 30 min, followed by adding a fluorescently labeled secondary antibody (Anti-human Fc-PE, Abcam, ab98596, diluted in a 1:200 ratio), and incubated at 4°C for 30 min. Finally, the cell fluorescence intensity was measured by flow cytometry (BD), and then curve fitting was performed and EC₅₀ values were calculated using GraphPad Prism 8.0.

The GS CHO cell lines overexpressing BCMA mutants (GS CHO-hBCMA-P34del, GS CHO-hBCMA-S30del, GS CHO-hBCMA-R39A, GS CHO-hBCMA-R27P) were removed from the culture and centrifuged at 400 × g for 5 min, the supernatant was discarded, the cells were resuspended in the medium, and 20 µl of the cell suspension was transferred to a 1.5 ml EP tube. The cells were counted and plated into a 96-well U-bottom plate at 1E5 cells/well. Then, the to-be-tested and the control molecules were serially diluted. The antibody was serially diluted 3-fold with PBS from an initial concentration of 800 nM, with a total of 12 dilutions. 50 ul of the serially diluted drugs were incubated with the plated cells at 4°C for 30 min, and then fluorescently labeled secondary antibodies (diluted in a 1:200 ratio) were added and incubated at 4°C for 30 min. Finally, the cell fluorescence intensity was measured by flow cytometry (BD), and then curve fitting was performed and EC₅₀ values were calculated using GraphPad Prism 8.0.

### 3 Experimental results:

FIG.2I and FIG.2J show the following results:
B3(24) can bind to cells overexpressing wild-type BCMA, cells overexpressing BCMA-P34del, cells overexpressing BCMA-P30del, cells overexpressing BCMA-R39A, and cells overexpressing BCMA-R27P;
Alnuctamab (Roche-BCMAxCD3) can bind to cells overexpressing wild-type BCMA, cells overexpressing BCMA-P34del, cells overexpressing BCMA-P30del, cells overexpressing BCMA-R39A, and cells overexpressing BCMA-R27P;
Elranatamab (Pfizer BCMAxCD3) can bind to cells overexpressing wild-type BCMA, cells overexpressing BCMA-P34del, and weakly bind to cells overexpressing BCMA-R39A;
Teclistamab/JNJ-BCMA/CD3 (J&J BCMAxCD3) can weakly bind to cells overexpressing wild-type BCMA and extremely weakly bind to cells overexpressing BCMA-R39A.

### Example 4. Determination of Affinity of Trispecific Antibodies by BLI

For the trispecific exemplary antibodies of the present invention, the affinity of the anti-BCMA end and the anti-CD3 end was detected by bio-layer interferometry (BLI). In the present invention, the binding kinetics of exemplary antibodies for human BCMA, monkey BCMA, human CD3 E&G, and monkey CD3 E&G were determined based on BLI using a fiber optic biosensor. When biomolecules bind to the surface of the sensor, a layer of biofilm is formed and causes an interference phenomenon to the waveform of light transmitted through the sensor, and the interference phenomenon is detected in a phase-shift manner, so that changes in the number of molecules binding to the sensor can be detected; and a kinetic curve is fitted from the changes in real-time response values, and kₒₙ, k_{off}, and K_{D} are calculated.

Specifically, sensors coupled with streptavidin protein (SA) or anti-human-Fc (AHC) were pre-wetted by immersion in 200 µL of SD buffer (1× PBS, 0.1% BSA, 0.05% tween-20). Exemplary antibodies and biotin-labeled or Fc-tagged BCMA and CD3 E&G heterodimer antigens were separately diluted in SD buffer, and then 200 µL of SD buffer was taken and each diluted sample (100 nM) was separately added into a 96-well black plate. The sensors and the samples were placed in Octet (Fortebio, Red96e). Data Acquisition 10.0 was executed and "New Kinetic Experiment" was selected. The sensors were arranged according to the positions of the samples, and the operation procedures and times were set as: Baseline 60 s, Loading 250 s, Baseline 100 s, Association 600 s and Dissociation 600 s. The experiment was conducted at 1000 rpm and 30 °C.

The results were analyzed in "Data Analysis 10.0" software, with the buffer reference channel being subtracted. 1:1 binding was selected to fit the data, and kₒₙ, k_{off}, K_{D} values for the exemplary antibodies were calculated.

### Antigen information

| Antigen/receptor name | Cat. No. | Manufacturer |
|---|---|---|
| Human BCMA | BCA-H522Y | ACRO |
| Cyno BCMA | BCA-C52H7 | ACRO |
| Human CD3E&G | CDG-H52W5 | ACRO |
| Cyno CD3E&G | CDG-C52W6 | ACRO |

### Reagent and material information

| Name | Cat. No. | Manufacturer |
|---|---|---|
| 1 *PBS | 10010-23 | Gibco |
| Tween-20 | A600560-0500 | BBI life sciences |
| BSA | 001-000-162 | Jackson Immuno Research |
| SA sensor | 18-5019 | Fortebio |
| 96-well black microplate | 655209 | Greiner |

The results of the experiments performed as described in the above assay are shown in Table 10. The results show that the affinity for BCMA and CD3 of the 4 exemplary antibodies of B3 was close to that of the F2 antibodies.

**Table 10. Affinity of exemplary antibodies as detected by BLI**

| Name | Antigen | KD (M) | kon(1/Ms) | kdis(1/s) |
|---|---|---|---|---|
| F2(87) | human CD3E&G-His/Free | 5.50E-08 | 2.39E+05 | 1.31E-02 |
| F2(24) | | 1.72E-08 | 4.45E+05 | 7.65E-03 |
| B3(87) | | 6.94E-08 | 2.18E+05 | 1.51E-02 |
| B3(24) | | 1.33E-08 | 6.46E+05 | 8.59E-03 |
| B3-7F5.3(87) | | 6.41E-08 | 2.36E+05 | 1.52E-02 |
| B3-7F5.3(24) | | 1.44E-08 | 6.08E+05 | 8.74E-03 |
| REGN-BCMA/CD3 | | N.D. | N.D. | N.D. |
| JNJ-BCMA/CD3 | | 1.54E-08 | 3.70E+05 | 5.70E-03 |
| Roche-BCMA/CD3 | | 1.30E-08 | 3.61E+05 | 4.69E-03 |
| F2(87) | cyno CD3E&G-His/Free | 7.11E-08 | 1.75E+05 | 1.24E-02 |
| F2(24) | | 1.25E-08 | 4.76E+05 | 5.97E-03 |
| B3(87) | | 6.11E-08 | 2.14E+05 | 1.31E-02 |
| B3(24) | | 9.51E-09 | 6.92E+05 | 6.58E-03 |
| B3-7F5.3(87) | | 5.86E-08 | 2.05E+05 | 1.20E-02 |
| B3-7F5.3(24) | | 1.02E-08 | 6.62E+05 | 6.75E-03 |
| REGN-BCMA/CD3 | | N.D. | N.D. | N.D. |
| JNJ-BCMA/CD3 | | 1.21E-08 | 3.96E+05 | 4.81E-03 |
| Roche-BCMA/CD3 | | 1.05E-08 | 3.77E+05 | 3.96E-03 |
| F2(87) | human BCMA-His | 1.33E-09 | 5.97E+05 | 7.95E-04 |
| B3(87) | | 1.24E-09 | 7.39E+05 | 9.16E-04 |
| B3-7F5.3(87) | | 1.04E-09 | 7.87E+05 | 8.20E-04 |
| REGN-BCMA/CD3 | | 1.13E-09 | 7.01E+05 | 7.92E-04 |
| JNJ-BCMA/CD3 | | 4.35E-10 | 1.66E+06 | 7.22E-04 |
| Roche-BCMA/CD3 | | 2.05E-10 | 1.28E+06 | 2.62E-04 |
| F2(87) | Cyno BCMA-His | 1.55E-09 | 6.21E+05 | 9.61E-04 |
| B3(87) | | 1.58E-09 | 7.73E+05 | 1.22E-03 |
| B3-7F5.3(87) | | 2.44E-10 | 8.21E+05 | 2.00E-04 |
| REGN-BCMA/CD3 | | 1.40E-08 | 7.67E+05 | 1.08E-02 |
| JNJ-BCMA/CD3 | | 1.75E-08 | 1.94E+06 | 3.38E-02 |
| Roche-BCMA/CD3 | | 8.90E-10 | 1.40E+06 | 1.25E-03 |

| | | | | |
|---|---|---|---|---|
| N.D.: not detected | | | | |

### Example 5. Killing Assay and Cytokine Release of Human Multiple Myeloma Cells by Trispecific Antibodies

When polyclonal antibodies bind to GPRC5D and/or BCMA on the surface of MM cells and also to CD3E on the surface of primary T cells, T cell activation can be stimulated by tumor-associated antigen (TAA, i.e., GPRC5D and/or BCMA)-dependent cross-linking to T cells, mediating the killing of tumor cells. Due to the strong heterogeneity of BCMA and GPRC5D-expressing tumor cells in actual multiple myeloma patients, the specificity is manifested by the presence of different subtypes of tumor cells expressing one or more antigens, or by individual differences in the levels of antigens expressed by tumor cells.

### KO cell line preparation and flow cytometry

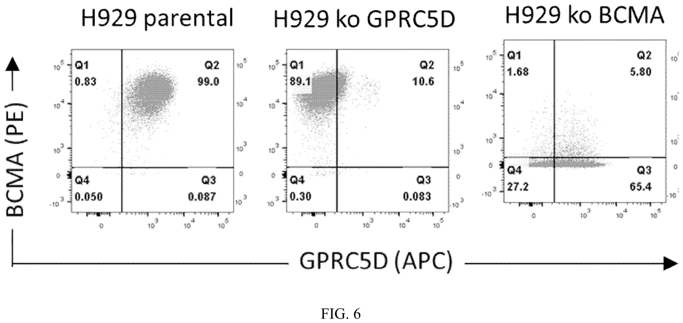

H929 ko BCMA and H929 ko GPRC5D were prepared by transfecting H929 cells by means of RNP. gRNAs with higher MIT specificity scores (Genescript) were selected by the gRNA design website (broadinstitute.org). Chemically synthesized gRNAs (three per target protein) and Cas9 protein (Genscript SC1841 NA) were mixed and transferred into cells by electrotransfection. After a stable culture was obtained, primer PCR was designed near the gRNA cleavage sites, and the amplified products were sequenced. Cleavage efficiency was analyzed by CRISPR after Sanger sequencing (Genescript), and the ratio of target gene cleavage was calculated. The knock-out cell pools were labeled for BCMA and GPRC5D using a flow cytometric sorter (BD) to sort out target protein-negative cell populations. Results: The final selected gRNA sequences and the results of flow cytometry of the obtained cell lines are shown in the table below and FIG. 6, respectively.

**Table 11. gRNA sequences selected from knock-out H929 cell line**

| Target gene | gRNA Sequence | Cleavage efficiency before sorting | Cell pool |
|---|---|---|---|
| GPRC5D | GACTCCAGAATGATGCCCCA SEQ ID NO:12 | 94% | H929 ko GPRC5D pool |
| BCMA | CAATAACGCTGACATGTTAG SEQ ID NO:13 | 94% | H929 ko GPRC5D pool |

### Killing assay and cytokine release

To further examine the killing effect of the exemplary antibodies in multiple myeloma, 1) cells with single expression of a single TAA, i.e., H929 ko BCMA and H929 ko GPRC5D (prepared as described above) and 2) H929 cells (MM cells, Nanjing Cobioer, CBP60243) with high expression of double TAAs or L363 cells (MM cells, Nanjing Cobioer, CBP60240) with low expression of double TAAs were co-incubated with PBMCs (Saily, SLB-HP050B) at an effector cell-to-tumor cell ratio of 10:1, the exemplary and control antibodies were added, and dead cells were detected 16 h later by lactate dehydrogenase (LDH) release assay. Non-target cell Calu-6 (NSCLC cells, the Cell Bank of the Chinese Academy of Sciences, TCHu144), which does not express the two antigens, were co-incubated with the corresponding antibodies and PBMCs to serve as a control for non-specific killing. 3) H929 KO GPRC5D and H929 KO BCMA expressing a single TAA and parent H929 cells expressing double antigens were mixed at a 1:1:1 ratio and then co-incubated with PBMCs. The exemplary and control antibodies were added, and the survival rate of target tumor cells was analyzed 16 h later by flow cytometry.

### Reagents and materials:

| Name | Cat. No. | Manufacturer |
|---|---|---|
| RPMI medium 1640 (1×), phenol red-free | L230KJ | Shanghai BasalMedia |
| FBS | SH30406.06 | Hyclone |
| CytoTox 96 LDH | G1781 | Promega |
| Human TNF alpha kit | 62HTNFAPEH | Cisbio |
| Human IL6 KIT | 62HIL06PEH | Cisbio |
| Human IFN gamma kit | 62HIFNGPEH | Cisbio |
| Human IL2 kit | 62HIL02PEH | Cisbio |
| DCM-APC/CY7 | L34975 | Invitrogen |
| CellTrace^{™} Violet | C34557 | Thermo Fisher |

The experimental procedures were as follows: PBMC cells (Saily, SLB-HP050B) were removed from liquid nitrogen, rapidly thawed in a water bath at 37 °C, and added to 9 mL of a serum-free medium. The mixture was centrifuged at 300 g for 5 min, and the supernatant was discarded. The cells were resuspended in 10% FBS phenol red-free 1640 medium, and the cell density was adjusted to 2 × 10⁶ cells/mL. Tumor cells growing at log phase were collected and washed twice with PBS to remove excess serum. At this point, if cell death was required to be detected by flow cytometry, CTV staining solution (1 µM, CellTrace^{™} Violet DMSO stock solution:PBS = 1:50,000; 2E6 cells/ mL CTV staining solution) was added after collection of MM cells, and the mixture was incubated at 37 °C in the dark for 15 min. This step was skipped and centrifugation was performed directly if tumor cell death was required to be detected by LDH. The centrifugation was performed at 300× g for 5 min, the supernatant was discarded, and the cell pellet was retained. The cells were resuspended in a phenol red-free medium, and the cell density was adjusted to 4 × 10⁵ cells/mL. As required for different cells, the antibody was serially diluted 10-fold using a complete medium to obtain 8 concentrations in total (with an initial concentration of 200 nM and a maximum final concentration of 50 nM for H929 line cells (including KO cells), and an initial concentration of 400 nM and a maximum final concentration of 100 nM for L363 and Calu-6), plus the last concentration of 0. 50 µL of tumor cells, 50 µL of serially diluted antibody, and 100 µL of PBMC cells were added into a 96-well cell culture plate (U-shaped bottom, COSTAR, Cat No. 7007) for co-incubation. If tumor cell death was required to be detected by LDH, target cell spontaneous LDH release wells (containing only 50 µL of tumor cells) and target cell maximum LDH release wells (containing 50 µL of tumor cells with the addition of a lysis buffer (included in the LDH kit, diluted in a 1:10 ratio)) were set up, and the volume was made up to 200 µL/well with a complete medium. The mixed cells were cultured in a CO₂ incubator at 37 °C for 16 h.

After co-incubation, the cell culture plate was centrifuged at 400 g for 5 min, 160 µL of supernatant was aspirated out of each well, and 50 µL of supernatant from each well was transferred into a new 96-well flat-bottom plate (Beyotime, Cat. No. FPT019) for LDH assay. The remaining cells were cryopreserved (-80 °C) for subsequent cytokine assay. The cell pellet was used in flow cytometry to detect cell death.

Detection of tumor cell death by LDH: 50 µL of cell supernatant was added to each well of a 96-well white-bottom plate, followed by the addition of 50 µL of an assay solution. After incubation in the dark at room temperature for 10-30 min (depending on the cell type and the reaction color), 50 µL of a stop solution was added. The absorbance values were read at 492/650 nm on a multifunctional microplate reader (SPARK) within 1 h after the addition of the stop solution. The wells containing the 0-concentration antibody plus tumor cells and immune cells were taken as the background, the wells containing only tumor cells were taken as the minimum value, and the wells containing the tumor cells treated with the lysis buffer were taken as the maximum value. The killing was calculated as: (sample value - background value) / (maximum value - minimum value).

Detection of tumor cell death by flow cytometry: The above cell culture plate was washed once with 200 µL of FACS buffer (1× PBS, 2% FBS, 2 mM EDTA) per well and centrifuged. The liquid was decanted and 45 µL of a DCM staining solution (prepared at 1:2000 with FACS buffer) was added to each well. The plate was incubated at 4 °C in the dark for 20 min, washed once with 200 µL of FACS buffer, and dried. The cells were resuspended in 150 µL of FACS buffer and then detected on a flow cytometer (BD). The killing was calculated as: the number of DCM⁺CTV⁺ cells / the total number of CTV⁺ cells.

Cytokine assay by Cisbio: The standard substance was dissolved in H₂O to obtain a standard stock solution; 3-fold dilution was performed with a diluent to give Std 7 (Standard 7); then 2-fold serial dilution was performed in a total of 6 wells, i.e., Std 1-6, with Std 0 being the diluent blank control. 16 µL of the supernatant to be tested was added into each well of a 96-well plate (#66PL96025), and the standard substance was also added at 16 µL/well. Eu Cryptate and d2 antibody were both diluted in a 1:20 ratio with a detection buffer. The two dilutions were mixed at a 1:1 ratio, and 4 µL of the mixture was added to each well. The plate was sealed with a sealing membrane and incubated at room temperature for 2 h. The emission signal was detected by a multifunctional microplate reader (SPARK), and the value read at 665 nm/620 nm was the relative value of cytokine content.

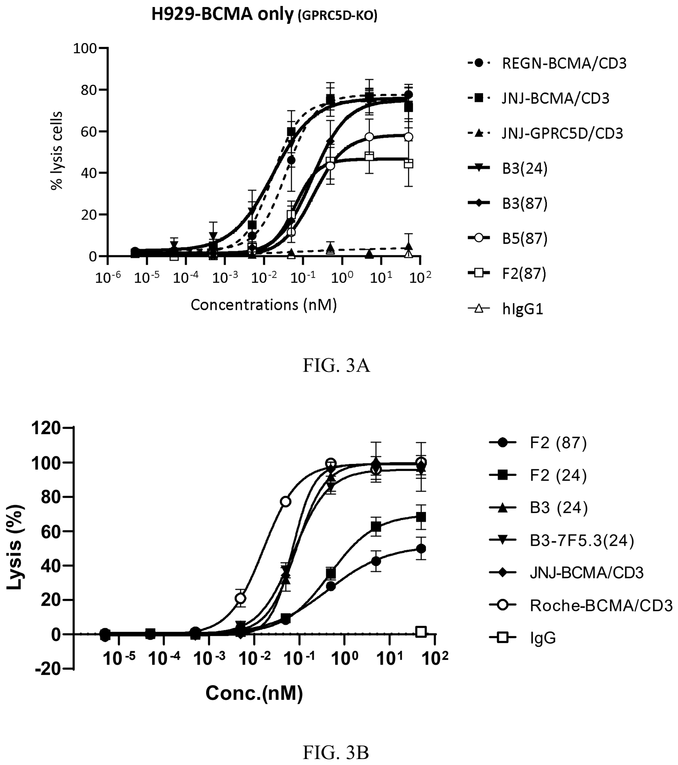

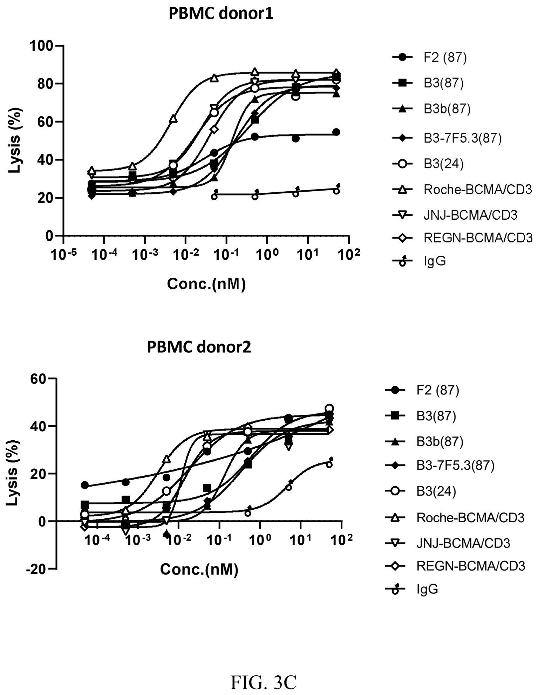

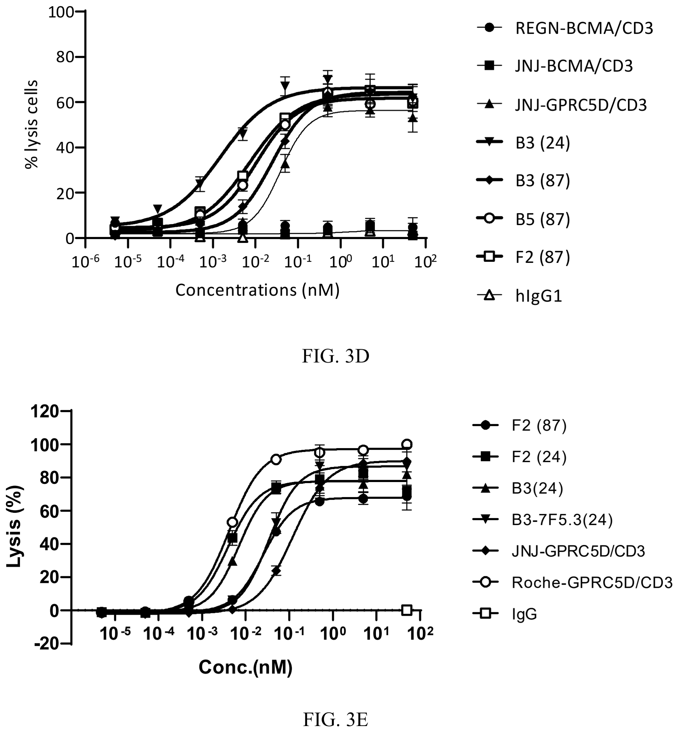

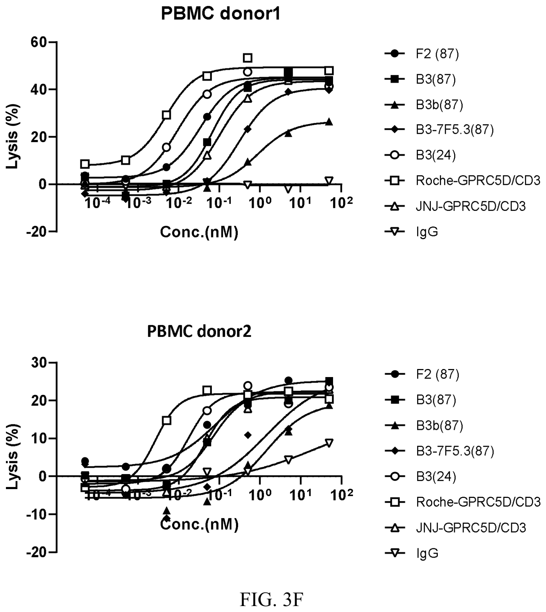

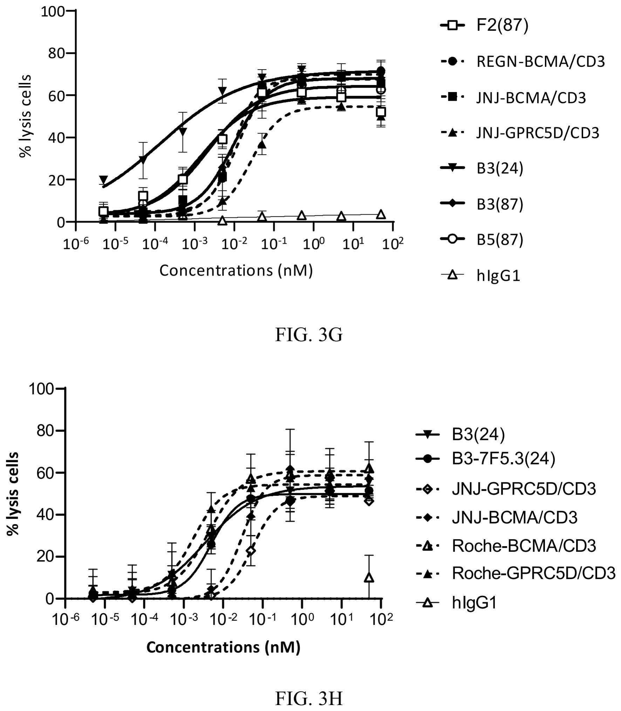

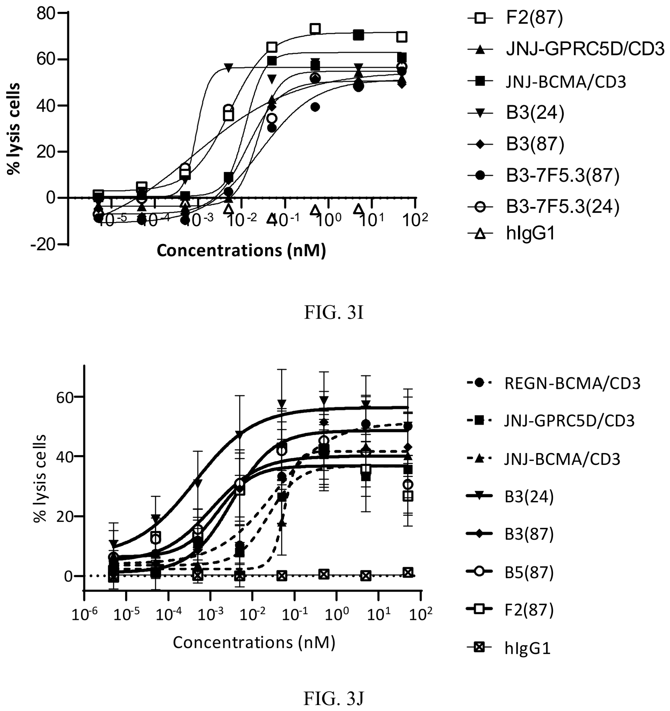

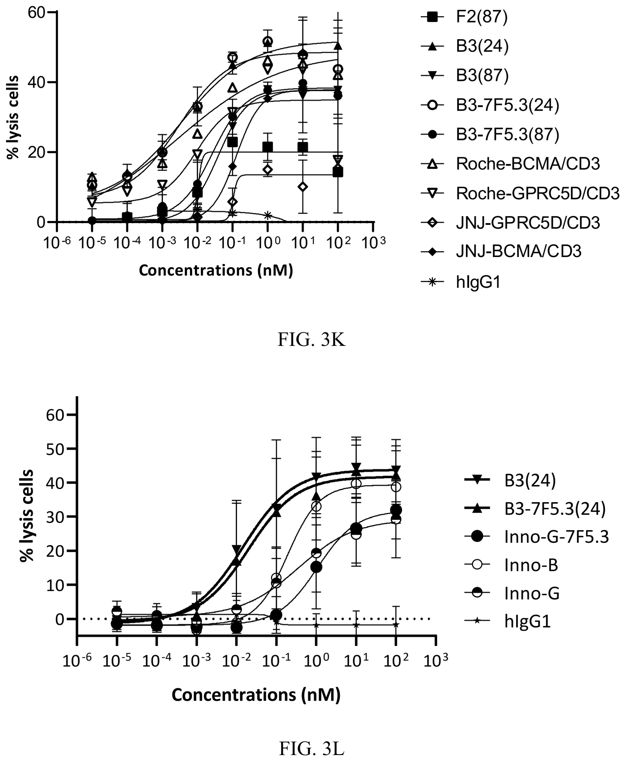

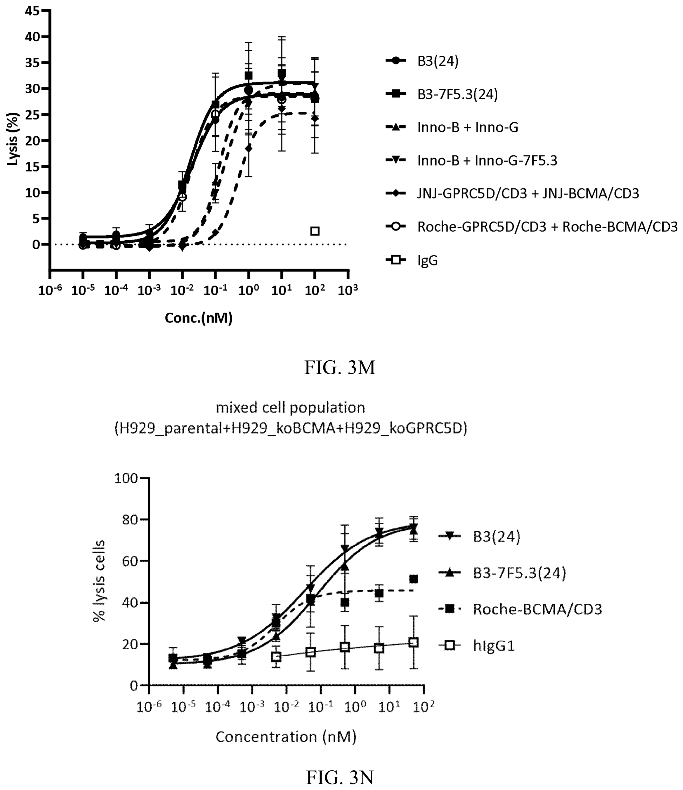

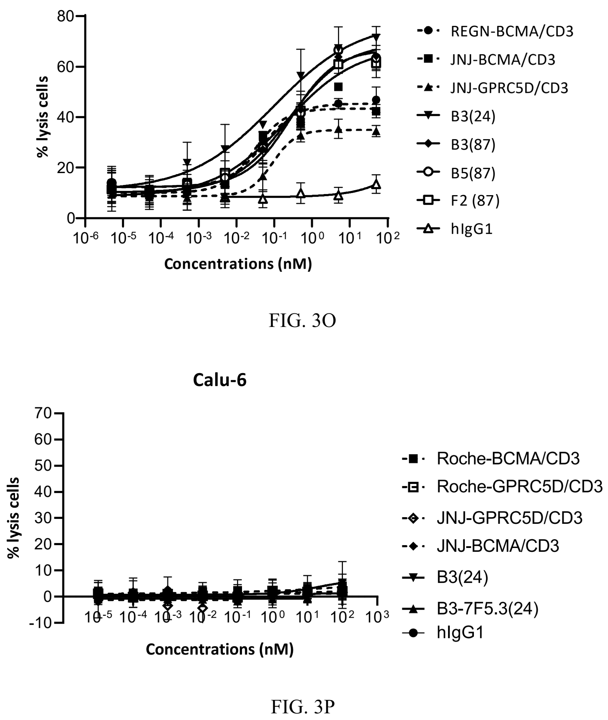

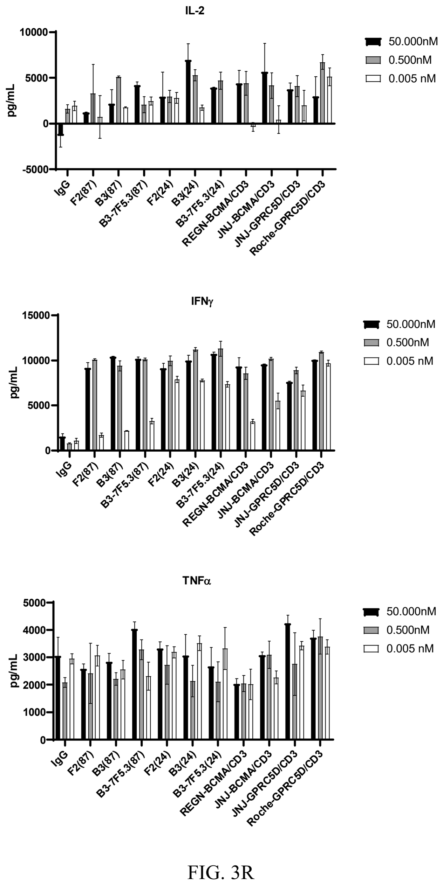

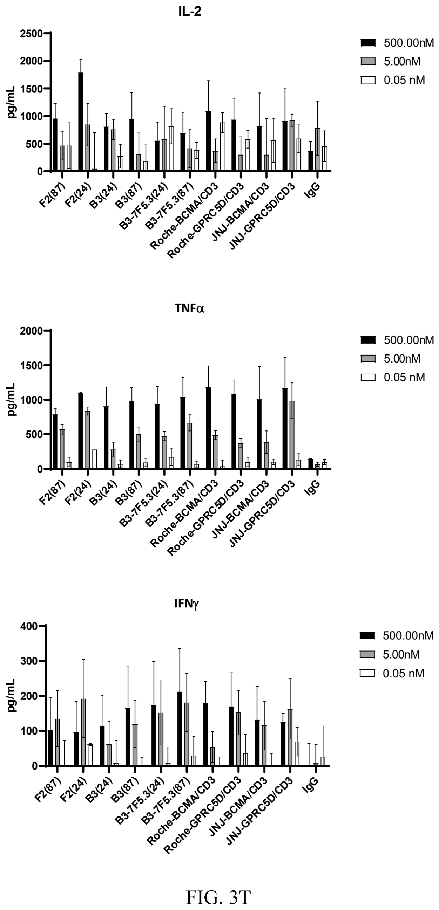

Experimental results: The exemplary antibodies could dose-dependently activate and mediate the killing effect of PBMCs on MM cells (FIGs. 3A-3O, Tables 12-24).
1. The results of killing by the corresponding antibodies in H929 ko GPRC5D and H929 ko BCMA expressing a single TAA are shown in FIGs. 3A-3F and Tables 12-16. The specific experimental results are as follows:
   In cells with the presence of TAA expression, the exemplary antibodies, like the corresponding antibody control molecules, could dose-dependently activate the killing by T cells in PBMCs. In contrast, no killing effect was observed in non-target cells that did not express TAA (FIG. 3P), indicating that the killing was specific killing dependent on the binding to the corresponding TAA. The higher the affinity of the anti-CD3 end of the exemplary antibodies, the better the ability to kill tumor cells, that is, molecules in which the antigen-binding region specifically binding to CD3 contained a heavy chain variable region (SEQ ID NO: 77) derived from sp34.24 (i.e., marked with (24) in the figures) were stronger than molecules in which the antigen-binding region specifically binding to CD3 contained a heavy chain variable region (SEQ ID NO: 78) derived from sp34.87 (i.e., marked with (87) in the figures).

From the results of mediating the killing effect of PBMCs on H929 ko GPRC5D, it can be seen that the ability of the exemplary antibodies in B3, B3b, and B5 formats to mediate the killing of tumor cells was superior to that of the molecules in the F2 format, indicating that the design of B3, B3b, and B5 to avoid scFv improved the functionality of BCMA.

From the results of mediating the killing effect of PBMCs on H929 ko BCMA, it can be seen that the ability of the exemplary antibodies in the B3 and B5 formats to mediate the killing of tumor cells was similar to that of the molecules in the F2 format and superior to that of the molecules in the B3b format.

The above results indicate that the anti-GPRC5D and anti-BCMA ends of the exemplary antibodies in the B3, B5, and B3b formats could successfully act as TAA antibodies of TCE and exert the function of mediating T cell killing against tumor cells.

2. In H929 and L363 cells expressing both antigens (FIGs. 3G-3M, Tables 18-24): both BCMA and GPRC5D levels were low on the surface of L363 cells; both BCMA and GPRC5D levels were higher on the surface of H929 cells (compared to the antibody disclosed in WO2022174813). The specific results are as follows:
In H929 cells, as observed above, molecules in which the antigen-binding region specifically binding to CD3 contained a heavy chain variable region (SEQ ID NO: 77) derived from sp34.24 were stronger than molecules in which the corresponding antigen-binding region specifically binding to CD3 contained a heavy chain variable region (SEQ ID NO: 78) derived from sp34.87. Moreover, the killing effect of the trispecific exemplary antibodies B3 (24) and B3-7F5.3 (24) was superior to that of the control bispecific antibody molecules from JNJ, i.e., JNJ-BCMA/CD3 and JNJ-GPRC5D/CD3, and slightly weaker or comparable to that of the control bispecific antibody molecules from Roche, i.e., Roche-GPRC5D and Roche-BCMA/CD3.

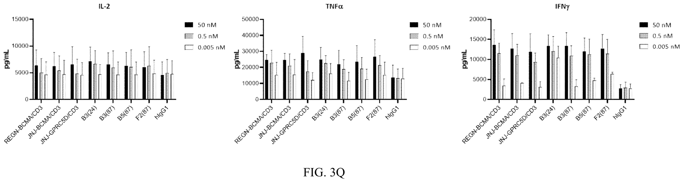

Meanwhile, in the comparison of cytokine release induced during H929 cell killing, the trispecific exemplary antibodies did not remarkably differ from the control bispecific antibodies, i.e., JNJ-BCMA/CD3, JNJ-GPRC5D/CD3, and REGN-BCMA/CD3, in inducing cytokine release (FIGs. 3Q-3R).

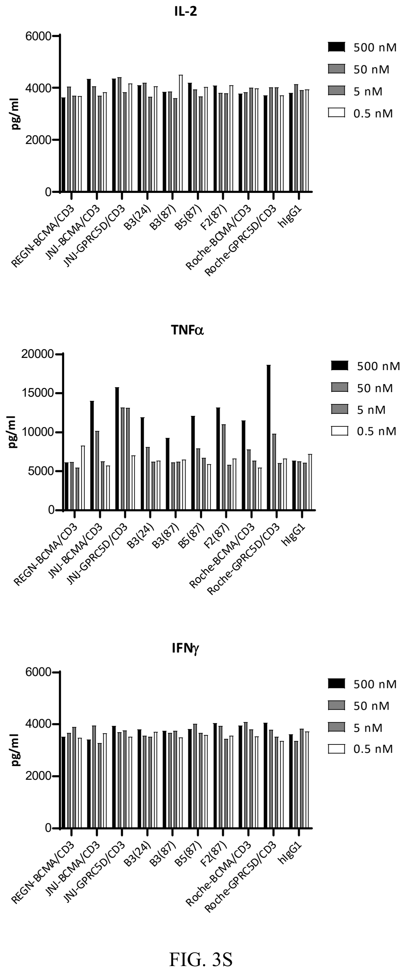

However, in the absence of target cells, the trispecific exemplary antibodies, like the control bispecific antibodies, i.e., JNJ-BCMA/CD3, JNJ-GPRC5D/CD3, Roche-GPRC5D/CD3, Roche-BCMA/CD3, andREGN-BCMA/CD3, did not induce non-specific cytokine release, i.e., the levels were comparable to those of the hIgG control (FIGs. 3S-3T).

In L363 cells, the killing effect of the exemplary trispecific antibodies B3 (24) and B3-7F5.3 (24) was stronger than that of the control bispecific antibody molecules from JNJ (JNJ-BCMA/CD3 and JNJ-GPRC5D/CD3) and comparable to that of the control bispecific antibody molecules from Roche (Roche-GPRC5D/CD3 and Roche-BCMA/CD3). The anti-GPRC5D and anti-BCMA ends of the exemplary trispecific antibodies of the present invention were functional when utilized for killing separately from the control molecule (Inno-B, Inno-G, or Inno-G-7F5.3). The control BCMA/CD3 and GPRC5D/CD3 bispecific antibody molecules from JNJ and Roche were mixed at a 1:1 ratio and compared to the exemplary trispecific antibodies of the present invention at the same concentration. In the comparison, the killing of L363 cells mediated by the exemplary trispecific antibodies of the present invention was superior to that of the combination of the control BCMA/CD3 and GPRC5D/CD3 bispecific antibody molecules. The above results indicate that the anti-GPRC5D and anti-BCMA ends of the trispecific antibodies had a mutual synergistic effect on tumor cell killing, which was superior to the combination of two bispecific antibodies.

3. After H929 ko GPRC5D and H929 ko BCMA expressing a single TAA and parent H929 cells expressing double antigens were mixed at a 1:1:1 ratio, the exemplary trispecific antibodies of the present invention had a higher killing curve, i.e., a higher maximal killing ratio, than the control molecules (FIGs. 3N and 3O).

**Table 12. Killing effect-1 of PBMCs mediated by exemplary antibodies on H929 ko GPRC5D cells ("N.A." indicates not analyzed)**

| Exemplary antibodies | EC50 (pM) |
|---|---|
| F2(87) | 67.67 |
| B3(87) | 175.53 |
| B3(24) | 16.97 |
| B5(87) | 182.03 |
| REGN-BCMA/CD3 | 37.47 |
| JNJ-BCMA/CD3 | 16.52 |
| JNJ-GPRC5D/CD3 | N.A. |

**Table 13. Killing effect-2 of PBMCs mediated by exemplary antibodies on H929 ko GPRC5D cells**

| Exemplary antibodies | EC₅₀ (pM) |
|---|---|
| F2(87) | 427.68 |
| F2(24) | 474.81 |
| B3(24) | 86.68 |
| B3-7F5.3(24) | 75.12 |
| JNJ-BCMA/CD3 | 70.38 |
| Roche-BCMA/CD3 | 16.08 |

**Table 14. Killing effect-3 of PBMCs mediated by exemplary antibodies on H929 ko GPRC5D cells**

| Exemplary antibodies | EC₅₀ (pM); donor1 | EC₅₀ (pM); donor2 |
|---|---|---|
| F2 (87) | 30.35 | N.A. |
| B3(87) | 345.95 | 601.53 |
| B3b(87) | 139.52 | 131.73 |
| B3-7F5.3(87) | 166.83 | 346.35 |
| B3(24) | 17.48 | 18.33 |
| Roche-BCMA/CD3 | 4.50 | 3.11 |
| JNJ-BCMA/CD3 | 24.13 | 11.40 |
| REGN-BCMA/CD3 | 39.48 | 13.79 |

**Table 15. Killing effect-1 of PBMCs mediated by exemplary antibodies on H929 ko BCMA cells ("N.A." indicates not analyzed)**

| Exemplary antibodies | EC₅₀ (pM) |
|---|---|
| F2(87) | 7.39 |
| B3(87) | 25.00 |
| B3(24) | 1.49 |
| B5(87) | 10.46 |
| REGN-BCMA/CD3 | N.A. |
| JNJ-BCMA/CD3 | N.A. |
| JNJ-GPRC5D/CD3 | 38.50 |

**Table 16. Killing effect-2 of PBMCs mediated by exemplary antibodies on H929 ko BCMA cells ("N.A." indicates not analyzed)**

| Exemplary antibodies | EC₅₀ (pM) |
|---|---|
| F2(87) | 25.56 |
| F2(24) | 4.00 |
| B3(24) | 6.98 |
| B3-7F5.3(24) | 34.56 |
| JNJ-GPRC5D/CD3 | 120.44 |
| Roche-GPRC5D/CD3 | 4.19 |

**Table 17. Killing effect-3 of PBMCs mediated by exemplary antibodies on H929 ko BCMA cells ("N.A." indicates not analyzed)**

| Exemplary antibodies | EC₅₀ (pM); donor1 | EC₅₀ (pM); donor2 |
|---|---|---|
| F2 (87) | 34.24 | 83.22 |
| B3(87) | 66.25 | 72.33 |
| B3b(87) | 878.88 | 1453.36 |
| B3-7F5.3(87) | 308.30 | 1436.04 |
| B3(24) | 9.31 | 17.56 |
| Roche-GPRC5D/CD3 | 4.86 | 2.67 |
| JNJ-GPRC5D/CD3 | 99.83 | 44.74 |

**Table 18. Killing effect-1 of PBMCs mediated by exemplary antibodies on H929 cells**

| Exemplary antibodies | EC₅₀ (pM) |
|---|---|
| F2(87) | 1.41 |
| B3(87) | 9.25 |
| B3(24) | 0.14 |
| B5(87) | 2.18 |
| REGN-BCMA/CD3 | 12.07 |
| JNJ-BCMA/CD3 | 9.77 |
| JNJ-GPRC5D/CD3 | 26.16 |

**Table 19. Killing effect-2 of PBMCs mediated by exemplary antibodies on H929 cells**

| Exemplary antibodies | EC₅₀ (pM) |
|---|---|
| Roche-BCMA/CD3 | 4.28 |
| Roche-GPRC5D/CD3 | 1.76 |
| JNJ-BCMA/CD3 | 30.01 |
| JNJ-GPRC5D/CD3 | 54.94 |
| B3(24) | 3.41 |
| B3-7F5.3(24) | 4.95 |

**Table 20. Killing effect-3 of PBMCs mediated by exemplary antibodies on H929 cells**

| Exemplary antibodies | EC₅₀ (pM) |
|---|---|
| B3-7F5.3(24) | 0.77 |
| JNJ-BCMA/CD3 | 12.57 |
| JNJ-GPRC5D/CD3 | 23.71 |
| B3(24) | 0.92 |
| B3(87) | 13.51 |
| B3-7F5.3(87) | 26.83 |
| F2(87) | 5.30 |

**Table 21. Killing effect-1 of PBMCs mediated by exemplary antibodies on L363 cells**

| Exemplary antibodies | EC₅₀ (pM) |
|---|---|
| REGN-BCMA/CD3 | 32.89 |
| JNJ-GPRC5D/CD3 | 23.96 |
| JNJ-BCMA/CD3 | 57.30 |
| B3(24) | 0.43 |
| B3(87) | 3.29 |
| B5(87) | 1.08 |
| F2(87) | 1.52 |

**Table 22. Killing effect-2 of PBMCs mediated by exemplary antibodies on L363 cells**

| Exemplary antibodies | EC₅₀ (pM) |
|---|---|
| Roche-BCMA/CD3 | 2.58 |
| Roche-GPRC5D/CD3 | 9.35 |
| JNJ-BCMA/CD3 | 127.80 |
| JNJ-GPRC5D/CD3 | NA |
| F2(87) | 10.54 |
| B3(24) | 3.43 |
| B3(87) | 38.89 |
| B3-7F5.3(24) | 3.01 |
| B3-7F5.3(87) | 26.48 |

**Table 23. Killing effect-3 of PBMCs mediated by exemplary antibodies on L363 cells.**

| Exemplary antibodies | EC₅₀ (pM) |
|---|---|
| B3(24) | 14.86 |
| B3-7F5.3(24) | 20.36 |
| Inno-B | 192.27 |
| Inno-G | 355.50 |
| Inno-G-7F5.3 | 1069.29 |

**Table 24. Killing effect-4 of PBMCs mediated by exemplary antibodies on L363 cells**

| Exemplary antibodies | EC₅₀ (pM) |
|---|---|
| B3(24) | 19.79 |
| B3-7F5.3(24) | 16.88 |
| Inno-B+Inno-G | 127.42 |
| Inno-B+ Inno-G-7F5.3 | 187.69 |
| JNJ-GPRC5D/CD3 + JNJ-BCMA/CD3 | 489.68 |
| Roche-GPRC5D/CD3 + Roche-BCMA/CD3 | 18.16 |

### Killing Assay for Cells Expressing Mutant Antigens

The experimental procedures were as follows: PBMC cells (Saily, SLB-HP100B) were removed from liquid nitrogen, and rapidly thawed in a water bath at 37°C, and added to 9 ml of serum-free medium. The mixture was centrifuged at 300 g for 5 min, and the supernatant was discarded. The cells were resuspended in 10% FBS phenol red-free 1640 medium, and the cell density was adjusted to 2 × 10⁶ cells/ml. Tumor cells growing at log phase were collected and washed twice with PBS to remove excess serum. The GS CHO cell lines overexpressing the BCMA mutant constructed in Example 1 was centrifuged at 300 × g for 5 min, respectively. The supernatant was discarded, and the cell pellets were retained. The cells were resuspended in a phenol red-free medium, and the cell density was adjusted to 4 × 10⁵ cells/mL. The antibody was serially diluted 10-fold using a complete medium to obtain 8 concentrations in total (with an initial concentration of 200 nM and a maximum final concentration of 50 nM), plus the last concentration of 0. 50 µL of tumor cells, 50 µL of serially diluted antibody, and 100 µL of PBMC cells were added into a 96-well cell culture plate (U-bottom, COSTAR, Cat. 7007) for co-incubation. Target cell spontaneous LDH release wells (containing only 50 µL of tumor cells) and target cell maximum LDH release wells (containing 50 µL of tumor cells with the addition of a lysis buffer (included in the LDH kit, diluted in a 1:10 ratio)) were set up, and the volume was made up to 200 µL/well with a complete medium. The mixed cells were cultured in a CO₂ incubator at 37°C for 16 h.

After co-incubation, the cell culture plate was centrifuged at 400g for 5 min,160µL of supernatant was aspirated out of each well, and 50 µL of supernatant from each well was transferred to a new 96-well flat-bottom plate (Beyotime, Cat. FPT019) for LDH assay.

Detection of tumor cell death by LDH: 50 µL of cell supernatant was added to each well of a 96-well white-bottom plate, followed by the addition of 50 µL of an assay solution. After incubation in the dark at room temperature for 10-30 min (depending on the cell type and the reaction color), 50 µL of a stop solution was added. The absorbance values were read at 492/650 nm on a multifunctional microplate reader (SPARK) within 1 h after the addition of the stop solution. The wells containing the 0-concentration antibody plus tumor cells and immune cells were taken as the background, the wells containing only tumor cells were taken as the minimum value, and the wells containing the tumor cells treated with the lysis buffer were taken as the maximum value. The killing was calculated as: (sample value - background value) / (maximum value - minimum value).

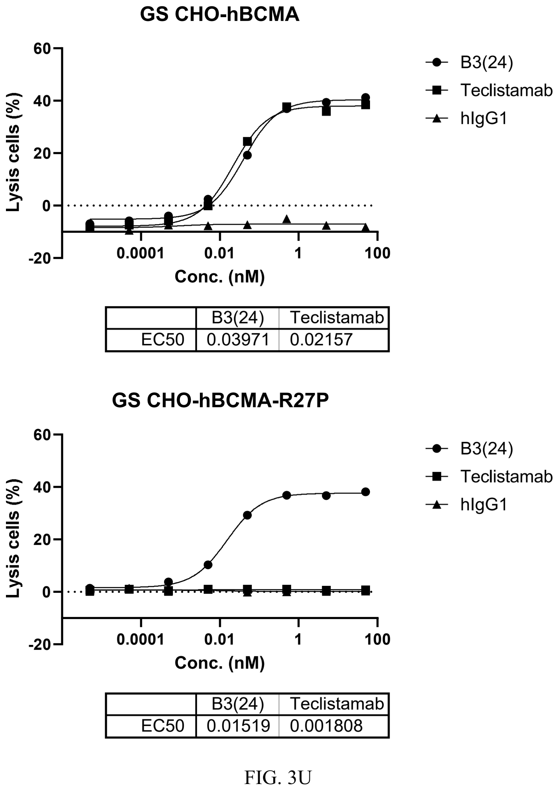

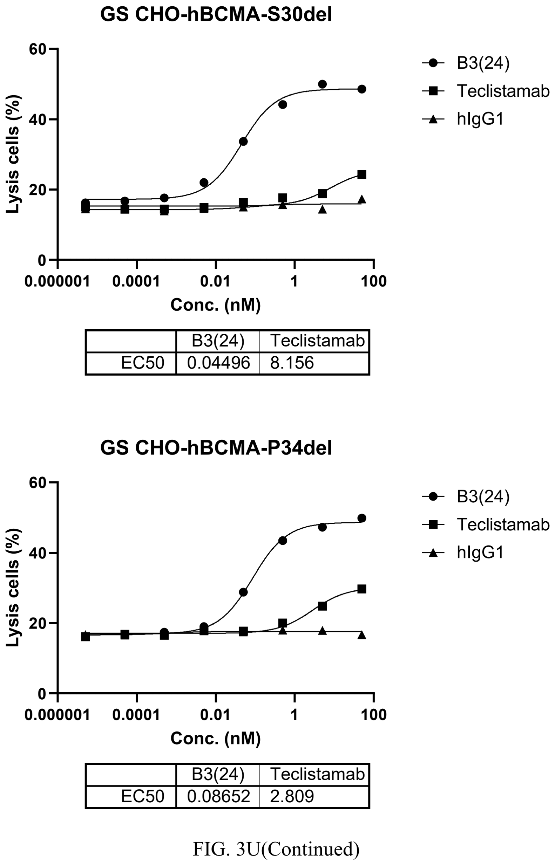

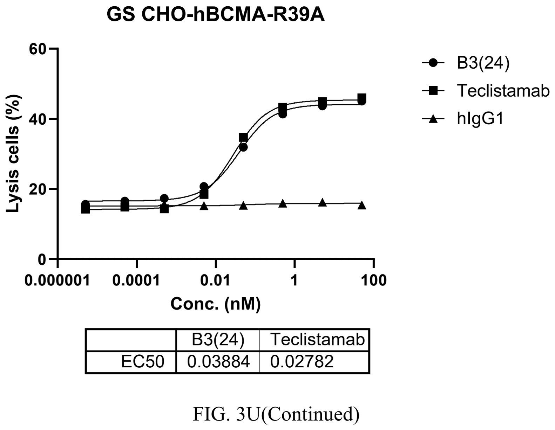

Experimental results: B3(24) could dose-dependently activate and mediate the killing effect of PBMCs on GS CHO cells overexpressing BCMA mutants (GS CHO-hBCMA-P34del, GS CHO -hBCMA-S30del, GS CHO -hBCMA-R39A, GS CHO -hBCMA-R27P); Teclistamab has no in vitro killing function on BCMA mutants R27P and R39A (FIG. 3U).

### Example 6. Overcoming Effects of sBCMA by Trispecific Antibodies

The soluble form of BCMA (sBCMA), which results from shedding from the surface of plasma cells due to the lysis by γ-secretase, is elevated in MM patients and associated with poor clinical outcomes (https://www.ncbi.nlm.nih.gov/pmc/articles/PMC5395119/). sBCMA, which consists of an extracellular domain and a portion of the transmembrane domain of BCMA, not only reduces the density of the target antigen, but also provides a soluble decoy capable of restricting the potency of the BCMA/CD3 molecule. The GPRCSD/BCMA/CD3 molecule is theoretically less affected by sBCMA because it contains a GPRC5D end that is not restricted by sBMCA.

The experimental procedures were as follows: Human cryopreserved PBMC cells (SAILY) were removed from liquid nitrogen, rapidly thawed in a water bath at 37 °C, and added to 9 mL of a serum-free medium. The mixture was centrifuged at 300 g for 4 min, and the supernatant was discarded. The cells were resuspended in 1% FBS phenol red-free 1640 medium (complete medium), and the cell density was adjusted to 4 × 10⁶ cells/mL. The cell culture medium was collected into a 50 mL sterile centrifuge tube and centrifuged at 300× g for 5 min. The supernatant was discarded and the cell pellet was retained. The cell density was adjusted to 4 × 10⁵ cells/mL with a complete medium. The antibody was serially diluted 10-fold to 200 nM in a complete medium. Meanwhile, human recombinant BCMA (hBCMA; stock solution of 0.5 mg/mL, prepared with PBS) was diluted to 800 ng/mL in a complete medium to give an sBCMA solution. 50 µL of tumor cells (H929), 50 µL of serially diluted antibody, 50 µL of PBMC cells, and 50 µL of the sBCMA solution were added into a 96-well cell culture plate (U-type, Costar), at which point the co-incubation system contained 200 ng/mL sBCMA. The mixture was incubated in a CO₂ incubator at 37 °C for 16 h. After co-incubation, the cell culture plate was centrifuged at 400 g for 5 min and 50 µL of supernatant from each well was transferred into a new 96-well flat-bottom plate (Beyotime, Cat. No. FPT019) for LDH assay. The cell pellet was assayed by flow cytometry (BD) for the percentage of CD69-positive cells in T cells. See Example 5 for the information on the cells used.

Detection of tumor cell death by LDH: 50 µL of cell supernatant was added to each well of a 96-well white-bottom plate, followed by the addition of 50 µL of an assay solution. After incubation in the dark at room temperature for 10-30 min (depending on the cell type and the reaction color), 50 µL of a stop solution was added. The absorbance values were read at 492/650 nm on a multifunctional microplate reader (SPARK) within 1 h after the addition of the stop solution. The wells containing the 0-concentration antibody plus tumor cells and immune cells were taken as the background, the wells containing only tumor cells were taken as the minimum value, and the wells containing the tumor cells treated with the lysis buffer were taken as the maximum value. The killing was calculated as: (sample value - background value) / (maximum value - minimum value).

Detection of CD8 and CD4 T cell activation by flow cytometry: The above cell culture plate was washed once with 200 µL of FACS buffer (1× PBS, 2% FBS, 2 mM EDTA) per well and centrifuged. The liquid was decanted and 45 µL of a staining solution (prepared with FACS buffer) was added to each well. The plate was incubated at 4 °C in the dark for 20 min, washed once with 200 µL of FACS buffer, and dried. The cells were resuspended in 150 µL of FACS buffer and then detected on a flow cytometer. The proportion of CD69-positive cells in CD8 T cells and CD4 T cells was calculated.

### Reagents and materials:

| Name | Cat. No. | Manufacturer |
|---|---|---|
| RPMI medium 1640 (1×), phenol red-free | L230KJ | Shanghai BasalMedia |
| FBS | SH30406.06 | Hyclone |
| CytoTox 96 LDH | G1781 | Promega |
| PE-Anti-human CD2 | Biolegend | 309208 |
| PE/Cy7-anti-human CD4 | BD | 560644 |
| BUV805-anti-human CD8 | BD | 612889 |
| FITC-anti-human CD69 | Biolegend | 310904 |
| DCM-APC/CY7 | L34975 | Invitrogen |
| CellTrace^{™} Violet | C34557 | Thermo Fisher |

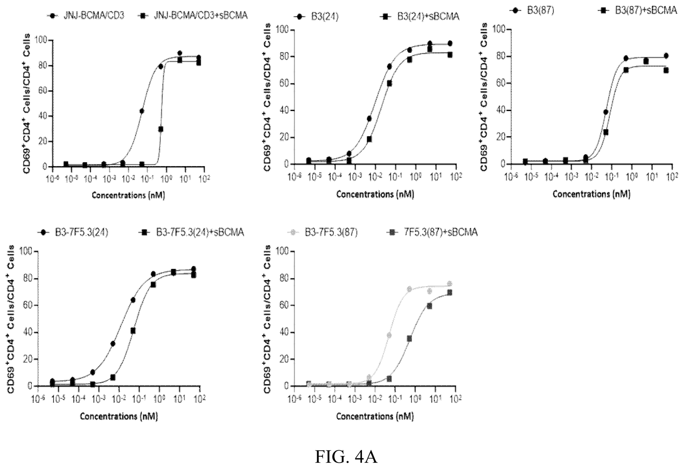

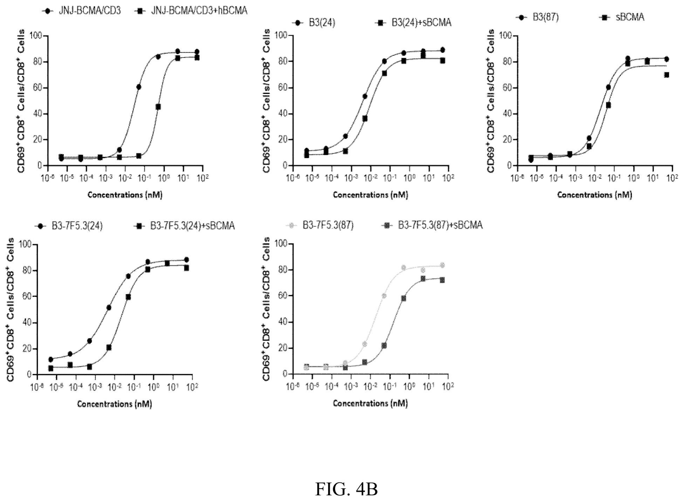

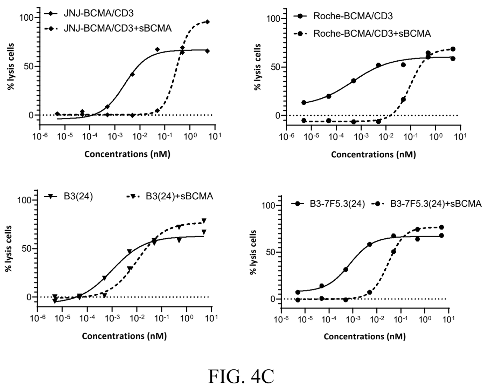

The experimental results are shown in FIGs. 4A, 4B and 4C, and Tables 25 and 26. The results indicate that activation of T cells and killing of H929 cells mediated by the trispecific antibodies were less affected by sBCMA.

**Table 25. Activation of T cells in PBMCs mediated by exemplary antibodies (w/o: without, w: with)**

| Activation EC₅₀ (nM) | JNJ-BCMA/CD3 | | B3(24) | | B3(87) | | B3-7F5.3(24) | | B3-7F5.3(87) | |
|---|---|---|---|---|---|---|---|---|---|---|
| | w/o sBCMA | w sBCMA | w/o sBCMA | w sBCMA | w/o sBCMA | w sBCMA | w/o sBCMA | w sBCMA | w/o sBCMA | w sBCMA |
| CD4 | 0.05207 | 0.5461 | 0.009738 | 0.01971 | 0.05289 | 0.08106 | 0.01256 | 0.05445 | 0.04927 | 0.5367 |
| CD8 | 0.02906 | 0.4948 | 0.004015 | 0.008615 | 0.02026 | 0.03852 | 0.004301 | 0.02193 | 0.01859 | 0.1515 |

**Table 26. Activation of T cells in PBMCs mediated by exemplary antibodies (w/o: without, w: with)**

| | B3-7F5.3 (24) | | B3(24) | | JNJ-BCMA/CD3 | | Roche-BCMA/CD3 | |
|---|---|---|---|---|---|---|---|---|
| | w/o sBCMA | w sBCMA | w/o sBCMA | w sBCMA | w/o sBCMA | w sBCMA | w/o sBCMA | w sBCMA |
| EC₅₀ (pM) | 0.7377 | 29.83 | 1.020 | 10.41 | 2.43 | 294.5 | 0.4525 | 96.55 |

### Example 7. Anti-Tumor Efficacy of Trispecific Antibodies in Tumor-Bearing Humanized Mouse Models

Female NOG mice (aged 35-48 days) were purchased from Beijing Vitalstar Biotechnology Co., Ltd. and were SPF mice. The study started after the mice were acclimated and quarantined for 5-7 days after arrival.

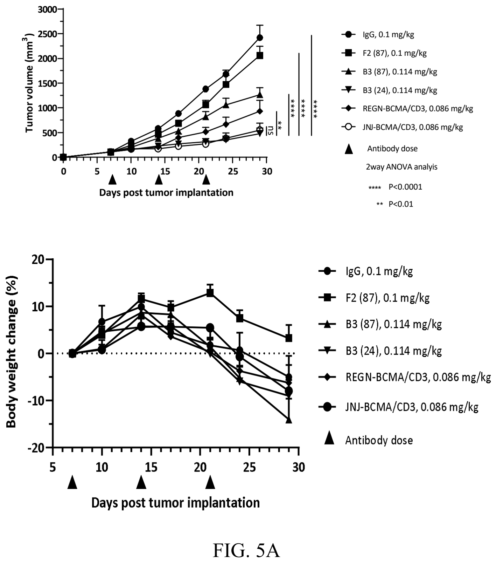

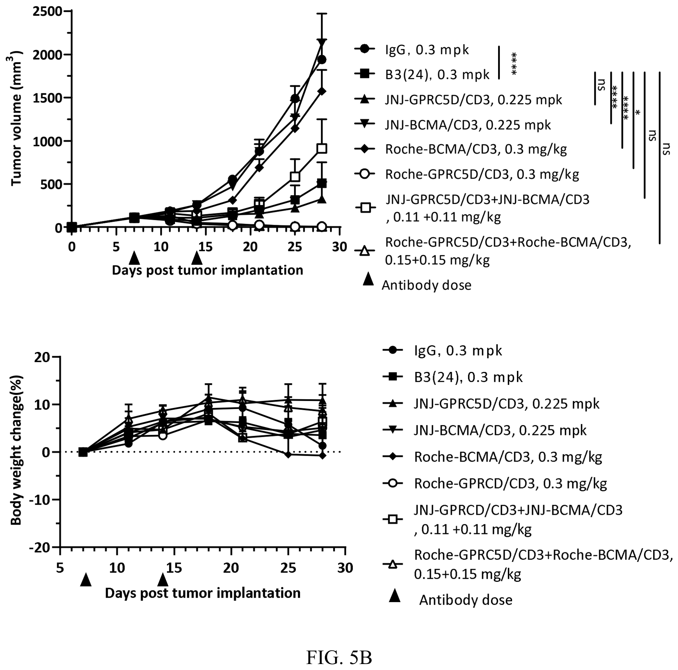

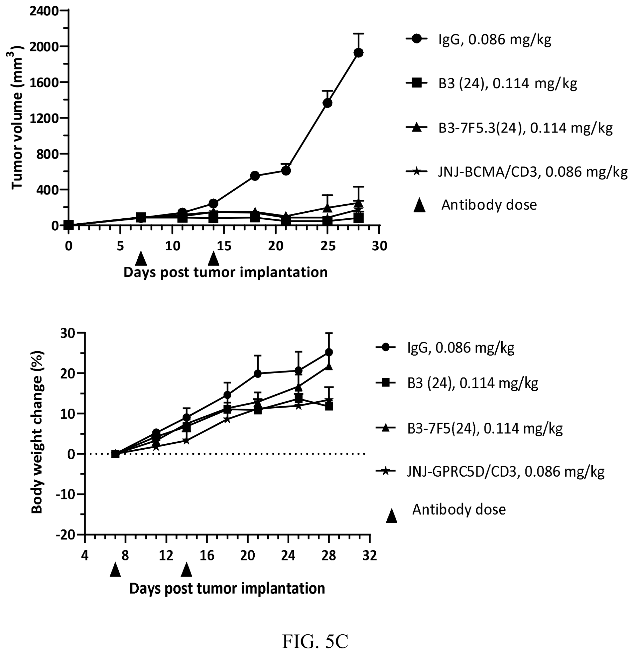

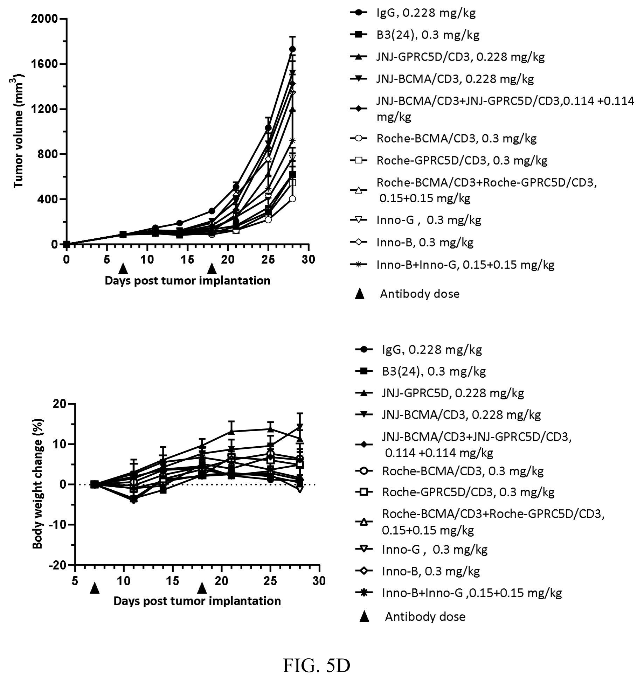

Tumor cells were subcultured conventionally for subsequent *in vivo* study. The cells were collected by centrifugation and dispersed in PBS. NOG mice were intravenously injected with PBMC cells at 4 × 10⁶ cells/mouse in a volume of 200 µL/mouse. On day 3 after PBMC cell inoculation, the mice were subjected to shaving on the right side of the back and abdomen, and subcutaneously injected with tumor cells in a volume of 200 µL/mouse (inoculation density: H929 ko BCMA: 4 × 10⁶ cells/mouse; H929 ko GPRC5D: 4 × 10⁶ cells/mouse, MM.1S (MM cells, Nanjing Cobioer, CBP60239): 3 × 10⁶/mouse; L363: 1 × 10⁶ cells/mouse). On day 7 after tumor cell inoculation, the mice were divided into groups (6-7 mice per group) based on tumor volume and intraperitoneal administration was performed (see FIG. 5 and the brief description of the drawing for details of the frequency and dose of administration). The mice were monitored for tumor volume and body weight twice a week. Tumor volume measurement: The maximum length of major axis (L) and maximum length of minor axis (W) of tumors were measured with a vernier caliper, and tumor volume was calculated using the following formula: V = L × W2/2. The mice were weighed using an electronic balance. Throughout the study, the mice were euthanized when the tumors reached an endpoint or when the mice lost more than 20% of body weight. The tumor size was counted.

### Experimental results:

H929 ko GPRC5D tumor (FIG. 5A): B3 (24) molecule could effectively inhibit the growth of H929 ko GPRC5D cells, and the anti-tumor ability of the B3 (24) molecule was substantially consistent with that of the JNJ-BCMA/CD3 control molecule and stronger than that of the B3 (87) molecule, the REGN-BCMA/CD3 molecule, and the F2 (87) molecule.

H929 ko BCMA tumor (FIG. 5B): B3 (24) molecule could effectively inhibit the growth of H929 ko BCMA cells, and the anti-tumor ability of the B3 (24) molecule was substantially consistent with that of the JNJ-GPRC5D/CD3 control molecule, stronger than that of all BCMA/CD3 and the combination of (JNJ-GPRC5D/CD3 + JNJ-BCMA/CD3), and slightly weaker than that of the Roche-GPRC5D/CD3 and the combination of (Roche-BCMA/CD3 + Roche-GPRC5D/CD3).

MM1S tumor (FIG. 5C): The exemplary antibodies and the control molecules all showed good anti-tumor effect.

L363 tumor (FIG. 5D): B3 (24) molecule could effectively inhibit the growth of L363 cells, and the anti-tumor ability of the B3 (24) molecule was stronger than that of the JNJ-GPRC5D/CD3, the JNJ-BCMA/CD3, and the combination of (JNJ-BCMA/CD3 + JNJ-GPRCSD/CD3), and was closer to that of the Roche-GPRC5D/CD3, the Roche-BCMA/CD3, and the combination of (Roche-GPRC5D/CD3 + Roche-GPRC5D/CD3).

The B3 (24) molecule showed no difference in the effect on the body weight of mice in all models compared to other control molecules.

### Sequence information

### 1. Antibody sequence information

| Abbreviation | Specific protein name | Structure | SEQ ID NO | Chain | Mutation information | Variable region sequence |
|---|---|---|---|---|---|---|
| B3 (24) | hz5E12_sp34.24_ 497_triFab | FIG. 1A | 1 | sp34_K_CL(lambda) | Q38K | SEQ ID NO:74 |
| | | | 2 | 497_DCS_CL | Q38D Q124C C214S | SEQ ID NO:75 |
| | | | 3 | 497(SGL)_KCS_sp34.24_D_hole_AA | Q39K F126C C220S (first Fab), Q39D (second Fab), L234A L235A, T366S L368A Y407V | SEQ ID NO: 76 (first variable region) SEQ ID NO: 77 (second variable region) |
| | | | 4 | hz5E12.1-L | N/A | SEQ ID NO:52 |
| | | | 5 | hz5E12.1-H | L234A L235A, T366W | SEQ ID NO:48 |
| B3 (87) | hz5E12_sp34.87_ 497_triFab | FIG. 1A | 1 | sp34_K_CL(lambda) | Q38K | SEQ ID NO:74 |
| | | | 2 | 497_DCS_CL | Q38D Q124C C214S | SEQ ID NO:75 |
| | | | 6 | 497(SGL)_KCS_sp34.87_D_hole_AA | Q39K F126C C220S (first Fab), Q39D (second Fab), L234A L235A, T366S L368A Y407V | SEQ ID NO: 76 (first variable region) |
| | | | | | | SEQ ID NO: 78 (second variable region) |
| | | | 4 | hz5E12.1-L | N/A | SEQ ID NO:52 |
| | | | 5 | hz5E12.1-H | L234A L235A, T366W | SEQ ID NO:48 |
| B5 (87) | hz5E12_sp34.87_ 497_triFab | FIG. IB | 1 | sp34_K_CL(lambda) | Q38K | SEQ ID NO:74 |
| | | | 2 | 497_DCS_CL | Q38D Q124C C214S | SEQ ID NO:75 |
| | | | 7 | KCS_sp34.87_D_hole_AA_497(SGL) | Q39D (first Fab), L234A L235A, T366S L368A Y407V, Q39K F126C C220S (second Fab) | SEQ ID NO: 78 (first variable region) |
| | | | | | | SEQ ID NO: 76 (second variable region) |
| | | | 4 | hz5E12.1-L | N/A | SEQ ID NO:52 |
| | | | 5 | hz5E12.1-H | L234A L235A, T366W | SEQ ID NO:48 |
| B3b (87) | 497(GLS)_sp34.8 7_5E 12 _triFab | FIG. 1A | 1 | sp34_K_CL(lambda) | Q38K | SEQ ID NO:74 |
| | | | 8 | GPRC5D_LC_Q38D_Q124C_C214S | Q38D Q124C C214S | SEQ ID NO:79 |
| | | | 9 | GPRC5D_KCS_sp34.87_D_hole_AA | Q39K F126C C220S (first Fab), Q39D (second Fab), L234A L235A, T366S L368A Y407V | SEQ ID NO: 80 (first variable region) |
| | | | | | | SEQ ID NO: 78 (second variable region) |
| | | | 10 | 497_L | NA | SEQ ID NO:60 |
| | | | 11 | 497(SGL)_H_knob_AA | L234A L235A, T366W | SEQ ID NO:56 |
| B3-7F5.3 (87) | 7F5.3_sp34.87_49 7_triFab | FIG. 1A | 1 | sp34_K_CL(lambda) | Q38K | SEQ ID NO:74 |
| | | | 2 | 497_DCS_CL | Q38D Q124C C214S | SEQ ID NO:75 |
| | | | 6 | 497(SGL)_ KCS_sp34.87_D_hole_AA | Q39K F126C C220S (first Fab), Q39D (second Fab), L234A L235A, T366S L368A Y407V | SEQ ID NO: 76 (first variable region) |
| | | | | | | SEQ ID NO: 78 (second variable region) |
| | | | 14 | hz7F5K.g0 | N/A | SEQ ID NO:44 |
| | | | 15 | hz7F5H.g3-knob | L234A L235A, T366W | SEQ ID NO:40 |
| lnno-G | hz5E12_sp34.24_ Hel_triFab | FIG. 1A | 1 | sp34_K_CL(lambda) | Q38K | SEQ ID NO:74 |
| | | | 18 | Hel_DCS_CL | Q38D Q124C C214S | \ |
| | | | 20 | Hel_KCS_sp34.24_D_hole_AA | Q39K F126C C220S (first Fab), Q39D (second Fab), L234A L235A, T366S L368A Y407V | \ |
| | | | 4 | hz5E12.1-L | N/A | SEQ ID NO:52 |
| | | | 5 | hz5E12.1-H | L234A L235A, T366W | SEQ ID NO:48 |
| Inno-B | Hel_sp34.24_497_ triFab | FIG. 1 A | 1 | sp34_K_CL(lambda) | Q38K | SEQ ID NO:74 |
| | | | 2 | 497_DCS_CL | Q38D Q124C C214S | SEQ ID NO:75 |
| | | | 3 | 497(SGL)_KCS_sp34.24_D_hole_AA | Q39K F126C C220S (first Fab), Q39D (second Fab), L234A L235A, T366S L368A Y407V | SEQ ID NO: 76 (first variable region) |
| | | | | | | SEQ ID NO: 77 (second variable region) |
| | | | 16 | Hel_CL | N/A | \ |
| | | | 17 | Hel_LALA_knob | L234A L235A, T366W | \ |
| B3-7F5.3 (24) | 7F5.3_sp34.24_49 7_triFab | FIG. 1A | 1 | sp34_K_CL(lambda) | Q38K | SEQ ID NO:74 |
| | | | 2 | 497_DCS_CL | Q38D Q124C C214S | SEQ ID NO:75 |
| | | | 3 | 497(SGL)_ KCS_sp34.24_D_hole_AA | Q39K F126C C220S (first Fab), Q39D (second Fab), L234A L235A, T366S L368A Y407V | SEQ ID NO: 76 (first variable region) |
| | | | | | | SEQ ID NO: 77 (second variable region) |
| | | | 14 | hz7F5K.g0 | N/A | SEQ ID NO:44 |
| | | | 15 | hz7F5H.g3-knob | L234A L235A, T366W | SEQ ID NO:40 |
| lnno-G-7F5.3 | | FIG. IA | 1 | sp34_K_CL(lambda) | Q38K | SEQ ID NO:74 |
| | | | 18 | Hcl_DCS_CL | Q38D Q124C C214S | \ |
| | | | 20 | Hel_KCS_sp34.24_D_hole_AA | Q39K F126C C220S (first Fab), Q39D (second Fab), L234A L235A, T366S L368A Y407V | \ |
| | | | 14 | hz7F5K.g0 | N/A | SEQ ID NO:44 |
| | | | 15 | hz7F5H.g3-knob | L234A L235A, T366W | SEQ ID NO:40 |
| F2 (24) | F2-SP34.24-SC38497 | FIG. 1C | 4 | hz5E12.1-H | L234A L235A, T366W | \ |
| | | | 5 | hz5E12.1-L | N/A | \ |
| | | | 21 | SP34.24-HC (hole) -sc38497 | L234A L235A, T366S L368A Y407V, 100C 44C(scFv) | \ |
| | | | 22 | SP34-CL | N/A | \ |
| F2 (87) | F2-SP34.87-SC38497 | FIG. 1C | 4 | hz5E12.1-H | L234A L235A, T366W | \ |
| | | | 5 | hz5E12.1-L | N/A | \ |
| | | | 23 | SP34.87-HC (hole) -sc38497 | L234A L235A, T366S L368A Y407V, 100C 44C(scFv) | \ |
| | | | 22 | SP34-CL | N/A | \ |
| REGN-BCMA/CD3 | REGN5458 | | 24 | bema-VH | \ | \ |
| | | | 25 | Common L chain-VL | \ | \ |
| | | | 26 | CD3-1 | \ | \ |
| | | | | | | |
| | JNJ-GPRC5D/CD3 | | 27 | JJ-GPRC5D-HC | \ | \ |
| | | | 28 | JJ-GPRC5D-LC | \ | \ |
| | | | 29 | JJ-CD3c-HC | \ | \ |
| | | | 30 | JJ-CD3e-LC | \ | \ |
| | Roche-GPRC5D/CD3 | | 31 | 5E11-GPRC5D-LC | \ | \ |
| | | | 32 | CD3-LC | \ | \ |
| | | | 33 | 5E11-TCB-HC-hole | \ | \ |
| | | | 34 | 5E11-TCB-HC-knob | \ | \ |
| | JNJ-BCMA/CD3 | | 35 | BCMA69-CH1-Fc(hIgG4_LALAF405L) | \ | \ |
| | | | 36 | BCMA69-CL(hLambda) | \ | \ |
| | | | 29 | CD3B219-CH1-Fc(hIgG4_LALAF405L) | \ | \ |
| | | | 30 | CD3B219(Teclistamab)-CL(li Lanibda) | \ | \ |
| | Roche-BCMA/CD3 | | 32 | CD3 VH-CL | \ | \ |
| | | | 37 | BCMA VH_CH1xCD3 VL_CH1 Fc knob LALA PG | \ | \ |
| | | | 38 | BCMA huIgG1 LC (BCMA LC) | \ | \ |
| | | | 39 | BCMA HC hole LALA PG (hole BCMA HC) | \ | \ |

| | | | | | | |
|---|---|---|---|---|---|---|
| "\" represents that no relevant information is shown "N/A" represents no mutation | | | | | | |

### 2. Antibody sequences from which antigen-binding regions are derived

| Type | Antibody name | Specific sequence information | SEQ ID NO |
|---|---|---|---|
| Anti-GPRC5D antibody | hz7F5.3 | Heavy chain variable region | 40 |
| | | HCDR1 | 41 |
| | | HCDR2 | 42 |
| | | HCDR3 | 43 |
| | | Light chain variable region | 44 |
| | | LCDR1 | 45 |
| | | LCDR2 | 46 |
| | | LCDR3 | 47 |
| | hz5E12.1.P1 | Heavy chain variable region | 48 |
| | | HCDR1 | 49 |
| | | HCDR2 | 50 |
| | | HCDR3 | 51 |
| | | Light chain variable region | 52 |
| | | LCDR1 | 53 |
| | | LCDR2 | 54 |
| | | LCDR3 | 55 |
| Anti-BCMA antibody | ADI-38497 | Heavy chain variable region | 56 |
| | | HCDR1 | 57 |
| | | HCDR2 | 58 |
| | | HCDR3 | 59 |
| | | Light chain variable region | 60 |
| | | LCDR1 | 61 |
| | | LCDR2 | 62 |
| | | LCDR3 | 63 |
| Anti-CD3 antibody | hzsp34.24 | Heavy chain variable region | 64 |
| | | HCDR1 | 65 |
| | | HCDR2 | 66 |
| | | HCDR3 | 67 |
| | | Light chain variable region | 68 |
| | | LCDR1 | 69 |
| | | LCDR2 | 70 |
| | | LCDR3 | 71 |
| | hzsp34.87 | Heavy chain variable region | 72 |
| | | HCDR1 | 65 |
| | | HCDR2 | 66 |
| | | HCDR3 | 73 |
| | | Light chain variable region | 68 |
| | | LCDR1 | 69 |
| | | LCDR2 | 70 |
| | | LCDR3 | 71 |

### 3. Other sequences

| Sequence name | Mutation information | SEQ ID NO |
|---|---|---|
| Wild-type IgG1 CH1 domain | N/A | 82 |
| Wild-type IgG1 CH2 domain | N/A | 83 |
| Wild-type IgG1 CH3 domain | N/A | 84 |
| Human IgG1 CH1 mutation | F126C | 85 |
| Human IgG1 CH1 mutation | F126C,C220S | 86 |
| Wild-type human IgG1 Fc region polypeptide | | 87 |
| Wild-type human IgG4 Fc region polypeptide | | 88 |
| Human IgG1 Fc region polypeptide (LALA mutation, no kih mutation) | L234A/L235A | 89 |
| Human IgG4 Fc region polypeptide (LALA mutation, no kih mutation) | L234A/L235A | 90 |
| Human IgG1 Fc region polypeptide (comprising hole mutation) | T366S, L368A, Y407V | 91 |
| Human IgG1 Fc region polypeptide (comprising knob mutation) | T366W | 92 |
| Human IgG1 Fc region polypeptide (comprising hole mutation and LALA) | L234A, L235A, T366S, L368A, Y407V | 93 |
| Human IgG1 Fc region polypeptide (comprising knob mutation and LALA) | L234A, L235A, T366W | 94 |
| Human kappa light chain CL region | N/A | 95 |
| Human lambda light chain CL region | N/A | 96 |
| Human kappa light chain CL region | Q124C | 97 |
| Human kappa light chain CL region | Q124C and C214S | 98 |
| Hinge region | | 99 |
| Negative control anti-Hel antibody light chain | | 100 |
| Negative control anti-Hel antibody heavy chain | | 101 |
| Full-length amnio acid sequence of human BCMA | | 102 |
| Amnio acid sequence of BCMA-P34del (amnio acid P deleted at position 34) | | 103 |
| Amnio acid sequence of BCMA- R27P (amnio acid R mutated to P at position 27) | | 104 |
| Amnio acid sequence of BCMA- R39A (amnio acid R mutated to A at position 39) | | 105 |
| Amnio acid sequence of BCMA- S30del (amnio acid S deleted at position 30) | | 106 |
| Full-length nucleic acid sequence of human BCMA | | 107 |
| Nucleic acid sequence of BCMA-P34del(amnio acid P deleted at position 34) | | 108 |
| Nucleic acid sequence of BCMA- R27P (amnio acid R mutated to P at position 27) | | 109 |
| Nucleic acid sequence of BCMA- R39A(amnio acid R mutated to A at position 39) | | 110 |
| Nucleic acid sequence of BCMA- S30del(amnio acid S deleted at position 30) | | 111 |

### 4. Specific sequence information

| SEQ ID NO: | Sequence |
|---|---|
| 1 | QAVVTQEPSLTVSPGGTVTLTCRSSTGAVTTSNYANWVQKKPGQAPRGLIGGTNKRAPGVP ARFSGSLLGDKAALTLLGAQPEDEAEYYCALWYSNLWVFGQGTKLTVLGQPKAAPSVTLFP PSSEELQANKATLVCLISDFYPGAVTVAWKADSSPVKAGVETTTPSKQSNNKYAASSYLSLTP EQWKSHRSYSCQVTHEGSTVEKTVAPTECS |
| 2 | DIQMTQSPSSLSASVGDRVTITCRASQSISRYLNWYQDKPGKAPKLLIYAASSLQSGVPSRFS GSGSGTDFTLTISSLQPEDFATYYCQQKYFDITFGGGTKVEIKRTVAAPSVFIFPPSDECLKSG TASVVCLLNNFYPREAKVQWKVDNALQSGNSQESVTEQDSKDSTYSLSSTLTLSKADYEKH KVYACEVTHQGLSSPVTKSFNRGES |
| 3 | QLQLQESGPGLVKPSETLSLTCTVSGGSISSSSYYWTWIRKPPGKGLEWIGSISIAGSTYYNPS LKSRVTISVDTSKNQFSLKLSSVTAADTAVYYCARDRGDQILDVWGQGTLVTVSS ASTKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS GGGGSGGGGS EVQLVESGGGLVQPGGSLKLSCAASGFTFNTYAMNWVRDASGKGLEWVGRIRSKYNNYAT YYADSVKDRFTISRDDSKSTLYLQMNSLKTEDTAVYYCARHGNFGQSYVSWFAYWGQGTT VTVSS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSC DKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLSCAVKGFYPSDIAVEWESNGQPENNYKTTPPVLDSDGS FFLVSKLTVDKSRWQQGNVFSCSVMHEALHNHYTQKSLSLSPGK |
| 4 | DIVMTQSPDSLAVSLGERATINCRASESVDSYGITFMNWYQQKPGQPPKLLIYAASNQGSGV PDRFSGSGSGTDFTLTISSLQAEDVAVYYCQQSKEVPWTFGQGTKLEIK RTVAAPSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC |
| 5 | QVQLVQSGAEVKKPGASVKVSCKASGYTFIGYVMHWVRQAPGQGLEWMGYINPYNDVS QHQGKFKGRVTMTSDTSTSTVYMELSSLRSEDTAVYYCAREGRLGLRVLAYWGQGTLVTV SS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEAAGGPSVFL FPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSRDELTKNQV SLWCLVKGFYPSDIAVEWESNGQPENNYKTTPPVLDSDGSFFLYSKLTVDKSRWQQGNVFS CSVMHEALHNHYTQKSLSLSPGK |
| 6 | QLQLQESGPGLVKPSETLSLTCTVSGGSISSSSYYWTWIRKPPGKGLEWIGSISIAGSTYYNPS LKSRVTISVDTSKNQFSLKLSSVTAADTAVYYCARDRGDQILDVWGQGTLVTVSS ASTKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS GGGGSGGGGS EVQLVESGGGLVQPGGSLKLSCAASGFTFNTYAMNWVRDASGKGLEWVGRIRSKYNNYAT YYADSVKDRFTISRDDSKSTLYLQMNSLKTEDTAVYYCARHYNFGQSYVSWFAYWGQGTT VTVSS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSC DKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLSCAVKGFYPSDIAVEWESNGQPENNYKTTPPVLDSDGS FFLVSKLTVDKSRWQQGNVFSCSVMHEALHNHYTQKSLSLSPGK |
| 7 | EVQLVESGGGLVQPGGSLKLSCAASGFTFNTYAMNWVRDASGKGLEWVGRIRSKYNNYAT YYADSVKDRFTISRDDSKSTLYLQMNSLKTEDTAVYYCARHYNFGQSYVSWFAYWGQGTT VTVSS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSC DKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLSCAVKGFYPSDIAVEWESNGQPENNYKTTPPVLDSDGS FFLVSKLTVDKSRWQQGNVFSCSVMHEALHNHYTQKSLSLSPGKGGGGSGGGGS QLQLQESGPGLVKPSETLSLTCTVSGGSISSSYYWTWIRKPPGKGLEWIGSISIAGSTYYNPS LKSRVTISVDTSKNQFSLKLSSVTAADTAVYYCARDRGDQILDVWGQGTLVTVSS ASTKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS |
| 8 | DIVMTQSPDSLAVSLGERATINCRASESVDSYGITFMNWYQDKPGQPPKLLIYAASNQGSGV PDRFSGSGSGTDFTLTISSLQAEDVAVYYCQQSKEVPWTFGQGTKLEIK RTVAAPSVFIFPPSDECLKSGTASVVCLLNNFYPREAKVQWKVDNALQSGNSQESVTEQDS KDSTYSLSSTLTLSKADYEKHKVYACEVTHQGLSSPVTKSFNRGES |
| 9 | QVQLVQSGAEVKKPGASVKVSCKASGYTFIGYVMHWVRKAPGQGLEWMGYINPYNDVS QHQGKFKGRVTMTSDTSTSTVYMELSSLRSEDTAVYYCAREGRLGLRVLAYWGQGTLVTV SS ASTKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS GGGGSGGGGS EVQLVESGGGLVQPGGSLKLSCAASGFTFNTYAMNWVRDASGKGLEWVGRIRSKYNNYAT YYADSVKDRFTISRDDSKSTLYLQMNSLKTEDTAVYYCARHYNFGQSYVSWFAYWGQGTT VTVSS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSC DKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLSCAVKGFYPSDIAVEWESNGQPENNYKTTPPVLDSDGS FFLVSKLTVDKSRWQQGNVFSCSVMHEALHNHYTQKSLSLSPGK |
| 10 | DIQMTQSPSSLSASVGDRVTITCRASQSISRYLNWYQQKPGKAPKLLIYAASSLQSGVPSRFS GSGSGTDFTLTISSLQPEDFATYYCQQKYFDITFGGGTKVEIK RTVAAPSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC |
| 11 | QLQLQESGPGLVKPSETLSLTCTVSGGSISSSYYWTWIRQPPGKGLEWIGSISIAGSTYYNPS LKSRVTISVDTSKNQFSLKLSSVTAADTAVYYCARDRGDQILDVWGQGTLVTVSS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSC DKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLWCLVKGFYPSDIAVEWESNGQPENNYKTTPPVLDSDGS FFLYSKLTVDKSRWQQGNVFSCSVMHEALHNHYTQKSLSLSPGK |
| 12 | GACTCCAGAATGATGCCCCA |
| 13 | CAATAACGCTGACATGTTAG |
| 14 | DIQMTQSPSSLSASVGDRVTITCRASQDIRNYLNWYQQKPGKAPKLLIYYTSRLHSGVPSRF SGSGSGTDFTLTISSLQPEDFATYYCQQGNMLPWTFGQGTKLEIK RTVAAPSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC |
| 15 | QVQLQESGPGLVKPSQTLSLTCTVSGYSITSDYAWNWIRQPPGKGLEWIVYIDYTGRTSYNP SLKSRVTLSRDTSKNQFSLKLSSVTAADTAVYYCARGNYGRYYYVMDYWGQGTLVTVSS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEAAGGPSVFL FPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSRDELTKNQV SLWCLVKGFYPSDIAVEWESNGQPENNYKTTPPVLDSDGSFFLYSKLTVDKSRWQQGNVFS CSVMHEALHNHYTQKSLSLSPGK |
| 16 | DIQMTQSPSSLSASVGDRVTITCRASQSISSYLNWYQQKPGKAPKLLIYAASSLQSGVPSRFS GSGSGTDFTLTISSLQPEDFATYYCQQADLPAFAFGGGTKVEIKRTVAAPSVFIFPPSDEQLKS GTASVVCLLNNFYPREAKVQWKVDNALQSGNSQESVTEQDSKDSTYSLSSTLTLSKADYEK HKVYACEVTHQGLSSPVTKSFNRGEC |
| 17 | EVRLLESGGGLVQPGGSLRLSCAASGFTFSNYAMGWVRQAPGKGLEWVSAISGSGGSTYY ADSVKGRFTTSRDDSKNALYLQMNSLRAEDTAVYYCARGGPGWYAADVWGQGTTVTVSS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEAAGGPSVFL FPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSRDELTKNQV SLWCLVKGFYPSDIAVEWESNGQPENNYKTTPPVLDSDGSFFLYSKLTVDKSRWQQGNVFS CSVMHEALHNHYTQKSLSLSPGK |
| 18 | DIQMTQSPSSLSASVGDRVTITCRASQSISSYLNWYQDKPGKAPKLLIYAASSLQSGVPSRFS GSGSGTDFTLTISSLQPEDFATYYCQQADLPAFAFGGGTKVEIKRTVAAPSVFIFPPSDECLKS GTASVVCLLNNFYPREAKVQWKVDNALQSGNSQESVTEQDSKDSTYSLSSTLTLSKADYEK HKVYACEVTHQGLSSPVTKSFNRGES |
| 19 | HTCPXCP |
| 20 | EVRLLESGGGLVQPGGSLRLSCAASGFTFSNYAMGWVRKAPGKGLEWVSAISGSGGSTYY ADSVKGRFTTSRDDSKNALYLQMNSLRAEDTAVYYCARGGPGWYAADVWGQGTTVTVSS ASTKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSSGGGGSGGGGSEVQLVESGGG LVQPGGSLKLSCAASGFTFNTYAMNWVRDASGKGLEWVGRIRSKYNNYATYYADSVKDRF TISRDDSKSTLYLQMNSLKTEDTAVYYCARHGNFGQSYVSWFAYWGQGTTVTVSSASTKGP SVFPLAPSSKSTSGGTAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEAAGGPSVFLFPPKPK DTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSRDELTKNQVSLSCAV KGFYPSDIAVEWESNGQPENNYKTTPPVLDSDGSFFLVSKLTVDKSRWQQGNVFSCSVMHE ALHNHYTQKSLSLSPGK |
| 21 | EVQLVESGGGLVQPGGSLKLSCAASGFTFNTYAMNWVRQASGKGLEWVGRIRSKYNNYAT YYADSVKDRFTISRDDSKSTLYLQMNSLKTEDTAVYYCARHGNFGQSYVSWFAYWGQGTT VTVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEAAG GPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDELT KNQVSLSCAVKGFYPSDIAVEWESNGQPENNYKTTPPVLDSDGSFFLVSKLTVDKSRWQQG NVFSCSVMHEALHNHYTQKSLSLSPGAGGGGSGGGGSDIQMTQSPSSLSASVGDRVTITCR ASQSISRYLNWYQQKPGKAPKLLIYAASSLQSGVPSRFSGSGSGTDFTLTISSLQPEDFATYYC QQKYFDITFGCGTKVEIKGGGGSGGGGSGGGGSQLQLQESGPGLVKPSETLSLTCTVSGGSI VSSSYYWTWIRQPPGKCLEWIGSISIAGSTYYNPSLKSRVTISVDTSKNQFSLKLSSVTAADT AVYYCARDRGDQILDVWSQGTMVTVSS |
| 22 | QAVVTQEPSLTVSPGGTVTLTCRSSTGAVTTSNYANWVQQKPGQAPRGLIGGTNKRAPGVP ARFSGSLLGDKAALTLLGAQPEDEAEYYCALWYSNLWVFGQGTKLTVLGQPKAAPSVTLFP PSSEELQANKATLVCLISDFYPGAVTVAWKADSSPVKAGVETTTPSKQSNNKYAASSYLSLTP EQWKSHRSYSCQVTHEGSTVEKTVAPTECS |
| 23 | EVQLVESGGGLVQPGGSLKLSCAASGFTFNTYAMNWVRQASGKGLEWVGRIRSKYNNYAT YYADSVKDRFTISRDDSKSTLYLQMNSLKTEDTAVYYCARHYNFGQSYVSWFAYWGQGTT VTVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEAAG GPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDELT KNQVSLSCAVKGFYPSDIAVEWESNGQPENNYKTTPPVLDSDGSFFLVSKLTVDKSRWQQG NVFSCSVMHEALHNHYTQKSLSLSPGAGGGGSGGGGSDIQMTQSPSSLSASVGDRVTITCR ASQSISRYLNWYQQKPGKAPKLLIYAASSLQSGVPSRFSGSGSGTDFTLTISSLQPEDFATYYC QQKYFDITFGCGTKVEIKGGGGSGGGGSGGGGSQLQLQESGPGLVKPSETLSLTCTVSGGSI VSSSYYWTWIRQPPGKCLEWIGSISIAGSTYYNPSLKSRVTISVDTSKNQFSLKLSSVTAADT AVYYCARDRGDQILDVWSQGTMVTVSS |
| 24 | EVQLVESGGGLVQPGGSLRLSCAASGFTFSNFWMTWVRQAPGKGLEWVANMNQDGSEKY YVDSVKGRFTISRDNAKSSLYLQMNSLRAEDTAVYYCARDREYCISTSCYDDFDYWGQGTL VTVSS |
| 25 | DIQMTQSPSSLSASVGDRVTITCRASQSISSYLNWYQQKPGKAPKLLIYAASSLQSGVPS RFSGSGSGTDFTLTISSLQPEDFATYYCQQSYSTPPITFGQGTRLEIK |
| 26 | EVQLVESGGGLVQPGRSLRLSCAASGFTFDDYSMHWVRQAPGKGLEWVSGISWNSGSKGY ADSVKGRFTISRDNAKNSLYLQMNSLRAEDTALYYCAKYGSGYGKFYHYGLDVWGQGTT V TVSS |
| 27 | QVQLVQSGAEVKKPGASVKVSCKASGYSFTGYTMNWVRQAPGQGLEWMGLINPYNSDTN YAQKLQGRVTMTTDTSTSTAYMELRSLRSDDTAVYYCARVALRVALDYWGQGTLVTVSSAS TKGPSVFPLAPCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPPCPAPEAAGGPSVFLFPPKP KDTLMISRTPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMTKNQVSLTCL VKGFYPSDIAVEWESNGQPENNYKTTPPVLDSDGSFFLYSRLTVDKSRWQEGNVFSCSVMH EALHNHYTQKSLSLSLGK |
| 28 | DIQMTQSPSSLSASVGDRVTITCKASQNVATHVGWYQQKPGKAPKRLIYSASYRYSGVPSRF SGSGSGTEFTLTISNLQPEDFATYYCQQYNRYPYTFGQGTKLEIKRTVAAPSVFIFPPSDEQLK SGTASVVCLLNNFYPREAKVQWKVDNALQSGNSQESVTEQDSKDSTYSLSSTLTLSKADYE KHKVYACEVTHQGLSSPVTKSFNRGEC |
| 29 | EVQLVESGGGLVQPGGSLRLSCAASGFTFNTYAMNWVRQAPGKGLEWVARIRSKYNNYAT YYAASVKGRFTISRDDSKNSLYLQMNSLKTEDTAVYYCARHGNFGNSYVSWFAYWGQGTL VTVSSASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPPCPAPEAAGGPSV FLFPPKPKDTLMISRTPEVTCVVVDVSQEDPEVQFNWYVDGVEVHNAKTKPREEQFNSTYR VVSVLTVLHQDWLNGKEYKCKVSNKGLPSSIEKTISKAKGQPREPQVYTLPPSQEEMTKNQ VSLTCLVKGFYPSDIAVEWESNGQPENNYKTTPPVLDSDGSFLLYSKLTVDKSRWQEGNVFS CSVMHEALHNHYTQKSLSLSLGK |
| 30 | QTVVTQEPSLTVSPGGTVTLTCRSSTGAVTTSNYANWVQQKPGQAPRGLIGGTNKRAPGTP ARFSGSLLGGKAALTLSGVQPEDEAEYYCALWYSNLWVFGGGTKLTVLGQPKAAPSVTLFP PSSEELQANKATLVCLISDFYPGAVTVAWKADSSPVKAGVETTTPSKQSNNKYAASSYLSLTP EQWKSHRSYSCQVTHEGSTVEKTVAPTECS |
| 31 | DIVLTQSPALAVSPGQRATISCRASQSVSISGINLMNWYQQKPGQQPKLLIYHASILASGIPTR FSGSGSGTDFTLTIDPVQADDIATYYCQQTRESPLTFGSGTNLEIKRTVAAPSVFIFPPSDRKLK SGTASVVCLLNNFYPREAKVQWKVDNALQSGNSQESVTEQDSKDSTYSLSSTLTLSKADYE KHKVYACEVTHQGLSSPVTKSFNRGEC |
| 32 | EVQLLESGGGLVQPGGSLRLSCAASGFTFSTYAMNWVRQAPGKGLEWVSRIRSKYNNYAT YYADSVKGRFTISRDDSKNTLYLQMNSLRAEDTAVYYCVRHGNFGNSYVSWFAYWGQGTL VTVSSASVAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |
| 33 | ELQLEQSGGGLVQPGGSLTLSCAASGFTFSKYAMAWVRQAPTKGLEWVASISTGGVNTYYR DSVKARFTISRDNAKNTQYLQMDSLRSEDTATYYCATHTGDYFDYWGQGVMVTVSSASTK GPSVFPLAPSSKSTSGGTAALGCLVEDYFPEPVTVSWNSGALTSGVHTFPAVLQSSGLYSLSS VVTVPSSSLGTQTYICNVNHKPSNTKVDEKVEPKSCDKTHTCPPCPAPEAAGGPSVFLFPPK PKDTLMISRTPEVTCVVVDVSHEDPEVKFNWYVDGVEVHNAKTKPREEQYNSTYRVVSVL TVLHQDWLNGKEYKCKVSNKALGAPIEKTISKAKGQPREPQVCTLPPSRDELTKNQVSLSC AVKGFYPSDIAVEWESNGQPENNYKTTPPVLDSDGSFFLVSKLTVDKSRWQQGNVFSCSVM HEALHNHYTQKSLSLSPGK |
| 34 | ELQLEQSGGGLVQPGGSLTLSCAASGFTFSKYAMAWVRQAPTKGLEWVASISTGGVNTYYR DSVKARFTISRDNAKNTQYLQMDSLRSEDTATYYCATHTGDYFDYWGQGVMVTVSSASTK GPSVFPLAPSSKSTSGGTAALGCLVEDYFPEPVTVSWNSGALTSGVHTFPAVLQSSGLYSLSS VVTVPSSSLGTQTYICNVNHKPSNTKVDEKVEPKSCDGGGGSGGGGSQAVVTQEPSLTVSP GGTVTLTCGSSTGAVTTSNYANWVQEKPGQAFRGLIGGTNKRAPGTPARFSGSLLGGKAAL TLSGAQPEDEAEYYCALWYSNLWVFGGGTKLTVLSSASTKGPSVFPLAPSSKSTSGGTAAL GCLVKDYFPEPVTVSWNSGALTSGVHTFPAVLQSSGLYSLSSVVTVPSSSLGTQTYICNVNH KPSNTKVDKKVEPKSCDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDWLNGKEYKCKVSN KALGAPIEKTISKAKGQPREPQVYTLPPCRDELTKNQVSLWCLVKGFYPSDIAVEWESNGQP ENNYKTTPPVLDSDGSFFLYSKLTVDKSRWQQGNVFSCSVMHEALHNHYTQKSLSLSPGK |
| 35 | QLQLQESGPGLVKPSETLSLTCTVSGGSISSGSYFWGWIRQPPGKGLEWIGSIYYSGITYYNP SLKSRVTISVDTSKNQFSLKLSSVTAADTAVYYCARHDGAVAGLFDYWGQGTLVTVSSAST KGPSVFPLAPCSRSTSESTAALGCLVKDYFPEPVTVSWNSGALTSGVHTFPAVLQSSGLYSLS SVVTVPSSSLGTKTYTCNVDHKPSNTKVDKRVESKYGPPCPPCPAPEAAGGPSVFLFPPKPK DTLMISRTPEVTCVVVDVSQEDPEVQFNWYVDGVEVHNAKTKPREEQFNSTYRVVSVLTV LHQDWLNGKEYKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQEGNVFSCSVMHE ALHNHYTQKSLSLSLGK |
| 36 | SYVLTQPPSVSVAPGQTARITCGGNNIGSKSVHWYQQPPGQAPVVVVYDDSDRPSGIPERFS GSNSGNTATLTISRVEAGDEAVYYCQVWDSSSDHVVFGGGTKLTVLGQPKAAPSVTLFPPSS EELQANKATLVCLISDFYPGAVTVAWKGDSSPVKAGVETTTPSKQSNNKYAASSYLSLTPEQ WKSHRSYSCQVTHEGSTVEKTVAPTECS |
| 37 | EVQLLESGGGLVQPGGSLRLSCAASGFTFSDNAMGWVRQAPGKGLEWVSAISGPGSSTYY ADSVKGRFTISRDNSKNTLYLQMNSLRAEDTAVYYCAKVLGWFDYWGQGTLVTVSSASTK GPSVFPLAPSSKSTSGGTAALGCLVEDYFPEPVTVSWNSGALTSGVHTFPAVLQSSGLYSLSS VVTVPSSSLGTQTYICNVNHKPSNTKVDEKVEPKSCDGGGGSGGGGSQAVVTQEPSLTVSP GGTVTLTCGSSTGAVTTSNYANWVQEKPGQAFRGLIGGTNKRAPGTPARFSGSLLGGKAAL TLSGAQPEDEAEYYCALWYSNLWVFGGGTKLTVLSSASTKGPSVFPLAPSSKSTSGGTAAL GCLVKDYFPEPVTVSWNSGALTSGVHTFPAVLQSSGLYSLSSVVTVPSSSLGTQTYICNVNH KPSNTKVDKKVEPKSCDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDWLNGKEYKCKVSN KALGAPIEKTISKAKGQPREPQVYTLPPCRDELTKNQVSLWCLVKGFYPSDIAVEWESNGQP ENNYKTTPPVLDSDGSFFLYSKLTVDKSRWQQGNVFSCSVMHEALHNHYTQKSLSLSPGK |
| 38 | EIVLTQSPGTLSLSPGERATLSCRASQSVSDEYLSWYQQKPGQAPRLLIHSASTRATGIPDRFS GSGSGTDFTLAISRLEPEDFAVYYCQQYGYPPDFTFGQGTKVEIKRTVAAPSVFIFPPSDRKL KSGTASVVCLLNNFYPREAKVQWKVDNALQSGNSQESVTEQDSKDSTYSLSSTLTLSKADY EKHKVYACEVTHQGLSSPVTKSFNRGEC |
| 39 | EVQLLESGGGLVQPGGSLRLSCAASGFTFSDNAMGWVRQAPGKGLEWVSAISGPGSSTYY ADSVKGRFTISRDNSKNTLYLQMNSLRAEDTAVYYCAKVLGWFDYWGQGTLVTVSSASTK GPSVFPLAPSSKSTSGGTAALGCLVEDYFPEPVTVSWNSGALTSGVHTFPAVLQSSGLYSLSS VVTVPSSSLGTQTYICNVNHKPSNTKVDEKVEPKSCDKTHTCPPCPAPEAAGGPSVFLFPPK PKDTLMISRTPEVTCVVVDVSHEDPEVKFNWYVDGVEVHNAKTKPREEQYNSTYRVVSVL TVLHQDWLNGKEYKCKVSNKALGAPIEKTISKAKGQPREPQVCTLPPSRDELTKNQVSLSC AVKGFYPSDIAVEWESNGQPENNYKTTPPVLDSDGSFFLVSKLTVDKSRWQQGNVFSCSVM HEALHNHYTQKSLSLSPGK |
| 40 | QVQLQESGPGLVKPSQTLSLTCTVSGYSITSDYAWNWIRQPPGKGLEWIVYIDYTGRTSYNP SLKSRVTLSRDTSKNQFSLKLSSVTAADTAVYYCARGNYGRYYYVMDYWGQGTLVTVSS |
| 41 | SDYAWN |
| 42 | YIDYTGRTSYNPSLKS |
| 43 | GNYGRYYYVMDY |
| 44 | DIQMTQSPSSLSASVGDRVTITCRASQDIRNYLNWYQQKPGKAPKLLIYYTSRLHSGVPSRF SGSGSGTDFTLTISSLQPEDFATYYCQQGNMLPWTFGQGTKLEIK |
| 45 | RASQDIRNYLN |
| 46 | YTSRLHS |
| 47 | QQGNMLPWT |
| 48 | QVQLVQSGAEVKKPGASVKVSCKASGYTFIGYVMHWVRQAPGQGLEWMGYINPYNDVS QIIQGKFKGRVTMTSDTSTSTVYMELSSLRSEDTAVYYCAREGRLGLRVLAYWGQGTLVTV SS |
| 49 | GYVMH |
| 50 | YINPYNDVSQHQGKFKG |
| 51 | EGRLGLRVLAY |
| 52 | DIVMTQSPDSLAVSLGERATINCRASESVDSYGITFMNWYQQKPGQPPKLLIYAASNQGSGV PDRFSGSGSGTDFTLTISSLQAEDVAVYYCQQSKEVPWTFGQGTKLEIK |
| 53 | RASESVDSYGITFMN |
| 54 | AASNQGS |
| 55 | QQSKEVPWT |
| 56 | QLQLQESGPGLVKPSETLSLTCTVSGGSISSSSYYWTWIRQPPGKGLEWIGSISIAGSTYYNPS LKSRVTISVDTSKNQFSLKLSSVTAADTAVYYCARDRGDQILDVWGQGTLVTVSS |
| 57 | SSSYYWT |
| 58 | SISIAGSTYYNPSLKS |
| 59 | DRGDQILDV |
| 60 | DIQMTQSPSSLSASVGDRVTITCRASQSISRYLNWYQQKPGKAPKLLIYAASSLQSGVPSRFS GSGSGTDFTLTISSLQPEDFATYYCQQKYFDITFGGGTKVEIK |
| 61 | RASQSISRYLN |
| 62 | AASSLQS |
| 63 | QQKYFDIT |
| 64 | EVQLVESGGGLVQPGGSLKLSCAASGFTFNTYAMNWVRQASGKGLEWVGRIRSKYNNYAT YYADSVKDRFTISRDDSKSTLYLQMNSLKTEDTAVYYCARHGNFGQSYVSWFAYWGQGTT VTVSS |
| 65 | TYAMN |
| 66 | RIRSKYNNYATYYADSVKD |
| 67 | HGNFGQSYVSWFAY |
| 68 | QAVVTQEPSLTVSPGGTVTLTCRSSTGAVTTSNYANWVQQKPGQAPRGLIGGTNKRAPGVP ARFSGSLLGDKAALTLLGAQPEDEAEYYCALWYSNLWVFGQGTKLTVL |
| 69 | RSSTGAVTTSNYAN |
| 70 | GTNKRAP |
| 71 | ALWYSNLWV |
| 72 | EVQLVESGGGLVQPGGSLKLSCAASGFTFNTYAMNWVRQASGKGLEWVGRIRSKYNNYAT YYADSVKDRFTISRDDSKSTLYLQMNSLKTEDTAVYYCARHYNFGQSYVSWFAYWGQGTT VTVSS |
| 73 | HYNFGQSYVSWFAY |
| 74 | QAVVTQEPSLTVSPGGTVTLTCRSSTGAVTTSNYANWVQKKPGQAPRGLIGGTNKRAPGVP ARFSGSLLGDKAALTLLGAQPEDEAEYYCALWYSNLWVFGQGTKLTVL |
| 75 | DIQMTQSPSSLSASVGDRVTITCRASQSISRYLNWYQDKPGKAPKLLIYAASSLQSGVPSRFS GSGSGTDFTLTISSLQPEDFATYYCQQKYFDITFGGGTKVEIK |
| 76 | QLQLQESGPGLVKPSETLSLTCTVSGGSISSSYYWTWIRKPPGKGLEWIGSISIAGSTYYNPS LKSRVTISVDTSKNQFSLKLSSVTAADTAVYYCARDRGDQILDVWGQGTLVTVSS |
| 77 | EVQLVESGGGLVQPGGSLKLSCAASGFTFNTYAMNWVRDASGKGLEWVGRIRSKYNNYAT YYADSVKDRFTISRDDSKSTLYLQMNSLKTEDTAVYYCARHGNFGQSYVSWFAYWGQGTT VTVSS |
| 78 | EVQLVESGGGLVQPGGSLKLSCAASGFTFNTYAMNWVRDASGKGLEWVGRIRSKYNNYAT YYADSVKDRFTISRDDSKSTLYLQMNSLKTEDTAVYYCARIIYNFGQSYVSWFAYWGQGTT VTVSS |
| 79 | DIVMTQSPDSLAVSLGERATINCRASESVDSYGITFMNWYQDKPGQPPKLLIYAASNQGSGV PDRFSGSGSGTDFTLTISSLQAEDVAVYYCQQSKEVPWTFGQGTKLEIK |
| 80 | QVQLVQSGAEVKKPGASVKVSCKASGYTFIGYVMHWVRKAPGQGLEWMGYINPYNDVS QHQGKFKGRVTMTSDTSTSTVYMELSSLRSEDTAVYYCAREGRLGLRVLAYWGQGTLVTV SS |
| 81 | CPXCP |
| 82 | ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSC |
| 83 | APELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAK |
| 84 | GQPREPQVYTLPPSRDELTKNQVSLTCLVKGFYPSDIAVEWESNGQPENNYKTTPPVLDSDG SFFLYSKLTVDKSRWQQGNVFSCSVMHEALHNHYTQKSLSLSPGK |
| 85 | ASTKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSC |
| 86 | ASTKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS |
| 87 | DKTHTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDSDGSFFL YSKLTVDKSRWQQGNVFSCSVMHEALHNHYTQKSLSLSPGK |
| 88 | PPCPSCPAPEFLGGPSVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGSFFLYS RLTVDKSRWQEGNVFSCSVMHEALHNHYTQKSLSLSLGK |
| 89 | DKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 90 | PPCPSCPAPEAAGGPSVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGSFFLYS RLTVDKSRWQEGNVFSCSVMHEALHNHYTQKSLSLSLGK |
| 91 | DKTHTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQP REPQVYTLPPSRDELTKNQVSLSCAVKGFYPSDIAVEWESNGQPENNYKTTPPVLDSDGSFF LVSKLTVDKSRWQQGNVFSCSVMHEALHNHYTQKSLSLSPGK |
| 92 | |
| 93 | DKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLSCAVKGFYPSDIAVEWESNGQPENNYKTTPPVLDSDGS FFLVSKLTVDKSRWQQGNVFSCSVMHEALHNHYTQKSLSLSPGK |
| 94 | DKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLWCLVKGFYPSDIAVEWESNGQPENNYKTTPPVLDSDGS FFLYSKLTVDKSRWQQGNVFSCSVMHEALHNHYTQKSLSLSPGK |
| 95 | |
| 96 | GQPKAAPSVTLFPPSSEELQANKATLVCLISDFYPGAVTVAWKADSSPVKAGVETTTPSKQS NNKYAASSYLSLTPEQWKSHRSYSCQVTHEGSTVEKTVAPTECS |
| 97 | RTVAAPSVFIFPPSDECLKSGTASVVCLLNNFYPREAKVQWKVDNALQSGNSQESVTEQDS KDSTYSLSSTLTLSKADYEKHKVYACEVTHQGLSSPVTKSFNRGEC |
| 98 | RTVAAPSVFIFPPSDECLKSGTASVVCLLNNFYPREAKVQWKVDNALQSGNSQESVTEQDS KDSTYSLSSTLTLSKADYEKHKVYACEVTHQGLSSPVTKSFNRGES |
| 99 | DKTHTCPXCP |
| 100 | DIQMTQSPSSLSASVGDRVTITCRASQSISSYLNWYQQKPGKAPKLLIYAASSLQSGVPSRFS GSGSGTDFTLTISSLQPEDFATYYCQQADLPAFAFGGGTKVEIKRTVAAPSVFIFPPSDEQLKS GTASVVCLLNNFYPREAKVQWKVDNALQSGNSQESVTEQDSKDSTYSLSSTLTLSKADYEK HKVYACEVTHQGLSSPVTKSFNRGEC |
| 101 | EVRLLESGGGLVQPGGSLRLSCAASGFTFSNYAMGWVRQAPGKGLEWVSAISGSGGSTYY ADSVKGRFTTSRDDSKNALYLQMNSLRAEDTAVYYCARGGPGWYAADVWGQGTTVTVSS ASTKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPEAAGGPSVFL FPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSRWQQGNVFSC SVMHEALHNHYTQKSLSLSPGK |
| 102 | MLQMAGQCSQNEYFDSLLHACIPCQLRCSSNTPPLTCQRYCNASVTNSVKGTNAILWTCLG LSLIISLAVFVLMFLLRKINSEPLKDEFKNTGSGLLGMANIDLEKSRTGDEIILPRGLEYTVEE CTCEDCIKSKPKVDSDHCFPLPAMEEGATILVTTKTNDYCKSLPAALSATEIEKSISAR |
| 103 | MLQMAGQCSQNEYFDSLLHACIPCQLRCSSNTPLTCQRYCNASVTNSVKGTNAILWTCLGL SLIISLAVFVLMFLLRKINSEPLKDEFKNTGSGLLGMANIDLEKSRTGDEIILPRGLEYTVEEC TCEDCIKSKPKVDSDHCFPLPAMEEGATILVTTKTNDYCKSLPAALSATEIEKSISAR |
| 104 | MLQMAGQCSQNEYFDSLLHACIPCQLPCSSNTPPLTCQRYCNASVTNSVKGTNAILWTCLG LSLIISLAVFVLMFLLRKINSEPLKDEFKNTGSGLLGMANIDLEKSRTGDEIILPRGLEYTVEE CTCEDCIKSKPKVDSDHCFPLPAMEEGATILVTTKTNDYCKSLPAALSATEIEKSISAR |
| 105 | MLQMAGQCSQNEYFDSLLHACIPCQLRCSSNTPPLTCQAYCNASVTNSVKGTNAILWTCLG LSLIISLAVFVLMFLLRKINSEPLKDEFKNTGSGLLGMANIDLEKSRTGDEIILPRGLEYTVEE CTCEDCIKSKPKVDSDHCFPLPAMEEGATILVTTKTNDYCKSLPAALSATEIEKSISAR |
| 106 | MLQMAGQCSQNEYFDSLLHACIPCQLRCSNTPPLTCQRYCNASVTNSVKGTNAILWTCLGL SLIISLAVFVLMFLLRKINSEPLKDEFKNTGSGLLGMANIDLEKSRTGDEIILPRGLEYTVEEC TCEDCIKSKPKVDSDHCFPLPAMEEGATILVTTKTNDYCKSLPAALSATEIEKSISAR |
| 107 | ATGTTGCAGATGGCTGGGCAGTGCTCCCAAAATGAATATTTTGACAGTTTGTTGCATGCT TGCATACCTTGTCAACTTCGATGTTCTTCTAATACTCCTCCTCTAACATGTCAGCGTTATTG TAATGCAAGTGTGACCAATTCAGTGAAAGGAACGAATGCGATTCTCTGGACCTGTTTGG GACTGAGCTTAATAATTTCTTTGGCAGTTTTCGTGCTAATGTTTTTGCTAAGGAAGATAAA CTCTGAACCATTAAAGGACGAGTTTAAAAACACAGGATCAGGTCTCCTGGGCATGGCTA ACATTGACCTGGAAAAGAGCAGGACTGGTGATGAAATTATTCTTCCGAGAGGCCTCGAG TACACGGTGGAAGAATGCACCTGTGAAGACTGCATCAAGAGCAAACCGAAGGTgGACT CTGACCATTGCTTTCCACTCCCAGCTATGGAGGAAGGCGCAACCATTCTTGTCACCACGA AAACGAATGACTATTGCAAGAGCCTGCCAGCTGCTTTGAGTGCTACGGAGATAGAGAAA TCAATTTCTGCTAGG |
| 108 | ATGTTGCAGATGGCTGGGCAGTGCTCCCAAAATGAATATTTTGACAGTTTGTTGCATGCT TGCATACCTTGTCAACTTCGATGTTCTTCTAATACTCCTCTAACATGTCAGCGTTATTGTAA TGCAAGTGTGACCAATTCAGTGAAAGGAACGAATGCGATTCTCTGGACCTGTTTGGGAC TGAGCTTAATAATTTCTTTGGCAGTTTTCGTGCTAATGTTTTTGCTAAGGAAGATAAACTC TGAACCATTAAAGGACGAGTTTAAAAACACAGGATCAGGTCTCCTGGGCATGGCTAACA TTGACCTGGAAAAGAGCAGGACTGGTGATGAAATTATTCTTCCGAGAGGCCTCGAGTAC ACGGTGGAAGAATGCACCTGTGAAGACTGCATCAAGAGCAAACCGAAGGTGGACTCTG ACCATTGCTTTCCACTCCCAGCTATGGAGGAAGGCGCAACCATTCTTGTCACCACGAAA ACGAATGACTATTGCAAGAGCCTGCCAGCTGCTTTGAGTGCTACGGAGATAGAGAAATC AATTTCTGCTAGG |
| 109 | ATGTTGCAGATGGCTGGGCAGTGCTCCCAAAATGAATATTTTGACAGTTTGTTGCATGCT TGCATACCTTGTCAACTTCCTTGTTCTTCTAATACTCCTCCTCTAACATGTCAGCGTTATTG TAATGCAAGTGTGACCAATTCAGTGAAAGGAACGAATGCGATTCTCTGGACCTGTTTGG GACTGAGCTTAATAATTTCTTTGGCAGTTTTCGTGCTAATGTTTTTGCTAAGGAAGATAAA CTCTGAACCATTAAAGGACGAGTTTAAAAACACAGGATCAGGTCTCCTGGGCATGGCTA ACATTGACCTGGAAAAGAGCAGGACTGGTGATGAAATTATTCTTCCGAGAGGCCTCGAG TACACGGTGGAAGAATGCACCTGTGAAGACTGCATCAAGAGCAAACCGAAGGTGGACT CTGACCATTGCTTTCCACTCCCAGCTATGGAGGAAGGCGCAACCATTCTTGTCACCACGA AAACGAATGACTATTGCAAGAGCCTGCCAGCTGCTTTGAGTGCTACGGAGATAGAGAAA TCAATTTCTGCTAGG |
| 110 | ATGTTGCAGATGGCTGGGCAGTGCTCCCAAAATGAATATTTTGACAGTTTGTTGCATGCT TGCATACCTTGTCAACTTCGATGTTCTTCTAATACTCCTCCTCTAACATGTCAGGCTTATTG TAATGCAAGTGTGACCAATTCAGTGAAAGGAACGAATGCGATTCTCTGGACCTGTTTGG GACTGAGCTTAATAATTTCTTTGGCAGTTTTCGTGCTAATGTTTTTGCTAAGGAAGATAAA CTCTGAACCATTAAAGGACGAGTTTAAAAACACAGGATCAGGTCTCCTGGGCATGGCTA ACATTGACCTGGAAAAGAGCAGGACTGGTGATGAAATTATTCTTCCGAGAGGCCTCGAG TACACGGTGGAAGAATGCACCTGTGAAGACTGCATCAAGAGCAAACCGAAGGTGGACT CTGACCATTGCTTTCCACTCCCAGCTATGGAGGAAGGCGCAACCATTCTTGTCACCACGA AAACGAATGACTATTGCAAGAGCCTGCCAGCTGCTTTGAGTGCTACGGAGATAGAGAAA TCAATTTCTGCTAGG |
| 111 | ATGTTGCAGATGGCTGGGCAGTGCTCCCAAAATGAATATTTTGACAGTTTGTTGCATGCT TGCATACCTTGTCAACTTCGATGTTCTAATACTCCTCCTCTAACATGTCAGCGTTATTGTAA TGCAAGTGTGACCAATTCAGTGAAAGGAACGAATGCGATTCTCTGGACCTGTTTGGGAC TGAGCTTAATAATTTCTTTGGCAGTTTTCGTGCTAATGTTTTTGCTAAGGAAGATAAACTC TGAACCATTAAAGGACGAGTTTAAAAACACAGGATCAGGTCTCCTGGGCATGGCTAACA TTGACCTGGAAAAGAGCAGGACTGGTGATGAAATTATTCTTCCGAGAGGCCTCGAGTAC ACGGTGGAAGAATGCACCTGTGAAGACTGCATCAAGAGCAAACCGAAGGTGGACTCTG ACCATTGCTTTCCACTCCCAGCTATGGAGGAAGGCGCAACCATTCTTGTCACCACGAAA ACGAATGACTATTGCAAGAGCCTGCCAGCTGCTTTGAGTGCTACGGAGATAGAGAAATC AATTTCTGCTAGG |

## Claims

1. A trispecific antibody specifically binding to BCMA, comprising a first antigen-binding region specifically binding to BCMA, and second and third antigen-binding regions specifically binding to other antigens.

2. The trispecific antibody according to claim 1, wherein the second antigen-binding region specifically binds to CD3, and/or the third antigen-binding region specifically binds to GPRC5D.

3. The trispecific antibody according to claim 1 or 2, wherein the first antigen-binding region, the second antigen-binding region, and the third antigen-binding region are a first Fab, a second Fab, and a third Fab, respectively.

4. The trispecific antibody according to claim 3, wherein
(1) the first Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the third Fab is fused to N-terminus of a VH of the Fab heavy chain of the second Fab fragment at C-terminus of a CH1 of an Fab heavy chain;
or
the third Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the first Fab is fused to N-terminus of a VH of the Fab heavy chain of the second Fab fragment at C-terminus of a CH1 of an Fab heavy chain;
or
(2) the first Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the third Fab is fused to C-terminus of the Fc region fused to the second Fab at N-terminus of a VH of an Fab heavy chain; or
the third Fab is fused to a CH2 or a hinge region of one of Fc regions of an Fc heterodimer at C-terminus of a CH1 of an Fab heavy chain, the second Fab is fused to a CH2 or a hinge region of another Fc region of the Fc heterodimer at C-terminus of a CH1 of an Fab heavy chain, and the first Fab is fused to C-terminus of the Fc region fused to the second Fab at N-terminus of a VH of an Fab heavy chain.

5. The trispecific antibody according to claim 4, wherein the fusion comprises direct fusion or fusion through a linker.

6. The trispecific antibody according to claim 5, wherein the linker is (GGGGS)n, wherein n = 1, 2, 3, or 4.

7. The trispecific antibody according to any one of claims 3 to 6, wherein the second Fab comprises a charge mutation, and the first or third Fab fused to the second Fab or to the second Fab through the Fc region comprises a charge mutation and a disulfide bond remodeling mutation.

8. The trispecific antibody according to claim 7, wherein the charge mutation is a mutation to D or K at position 39 of a VH of an Fab and to K or D at position 38 of a VL of the Fab, for example, the second Fab comprises a VH comprising 39D and a VL comprising 38K, and
the first or third Fab fused to the second Fab through the Fc region comprises a VH comprising 39K and a VL comprising 38D; or
the second Fab comprises a VH comprising 39K and a VL comprising 38D, and
the first or third Fab fused to the second Fab through the Fc region comprises a VH comprising 39D and a VL comprising 38K.

9. The trispecific antibody according to claim 7 or 8, wherein the disulfide bond remodeling mutation comprises F126C in a CH1 of an Fab and comprises Q124C in a CL of the Fab.

10. The trispecific antibody according to claim 9, wherein the Fab comprising a disulfide bond remodeling mutation comprises a CH1, wherein the CH1
(i) comprises an amino acid sequence having at least 90% identity to an amino acid sequence set forth in SEQ ID NO: 85, and comprises F126C; or
(ii) comprises or consists of an amino acid sequence set forth in SEQ ID NO: 85.

11. The trispecific antibody according to claim 9 or 10, wherein the Fab comprising a disulfide bond remodeling mutation comprises a CL, wherein the CL
(i) comprises an amino acid sequence having at least 90% identity to an amino acid sequence set forth in SEQ ID NO: 97, and comprises Q124C; or
(ii) comprises or consists of an amino acid sequence set forth in SEQ ID NO: 97.

12. The trispecific antibody according to claim 9, wherein the Fab comprising a disulfide bond remodeling mutation comprises a CH1 and a CL, wherein the CH1 comprises or consists of an amino acid sequence set forth in SEQ ID NO: 85, and the CL comprises or consists of an amino acid sequence set forth in SEQ ID NO: 97.

13. The trispecific antibody according to claim 7 or 8, wherein the disulfide bond remodeling mutation comprises F126C and C220S in a CH1 of an Fab and comprises Q124C and C214S in a CL of the Fab.

14. The trispecific antibody according to claim 13, wherein the Fab comprising a disulfide bond remodeling mutation comprises a CH1, wherein the CH1
(i) comprises an amino acid sequence having at least 90% identity to an amino acid sequence set forth in SEQ ID NO: 86, and comprises F126C and C220S; or
(ii) comprises or consists of an amino acid sequence set forth in SEQ ID NO: 86.

15. The trispecific antibody according to claim 13 or 14, wherein the Fab comprising a disulfide bond remodeling mutation comprises a CL, wherein the CL
(i) comprises an amino acid sequence having at least 90% identity to an amino acid sequence set forth in SEQ ID NO: 98, and comprises Q124C and C214S; or
(ii) comprises or consists of an amino acid sequence set forth in SEQ ID NO: 98.

16. The trispecific antibody according to claim 13, wherein the Fab comprising a disulfide bond remodeling mutation comprises a CH1 and a CL, wherein the CH1 comprises or consists of an amino acid sequence set forth in SEQ ID NO: 86, and the CL comprises or consists of an amino acid sequence set forth in SEQ ID NO: 98.

17. The trispecific antibody according to any one of claims 1 to 16, wherein the trispecific antibody comprises a first Fc region and a second Fc region, wherein the first Fc region and the second Fc region are identical or different.

18. The trispecific antibody according to claim 17, wherein the first Fc region and the second Fc region are human IgG Fcs, such as human IgG1 Fc, human IgG2 Fc, human IgG3 Fc, or human IgG4 Fc, for example, comprising or consisting of an amino acid sequence set forth in SEQ ID NO: 87 or 88, or an amino acid sequence having at least 90% identity, e.g., 95%, 96%, 97%, 99% or more identity, thereto.

19. The trispecific antibody according to claim 18, wherein the first and/or second Fc region comprises an L234A/L235A mutation.

20. The trispecific antibody according to claim 19, wherein the first and/or second Fc region comprises an amino acid sequence set forth in SEQ ID NO: 89 or 90, or comprises an amino acid sequence having at least 90% identity, e.g., 95%, 96%, 97%, 99% or more identity, to an amino acid sequence set forth in SEQ ID NO: 89 or 90 and comprises an amino acid substitution L234A/L235A.

21. The trispecific antibody according to any one of claims 17 to 20, wherein one of the first and second Fc regions comprises a Knob mutation and the other comprises a Hole mutation.

22. The trispecific antibody according to claim 21, wherein the one Fc region comprises an amino acid substitution T366W, and the other Fc region comprises amino acid substitutions T366S, L368A and Y407V (numbered according to EU index).

23. The trispecific antibody according to claim 22, wherein one Fc region polypeptide comprises an amino acid sequence having at least 85%, 90%, 95%, 96%, 97%, 98%, or 99% identity to an amino acid sequence set forth in SEQ ID NO: 91, and the other Fc region polypeptide comprises an amino acid sequence having at least 85%, 90%, 95%, 96%, 97%, 98%, or 99% identity to an amino acid sequence set forth in SEQ ID NO: 92.

24. The trispecific antibody according to claim 23, wherein the one Fc region polypeptide comprises or consists of an amino acid sequence set forth in SEQ ID NO: 91, and the other Fc region polypeptide comprises or consists of an amino acid sequence set forth in SEQ ID NO: 92.

25. The trispecific antibody according to claim 17, wherein the one Fc region comprises amino acid substitutions L234A/L235A and T366W, and the other Fc region comprises amino acid substitutions L234A/L235A, T366S, L368A and Y407V (numbered according to EU index).

26. The trispecific antibody according to claim 25, wherein one Fc region polypeptide comprises an amino acid sequence having at least 85%, 90%, 95%, 96%, 97%, 98%, or 99% identity to an amino acid sequence set forth in SEQ ID NO: 94, and the other Fc region polypeptide comprises an amino acid sequence having at least 85%, 90%, 95%, 96%, 97%, 98%, or 99% identity to an amino acid sequence set forth in SEQ ID NO: 93.

27. The trispecific antibody according to claim 26, wherein the one Fc region polypeptide comprises or consists of an amino acid sequence set forth in SEQ ID NO: 94, and the other Fc region polypeptide comprises or consists of an amino acid sequence set forth in SEQ ID NO: 93.

28. The trispecific antibody according to any one of claims 1 to 27, wherein the first antigen-binding region specifically binding to BCMA comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein
the HCDR1 comprises or consists of an amino acid sequence set forth in SEQ ID NO: 57; the HCDR2 comprises or consists of an amino acid sequence set forth in SEQ ID NO: 58; the HCDR3 comprises or consists of an amino acid sequence set forth in SEQ ID NO: 59; the LCDR1 comprises or consists of an amino acid sequence set forth in SEQ ID NO: 61; the LCDR2 comprises or consists of an amino acid sequence set forth in SEQ ID NO: 62; and the LCDR3 comprises or consists of an amino acid sequence set forth in SEQ ID NO: 63.

29. The trispecific antibody according to claim 28, wherein the VH of the first antigen-binding region specifically binding to BCMA comprises or consists of an amino acid sequence set forth in SEQ ID NO: 56, or an amino acid sequence having at least 90%, 91%, 92%, 93%, 94%, 95%, 96%, 97%, 98%, or 99% identity thereto.

30. The trispecific antibody according to claim 28 or 29, wherein the VL of the first antigen-binding region specifically binding to BCMA comprises or consists of an amino acid sequence set forth in SEQ ID NO: 60, or an amino acid sequence having at least 90%, 91%, 92%, 93%, 94%, 95%, 96%, 97%, 98%, or 99% identity thereto.

31. The trispecific antibody according to claim 30, wherein the first antigen-binding region specifically binding to BCMA comprises a VH and a VL, wherein the VH consists of an amino acid sequence set forth in SEQ ID NO: 56, and the VL of the first antigen-binding region specifically binding to BCMA consists of an amino acid sequence set forth in SEQ ID NO: 60.

32. The trispecific antibody according to claim 28, wherein the first antigen-binding region specifically binding to BCMA comprises a VH and a VL, wherein the VH comprises 39K and the VL comprises 38D; or the VH comprises 39D and the VL comprises 38K,
for example,
the VH of the first antigen-binding region
(i) comprises or consists of an amino acid sequence set forth in SEQ ID NO: 76, or
(ii) comprises an amino acid sequence having at least 90%, 91%, 92%, 93%, 94%, 95%, 96%, 97%, 98%, or 99% identity to an amino acid sequence set forth in SEQ ID NO: 76, and comprises a Q39K mutation;
and/or the VL of the first antigen-binding region
(i) comprises or consists of an amino acid sequence set forth in SEQ ID NO: 75, or
(ii) comprises an amino acid sequence having at least 90%, 91%, 92%, 93%, 94%, 95%, 96%, 97%, 98%, or 99% identity to an amino acid sequence set forth in SEQ ID NO: 75, and comprises a Q38D mutation.

33. The trispecific antibody according to any one of claims 1 to 32, wherein the third antigen-binding region specifically binds to GPRC5D and comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein
the HCDR1 comprises or consists of an amino acid sequence set forth in SEQ ID NO: 41; the HCDR2 comprises or consists of an amino acid sequence set forth in SEQ ID NO: 42; the HCDR3 comprises or consists of an amino acid sequence set forth in SEQ ID NO: 43; the LCDR1 comprises or consists of an amino acid sequence set forth in SEQ ID NO: 45; the LCDR2 comprises or consists of an amino acid sequence set forth in SEQ ID NO: 46; and the LCDR3 comprises or consists of an amino acid sequence set forth in SEQ ID NO: 47; or
the HCDR1 comprises or consists of an amino acid sequence set forth in SEQ ID NO: 49; the HCDR2 comprises or consists of an amino acid sequence set forth in SEQ ID NO: 50; the HCDR3 comprises or consists of an amino acid sequence set forth in SEQ ID NO: 51; the LCDR1 comprises or consists of an amino acid sequence set forth in SEQ ID NO: 53; the LCDR2 comprises or consists of an amino acid sequence set forth in SEQ ID NO: 54; and the LCDR3 comprises or consists of an amino acid sequence set forth in SEQ ID NO: 55.

34. The trispecific antibody according to claim 33, wherein the VH of the third antigen-binding region specifically binding to GPRC5D
(i) comprises or consists of an amino acid sequence set forth in SEQ ID NO: 40, or an amino acid sequence having at least 90%, 91%, 92%, 93%, 94%, 95%, 96%, 97%, 98%, or 99% identity thereto; or
(ii) comprises or consists of an amino acid sequence set forth in SEQ ID NO: 48, or an amino acid sequence having at least 90%, 91%, 92%, 93%, 94%, 95%, 96%, 97%, 98%, or 99% identity thereto.

35. The trispecific antibody according to claim 33 or 34, wherein the VL of the third antigen-binding region specifically binding to GPRC5D
(i) comprises or consists of an amino acid sequence set forth in SEQ ID NO: 44, or an amino acid sequence having at least 90%, 91%, 92%, 93%, 94%, 95%, 96%, 97%, 98%, or 99% identity thereto; or
(ii) comprises or consists of an amino acid sequence set forth in SEQ ID NO: 52, or an amino acid sequence having at least 90%, 91%, 92%, 93%, 94%, 95%, 96%, 97%, 98%, or 99% identity thereto.

36. The trispecific antibody according to claim 35, wherein the third antigen-binding region specifically binding to GPRC5D comprises a VH and a VL, wherein the VH consists of an amino acid sequence set forth in SEQ ID NO: 48, and the VL consists of an amino acid sequence set forth in SEQ ID NO: 52; or the VH consists of an amino acid sequence set forth in SEQ ID NO: 40, and the VL consists of an amino acid sequence set forth in SEQ ID NO: 44.

37. The trispecific antibody according to any one of claims 33 to 36, wherein the third antigen-binding region specifically binding to GPRC5D comprises a VH and a VL, wherein the VH comprises 39K and the VL comprises 38D; or the VH comprises 39D and the VL comprises 38K, for example,
the VH of the third antigen-binding region specifically binding to GPRC5D
(i) comprises or consists of an amino acid sequence set forth in SEQ ID NO: 80, or
(ii) comprises an amino acid sequence having at least 90%, 91%, 92%, 93%, 94%, 95%, 96%, 97%, 98%, or 99% identity to an amino acid sequence set forth in SEQ ID NO: 80, and comprises a Q39K mutation;
and the VL of the third antigen-binding region specifically binding to GPRC5D
(i) comprises or consists of an amino acid sequence set forth in SEQ ID NO: 79, or
(ii) comprises an amino acid sequence having at least 90%, 91%, 92%, 93%, 94%, 95%, 96%, 97%, 98%, or 99% identity to an amino acid sequence set forth in SEQ ID NO: 79, and comprises a Q38D mutation.

38. The trispecific antibody according to any one of claims 1 to 37, wherein the second antigen-binding region specifically binds to CD3 and comprises a heavy chain variable region VH and a light chain variable region VL, wherein the heavy chain variable region comprises HCDR1, HCDR2 and HCDR3, and the light chain variable region comprises LCDR1, LCDR2 and LCDR3, wherein the HCDR1 comprises or consists of an amino acid sequence set forth in SEQ ID NO: 65; the HCDR2 comprises or consists of an amino acid sequence set forth in SEQ ID NO: 66; the HCDR3 comprises or consists of an amino acid sequence set forth in SEQ ID NO: 67 or 73; the LCDR1 comprises or consists of an amino acid sequence set forth in SEQ ID NO: 69; the LCDR2 comprises or consists of an amino acid sequence set forth in SEQ ID NO: 70; and the LCDR3 comprises or consists of an amino acid sequence set forth in SEQ ID NO: 71.

39. The trispecific antibody according to claim 38, wherein the VH of the second antigen-binding region specifically binding to CD3 comprises or consists of an amino acid sequence set forth in SEQ ID NO: 64 or 72, or an amino acid sequence having at least 90%, 91%, 92%, 93%, 94%, 95%, 96%, 97%, 98%, or 99% identity thereto.

40. The trispecific antibody according to claim 38 or 39, wherein the VL of the second antigen-binding region specifically binding to CD3 comprises or consists of an amino acid sequence set forth in SEQ ID NO: 68, or an amino acid sequence having at least 90%, 91%, 92%, 93%, 94%, 95%, 96%, 97%, 98%, or 99% identity thereto.

41. The trispecific antibody according to claim 40, wherein the second antigen-binding region specifically binding to CD3 comprises a VH and a VL, wherein the VH consists of an amino acid sequence set forth in SEQ ID NO: 64 or 72, and the VL of the second antigen-binding region consists of an amino acid sequence set forth in SEQ ID NO: 68.

42. The trispecific antibody according to any one of claims 38 to 41, wherein the second antigen-binding region specifically binding to CD3 comprises a VH and a VL, wherein the VH comprises 39K and the VL comprises 38D; or the VH comprises 39D and the VL comprises 38K.

43. The trispecific antibody according to claim 42, wherein the VH of the second antigen-binding region specifically binding to CD3
(i) comprises or consists of an amino acid sequence set forth in SEQ ID NO: 77 or 78, or
(ii) comprises an amino acid sequence having at least 90%, 91%, 92%, 93%, 94%, 95%, 96%, 97%, 98%, or 99% identity to an amino acid sequence set forth in SEQ ID NO: 77 or 78, and comprises a Q39D mutation;
and the VL of the second antigen-binding region specifically binding to CD3
(i) comprises or consists of an amino acid sequence set forth in SEQ ID NO: 74, or
(ii) comprises an amino acid sequence having at least 90%, 91%, 92%, 93%, 94%, 95%, 96%, 97%, 98%, or 99% identity to an amino acid sequence set forth in SEQ ID NO: 74, and comprises a Q38K mutation.

44. The trispecific antibody according to any one of claims 3 to 43, wherein the trispecific antibody is a left-right asymmetric IgG-like pentamer consisting of 5 polypeptide chains and consists of the following peptide chains:
a peptide chain 1# sequentially comprising an Fab heavy chain specifically binding to GPRC5D and an Fc domain,
a peptide chain 2# comprising an Fab light chain specifically binding to GPRC5D,
a peptide chain 3# sequentially comprising an Fab heavy chain specifically binding to BCMA, an Fab heavy chain specifically binding to CD3, and an Fc domain,
a peptide chain 4# comprising an Fab light chain specifically binding to BCMA, and
a peptide chain 5# comprising an Fab light chain specifically binding to CD3,
wherein the Fab heavy chain of the peptide chain 1# is paired with the Fab light chain of the peptide chain 2# to form the third Fab, and the two Fab heavy chains of the peptide chain 3# are paired with the Fab light chains of the peptide chain 4# and the peptide chain 5# to form the first Fab and the second Fab, respectively.

45. The trispecific antibody according to claim 44, wherein
the peptide chain 1# comprises or consists of an amino acid sequence set forth in SEQ ID NO: 5, or an amino acid sequence having at least 85%, 90%, 91%, 92%, 93%, 94%, 95%, 96%, 97%, 98%, or 99% identity to an amino acid sequence set forth in SEQ ID NO: 5;
the peptide chain 2# comprises or consists of an amino acid sequence set forth in SEQ ID NO: 4, or an amino acid sequence having at least 85%, 90%, 91%, 92%, 93%, 94%, 95%, 96%, 97%, 98%, or 99% identity to an amino acid sequence set forth in SEQ ID NO: 4;
the peptide chain 3# comprises or consists of an amino acid sequence set forth in SEQ ID NO: 3 or 6, or an amino acid sequence having at least 85%, 90%, 91%, 92%, 93%, 94%, 95%, 96%, 97%, 98%, or 99% identity to an amino acid sequence set forth in SEQ ID NO: 3 or 6;
the peptide chain 4# comprises or consists of an amino acid sequence set forth in SEQ ID NO: 2, or an amino acid sequence having at least 85%, 90%, 91%, 92%, 93%, 94%, 95%, 96%, 97%, 98%,
or 99% identity to an amino acid sequence set forth in SEQ ID NO: 2; and/or
the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.

46. The trispecific antibody according to claim 44, wherein
the peptide chain 1# comprises or consists of an amino acid sequence set forth in SEQ ID NO: 15, or an amino acid sequence having at least 85%, 90%, 91%, 92%, 93%, 94%, 95%, 96%, 97%, 98%, or 99% identity to an amino acid sequence set forth in SEQ ID NO: 15;
the peptide chain 2# comprises or consists of an amino acid sequence set forth in SEQ ID NO: 14, or an amino acid sequence having at least 85%, 90%, 91%, 92%, 93%, 94%, 95%, 96%, 97%, 98%, or 99% identity to an amino acid sequence set forth in SEQ ID NO: 14;
the peptide chain 3# comprises or consists of an amino acid sequence set forth in SEQ ID NO: 3 or 6, or an amino acid sequence having at least 85%, 90%, 91%, 92%, 93%, 94%, 95%, 96%, 97%, 98%, or 99% identity to an amino acid sequence set forth in SEQ ID NO: 3 or 6;
the peptide chain 4# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2; and/or
the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.

47. The trispecific antibody according to any one of claims 1 to 43, wherein the trispecific antibody is a left-right asymmetric IgG-like pentamer consisting of 5 polypeptide chains and consists of the following peptide chains:
a peptide chain 1# sequentially comprising an Fab heavy chain specifically binding to BCMA and an Fc domain,
a peptide chain 2# comprising an Fab light chain specifically binding to BCMA,
a peptide chain 3# sequentially comprising an Fab heavy chain specifically binding to GPRC5D, an Fab heavy chain specifically binding to CD3, and an Fc domain,
a peptide chain 4# comprising an Fab light chain specifically binding to GPRC5D, and
a peptide chain 5# comprising an Fab light chain specifically binding to CD3,
wherein the Fab heavy chain of the peptide chain 1# is paired with the Fab light chain of the peptide chain 2# to form the first Fab, and the two Fab heavy chains of the peptide chain 3# are paired with the Fab light chains of the peptide chain 4# and the peptide chain 5# to form the third Fab and the second Fab, respectively.

48. The trispecific antibody according to claim 47, wherein
the peptide chain 1# comprises or consists of an amino acid sequence set forth in SEQ ID NO: 11, or an amino acid sequence having at least 85%, 90%, 91%, 92%, 93%, 94%, 95%, 96%, 97%, 98%, or 99% identity to an amino acid sequence set forth in SEQ ID NO: 11;
the peptide chain 2# comprises or consists of an amino acid sequence set forth in SEQ ID NO: 10, or an amino acid sequence having at least 85%, 90%, 91%, 92%, 93%, 94%, 95%, 96%, 97%, 98%, or 99% identity to an amino acid sequence set forth in SEQ ID NO: 10;
the peptide chain 3# comprises or consists of an amino acid sequence set forth in SEQ ID NO: 9, or an amino acid sequence having at least 85%, 90%, 91%, 92%, 93%, 94%, 95%, 96%, 97%, 98%, or 99% identity to an amino acid sequence set forth in SEQ ID NO: 9;
the peptide chain 4# comprises or consists of an amino acid sequence set forth in SEQ ID NO: 8, or an amino acid sequence having at least 85%, 90%, 91%, 92%, 93%, 94%, 95%, 96%, 97%, 98%, or 99% identity to an amino acid sequence set forth in SEQ ID NO: 8; and/or
the peptide chain 5# comprises or consists of an amino acid sequence set forth in SEQ ID NO: 1, or an amino acid sequence having at least 85%, 90%, 91%, 92%, 93%, 94%, 95%, 96%, 97%, 98%, or 99% identity to an amino acid sequence set forth in SEQ ID NO: 1.

49. The trispecific antibody according to any one of claims 1 to 43, wherein the trispecific antibody is a left-right asymmetric IgG-like pentamer consisting of 5 polypeptide chains and consists of the following peptide chains:
a peptide chain 1# sequentially comprising an Fab heavy chain specifically binding to GPRC5D and an Fc domain,
a peptide chain 2# comprising an Fab light chain specifically binding to GPRC5D,
a peptide chain 3# sequentially comprising an Fab heavy chain specifically binding to CD3, an Fc domain, and an Fab heavy chain of the antigen-binding region specifically binding to BCMA,
a peptide chain 4# comprising an Fab light chain specifically binding to CD3, and
a peptide chain 5# comprising an Fab light chain of the antigen-binding region specifically binding to BCMA,
wherein the Fab heavy chain of the peptide chain 1# is paired with the Fab light chain of the peptide chain 2# to form the third Fab, and the two Fab heavy chains of the peptide chain 3# are paired with the Fab light chains of the peptide chain 4# and the peptide chain 5# to form the second Fab and the first Fab, respectively.

50. The trispecific antibody according to claim 49, wherein
the peptide chain 1# comprises or consists of an amino acid sequence set forth in SEQ ID NO: 5, or an amino acid sequence having at least 85%, 90%, 91%, 92%, 93%, 94%, 95%, 96%, 97%, 98%, or 99% identity to an amino acid sequence set forth in SEQ ID NO: 5;
the peptide chain 2# comprises or consists of an amino acid sequence set forth in SEQ ID NO: 4, or an amino acid sequence having at least 85%, 90%, 91%, 92%, 93%, 94%, 95%, 96%, 97%, 98%, or 99% identity to an amino acid sequence set forth in SEQ ID NO: 4;
the peptide chain 3# comprises or consists of an amino acid sequence set forth in SEQ ID NO: 7, or an amino acid sequence having at least 85%, 90%, 91%, 92%, 93%, 94%, 95%, 96%, 97%, 98%, or 99% identity to an amino acid sequence set forth in SEQ ID NO: 7;
the peptide chain 4# comprises or consists of an amino acid sequence set forth in SEQ ID NO: 1, or
an amino acid sequence having at least 85%, 90%, 91%, 92%, 93%, 94%, 95%, 96%, 97%, 98%, or 99% identity to an amino acid sequence set forth in SEQ ID NO: 1; and/or
the peptide chain 5# comprises or consists of an amino acid sequence set forth in SEQ ID NO: 2, or an amino acid sequence having at least 85%, 90%, 91%, 92%, 93%, 94%, 95%, 96%, 97%, 98%, or 99% identity to an amino acid sequence set forth in SEQ ID NO: 2.

51. A nucleic acid molecule encoding any one of the chains of the trispecific antibody according to any one of claims 1 to 50, or consisting of a nucleic acid sequence.

52. An expression vector comprising the nucleic acid molecule according to claim 51, wherein, for example, the expression vector is a pCNDA vector, such as a pCDNA3.1 expression vector.

53. A host cell comprising the nucleic acid molecule according to claim 51 or the expression vector according to claim 52, wherein, preferably, the host cell is prokaryotic or eukaryotic, e.g., a 293 cell or a CHO cell, such as a HEK293 cell.

54. A method for preparing the trispecific antibody according to any one of claims 1 to 50, wherein the method comprises culturing the host cell comprising the nucleic acid molecule according to claim 51 or the expression vector according to claim 52 under a condition suitable for expressing the chains of the antibody, and optionally recovering the antibody from the host cell (or a host cell medium).

55. An immunoconjugate comprising the trispecific antibody according to any one of claims 1 to 50.

56. A pharmaceutical composition or a drug or a formulation comprising the trispecific antibody according to any one of claims 1 to 50 or the immunoconjugate according to claim 55, and optionally a pharmaceutical supplementary material.

57. A pharmaceutical combination comprising the trispecific antibody according to any one of claims 1 to 50 or the immunoconjugate according to claim 55, and one or more additional therapeutic agents, such as a chemotherapeutic agent.

58. A method for preventing or treating a cancer in a subject, comprising administering to the subject an effective amount of the trispecific antibody according to any one of claims 1 to 50, or the immunoconjugate according to claim 55, or the pharmaceutical composition or the formulation according to claim 56, or the pharmaceutical combination according to claim 57.

59. The method according to claim 58, wherein the cancer is a GPRC5D-single-positive cancer, a BCMA-single-positive cancer, a GPRC5D/BCMA-double-positive cancer, or a cancer with double low expression of BCMA and GPRC5D, such as a cancer with low expression of GPRC5D and/or low expression of BCMA (e.g., a cancer that recurs after anti-BCMA therapy or anti-GPRC5D therapy).

60. The method according to claim 58 or 59, wherein the cancer is a solid tumor or a hematological tumor, for example, the cancer is multiple myeloma.

61. The method according to any one of claims 58 to 60, wherein the method further comprises administering in combination with additional therapies, such as therapeutic modalities (e.g., surgical therapy or radiotherapy) and/or additional therapeutic agents (e.g., a chemotherapeutic agent).
